(19) Europäisches Patentamt
European Patent Office
Office européen des brevets

(11) **EP 4 183 333 A1**

(12) **EUROPEAN PATENT APPLICATION**
published in accordance with Art. 153(4) EPC

(43) Date of publication:
**24.05.2023 Bulletin 2023/21**

(21) Application number: **21842607.0**

(22) Date of filing: **15.07.2021**

(51) International Patent Classification (IPC):
**A61B 5/055** (2006.01)     **A61B 5/05** (2021.01)
**A61B 5/372** (2021.01)     **G06T 7/00** (2017.01)

(52) Cooperative Patent Classification (CPC):
**A61B 5/05; A61B 5/055; A61B 5/372; G06T 7/00**

(86) International application number:
**PCT/JP2021/026658**

(87) International publication number:
**WO 2022/014682 (20.01.2022 Gazette 2022/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.07.2020   JP 2020123208**

(71) Applicants:
• **Advanced Telecommunications Research
  Institute
  International
  Soraku-gun
  Kyoto 619-0288 (JP)**
• **Hiroshima University
  Higashi-Hiroshima-shi
  Hiroshima 739-8511 (JP)**
• **Shionogi & Co., Ltd
  Osaka-shi, Osaka 541-0045 (JP)**

(72) Inventors:
• **KASHIWAGI, Yuto**
  **Soraku-gun, Kyoto 619-0288 (JP)**
• **TOKUDA, Tomoki**
  **Soraku-gun, Kyoto 619-0288 (JP)**
• **TAKAHARA, Yuji**
  **Soraku-gun, Kyoto 619-0288 (JP)**
• **KAWATO, Mitsuo**
  **Soraku-gun, Kyoto 619-0288 (JP)**
• **YAMASHITA, Ayumu**
  **Soraku-gun, Kyoto 619-0288 (JP)**
• **YAMASHITA, Okito**
  **Soraku-gun, Kyoto 619-0288 (JP)**
• **SAKAI, Yuki**
  **Soraku-gun, Kyoto 619-0288 (JP)**
• **YOSHIMOTO, Junichiro**
  **Soraku-gun, Kyoto 619-0288 (JP)**
• **OKADA, Go**
  **Hiroshima-shi, Hiroshima 739-8511 (JP)**

(74) Representative: **Müller Hoffmann & Partner
Patentanwälte mbB
St.-Martin-Strasse 58
81541 München (DE)**

(54) **BRAIN FUNCTIONAL CONNECTIVITY CORRELATION VALUE CLUSTERING DEVICE, BRAIN FUNCTIONAL CONNECTIVITY CORRELATION VALUE CLUSTERING SYSTEM, BRAIN FUNCTIONAL CONNECTIVITY CORRELATION VALUE CLUSTERING METHOD, BRAIN FUNCTIONAL CONNECTIVITY CORRELATION VALUE CLASSIFIER PROGRAM, AND BRAIN ACTIVITY MARKER CLASSIFICATION SYSTEM**

(57) There is provided a therapy selection support device that generates a discriminator (identifier) as a diagnostic marker or a classifier as a stratification marker through machine learning on the basis of measurement data on brain activities and that uses the discriminator or the classifier as a biomarker.

A therapy selection support system 300a, 300b, 500 includes a clustering device 300b that executes stratification in which the results of measurement of brain functional connectivity correlation values acquired from a plurality of second subjects are divided into a plurality of clusters through a clustering process. The therapy selection support system further includes: a database device 5100 that stores clusters obtained as a result of stratification by a clustering classifier and corresponding predetermined therapy information in association with each other; and a support information providing device 300a that receives an input of the results of measurement of brain activities of a first subject and that outputs corresponding therapy information in accordance with the re-

**(Cont. next page)**

sults of classification by the clustering classifier for the
measurement results.

Fig.39

[Fig.39]

**Description**

Technical Field

The present invention relates to a technique for clustering brain functional connectivity correlation values measured by functional brain imaging by a plurality of devices and, more specifically, to a brain functional connectivity correlation value clustering device, a brain functional connectivity correlation value clustering system, a brain functional connectivity correlation value clustering method, a brain functional connectivity correlation value classifier program, and a brain activity marker classification system.

Background Art

(Data Driven Clustering Method)

Recent developments in artificial intelligence technology, particularly in data-driven artificial intelligence technology has realized applications which sometimes rival, and in some aspects surpass, human abilities in the fields of speech recognition, translation, image recognition, and so on (for example, see Patent Literature 1).

In the field of medical technology also, use of machine learning including deep learning is increasing in, for example, diagnostic imaging. Deep learning refers to machine learning using multi-layered neural networks and, in the field of image recognition, it is known that a method of learning using Convolutional Neural Network (hereinafter referred to as "CNN"), which is a type of deep learning, shows a performance significantly higher than conventional methods (for example, see Patent Literature 2).

By way of example, in the fields of endoscopic diagnostic imaging for colorectal cancer and the like, diagnostic devices are now used in practical services which show accuracy of diagnosis higher than those of humans (Non-Patent Literature 1).

It should be noted that, from the viewpoint of classification of machine learning, almost all of such artificial intelligence technologies belong to the category of so-called "supervised learning," which requires preparation of a huge number of pairs of correct data and input data (for example, image data) to be used as inputs for training artificial intelligence.

By contrast, data-driven artificial intelligence may be applied to performance of tasks of classifying given data into a number of clusters based on their features. For such applications, so-called "unsupervised learning" is known for which correct data is not available, as well as "semi-supervised learning" combining learning with a small amount of "correct-labeled training data" with a large amount of "correct-label-less training data" (for example, Patent Literature 3).

By way of example, according to Patent Literature 3, "semi-supervised learning refers to a method of learning based on a relatively small amount of labeled data and label-less data, and it includes, by way of example, a bootstrap method in which learning model for classification is generated using labeled data (teacher data T including state data S and label data L), and the learning model is additionally trained to improve the precision of learning by using the learning model and label-less data (state data S), and a graph base algorithm in which a learning model as a classifier is generated by grouping based on labeled and label-less data distributions." As described in the examples above, however, in the "semi-supervised learning," only a small number of training data have associated teacher data. It is assumed that with this data, a classifier is generated first, and then the classifier itself is retrained by using a huge amount of "correct-label-less training data."

(Biomarker)

In the following, we take examples of applications of discrimination and clustering using artificial intelligence technology in the medical field.

A "biomarker" refers to biological information converted into a numerical value or quantified as an index for quantitatively comprehending any biological changes in a living body.

The FDA (the United States Food and Drug Administration) defines a biomarker as "a characteristic that is objectively measured and evaluated as an indicator of normal biological processes, pathogenic processes, or pharmacological responses to a therapeutic intervention." Biomarkers representative of a state of or a change in a disease, or a degree of healing are used as surrogate markers (substitute markers) to monitor efficacy in clinical tests of new drugs. The blood sugar level, the cholesterol level, and the like are representative biomarkers used as indexes of lifestyle diseases. Biomarkers include not only substances of biological origin contained in urine or blood but also electrocardiogram, blood pressure, PET (positron emission tomography) images, bone density, lung function, and the like. Developments in genomic analysis and proteome analysis have led to the discovery of various biomarkers related to DNA, RNA, or biological protein.

Biomarkers are promising for measuring therapeutic efficacy after the onset of a disease and, in addition, as routine preventive indexes, promising for disease prevention. Further, biomarkers are expected to be applied to individualized medicine for selecting effective treatment avoiding side effects.

For example, a biomarker for determining possibility of having a disease using genetic information is disclosed (Patent Literature 4). According to Patent Literature 4, a "biomarker" or a "marker" is "biological molecules that can be objectively measured as representing features of physiological state of the biological system." Patent Literature 4 describes the biomarkers as "typically, biomarker measurements represent information related to quantitative measurement of expression product, which typically is protein or polypeptide. The present invention envisions determining biomarker measurements on the RNA (pre-translation) level or on protein level (including post-translational modification)." Patent Literature 4 shows examples of classifiers to be used as the "classification system" of such biomarker measurements, including a decision tree, a Bayesian classifier, a Bayesian belief network, k-nearest neighbor method, case-based reasoning, and a support vector machine.

However, because diagnosis at present in the field of neurological/mental disorders is largely based on so-called symptoms in accordance with the Diagnostic and Statistical Manual of Mental Disorders, v.5 (DSM-5), it should in fairness be stated that molecular markers and the like are still in a research phase although biomarkers usable as objective indexes from a biochemical or molecular genetics viewpoint have been studied.

Nevertheless, a disease determination system has been reported which uses an NIRS (Near-InfraRed Spectroscopy) technique to classify mental disorders such as schizophrenia and depression based on features of hemoglobin signals measured by biological optical measurement (Patent Literature 5).

(Biomarker Based on Brain Activities)

Meanwhile, in the field of so-called diagnostic imaging, there is a so-called "image biomarker" different from the concept of biomarker as "biological molecules" described above. For example, there are researches related to neurotransmission function or receptor function analysis using PET (positron emission tomography) for molecular imaging in cranial nerve regions.

Further, in nuclear Magnetic Resonance Imaging (MRI), changes appearing in detected signals in accordance with changes in the blood stream make it possible to visualize portions of a brain activated in response to an external stimulus or the like. Such nuclear magnetic resonance imaging is specifically referred to as fMRI (functional MRI).

An fMRI uses a common MRI apparatus with additional hardware and software necessary for fMRI measurement.

The changes in blood stream cause changes in NMR signal intensity, since oxygenated hemoglobin and deoxygenated hemoglobin in the blood have different magnetic properties. Oxygenated hemoglobin is diamagnetic and does not have any influence on relaxation time of hydrogen atoms in the surrounding water whereas deoxygenated hemoglobin is paramagnetic and changes surrounding magnetic field. Therefore, when the brain is stimulated and local blood stream increases, any resulting changes in oxygenated hemoglobin can be detected by MRI signals. Commonly used stimuli to a subject may include, for example, visual stimulus, audio stimulus, or performance of a prescribed task.

In the studies of the brain functions, brain activities are measured by measuring increase in the nuclear magnetic resonance signal (MRI signal) of hydrogen atoms representing a phenomenon where the deoxygenated hemoglobin level in red blood cells decrease in minute veins or capillary vessels (BOLD effect).

The blood oxygen level dependent signal that reflects a brain activity measured by fMRI devices is referred to as a BOLD signal (Blood Oxygen Level Dependent Signal).

Particularly, in studies related to the human motor functions, brain activities are measured by the fMRI as described above while a subject is performing some physical activity.

For human subjects, it is necessary to measure the brain activities in a non-invasive manner. In this aspect, a decoding technique has been developed to extract more detailed information from fMRI data. Specifically, a voxel-by-voxel (voxel: volumetric pixel) brain activity analysis of the brain by the fMRI enables the estimation of stimulus input or the state of recognition from spatial patterns of the brain activities.

Further, as a development of such a decoding technique, Patent Literature 6 discloses a method of brain function analysis for realizing "diagnostic biomarkers" based on functional brain imaging for a neurological/mental disorder. According to this method, from measurement data of resting-state functional connectivity MRI of a healthy cohort and a patient cohort, a correlation matrix (brain functional connectivity parameters) of degrees of activities between prescribed brain regions is derived for each subject. Feature extraction is performed by regularized canonical correlation analysis on the correlation matrix and on the attributes of subjects including diseased/healthy labels of the subjects. Based on the results of regularized canonical correlation analysis, a discriminator that functions as a biomarker is generated from discriminant analysis by sparse logistic regression (SLR). It has been indicated that by such a technique of machine learning, results of neurological disease diagnosis can be predicted based on connections among brain regions derived from fMRI data in the resting state. Further, verification of the prediction performance indicated that the prediction is not only applicable to the brain activities measured in a certain facility but also generalizable, to some extent, to the brain activities measured in a different facility.

Further, technical improvements are made to enhance generalization performance of the above-described "diagnostic biomarkers" (Patent Literature 7).

Recently, as in the human connectome project in the United States, obtaining and sharing large scale brain image data come to be recognized as having a significant meaning in filling in the gaps between basic neuro-scientific research and clinical applications such as diagnosis and therapy of mental diseases or psychiatric disorder (Non-Patent Literature 2).

In 2013, Japan Agency for Medical Research and Development, which is one of the Japanese National Research and Development Agencies, organized a Decoded Neurofeedback (DecNef) project, in which eight laboratories collected data of functional magnetic resonance in resting state (resting state functional MRI) covering five diseases and 2,239 samples from a plurality of sites, and the data was publicly shared through a database (https://bicr-resource.atr.jp/decnefpro/) of a plurality of diseases at a plurality of sites of SRPBS (Strategic Research Program for Brain Science, https://www.amed.go.jp/program/list/01/04/001_nopro.html). This project identified biomarkers based on resting state functional connectivity (resting state functional connectivity MRI) of several psychiatric disorders that could be generalized to fully independent cohorts.

As described above, diagnosis of a healthy cohort and a patient cohort have attained some positive results. Meanwhile, it is known that some patient cohorts, e.g. a group of patients generally diagnosed as having "depression," may actually be divided into several subtypes. For example, common "antidepressant" works so well to achieve remission for some patients while not so well to other patients who are "treatment-resistant."

There has been an attempt to classify such patients of "depression" by applying clustering using data-driven artificial intelligence to the above-described "brain functional connectivity parameters," and some references report that a certain tendency is observed (Non-Patent Literatures 3 and 4).

In order to practically utilize such a method of classifying subtypes of a disorder group, however, it is necessary to obtain large-scale data of the disorder group. However, large scale collection of brain image data of healthy persons, let alone patients, is difficult.

Therefore, when acquiring multisite measurement data to realize large-scale data collection, site differences between measured data in respective measuring sites will be the chief concern. Non-Patent Literature 4 mentioned above notes that the "generalization" of clustering of a huge amount of measurement data from many facilities is a problem to be solved in the future.

For instance, Non-Patent Literature 3 mentioned above indicated that patients of depression were stratified with four subtypes having different therapeutic responses to TMS (transcranial magnetic stimulation). It is pointed out, however, in a different reference (Non-Patent Literature 5) that in the process of finding brain functional connectivity indexes, data of depression symptoms are used twice, and because of the overtraining, the statistical significance of the relation with depression symptoms could not be confirmed and stratification stability was not satisfactory.

Therefore, at least at present, precision of stratification in independent validation data regarding depression, for example, has not been confirmed.

Meanwhile, by way of example, for evaluating the site-to-site differences of measurement data when MRI measurements are done at a plurality of measurement sites, adoption of a so-called "traveling subject" is proposed, wherein a large number of participants travel to and are measured at the plurality of sites, in order to evaluate an effect on measurement bias on the resting state functional connectivity (Non-Patent Literatures 6 and 7).

In any case, when attributes of subjects are to be classified based on fMRI data, in order to avoid the problem of overtraining, it is a common practice in the field of machine learning to evaluate a classifier using leave-one-subject-out cross validation in which one subject is left out to be used for validation, or using 10-fold cross validation in which data is divided to ten groups, nine of which are used for learning and the remaining one is used for validation. Recently, however, it is recognized in the field of psychiatry as well that machine learning applied to a small number of samples taken from a single facility possibly leads to inflated prediction.

Machine learning on a small amount of data is highly prone to overtraining because of noises or specific tendencies of training data derived from fMRI devices of specific facilities, or methods of measurement, experimenters or participant groups.

By way of example, it is reported that a classifier discriminating autism spectrum disorder from anatomic brain images shows sensitivity and specificity of 90 % or higher when used for training data of United Kingdom that was used for development, while its performance is as low as 50% when applied to data of Japanese people. From the foregoing, we assume that a classifier not validated using an independent validation cohort consisting of a group of subjects and a facility that are totally different from the training data does not bear either scientific or practical significance.

The applicant of the present application reports a "method of harmonization" in order to compensate for the above-described site-to-site differences among measurement sites (Non-Patent Literature 8).

Citation List

Patent Literature

PL1: JP 2018/147193 A1 (WO 2018/147193)
PL2: JP 2019-198376 A
PL3: JP 2020-024139 A
PL4: JP 2019-516950 A (WO 2017/162773)
PL5: JP 2005/025421 A1 (WO 2005/025421)
PL6: JP 2015-62817 A
PL7: JP 2017-196523 A

Non-Patent Literature

NPL1: Press release, dated December 10, 2018, from Japan Agency for Medical Research and Development "AI mounting endoscope diagnosis assisting program approved - Expected to assist physicians' diagnosis" https://www.amed.go.jp/news/release_20181210.html
NPL2: Glasser MF, et al. The Human Connectome Project's neuroimaging approach. NatNeurosci 19, 1175-1187 (2016)
NPL3: Andrew T Drysdale, Logan Grosenick, Jonathan Downar, Katharine Dunlop, Farrokh Mansouri, Yue Meng1, Robert N Fetcho, Benjamin Zebley, Desmond J Oathes, Amit Etkin, Alan F Schatzberg, Keith Sudheimer, Jennifer Keller, Helen S Mayberg, Faith M Gunning, George S Alexopoulos, Michael D Fox, Alvaro Pascual-Leone, Henning U Voss, BJ Casey, Marc J Dubin & Conor Liston, "Resting-state connectivity biomarkers define neurophysiological subtypes of depression", nature medicine, VOLUME 23, NUMBER 1, JANUARY 2017
NPL4: Tomoki Tokuda, Junichiro Yoshimoto, Yu Shimizu, Go Okada, Masahiro Takamura, Yasumasa Okamoto, Shigeto Yamawaki, Kenji Doya, "Identification of depression subtypes and relevant brain regions using a data-driven approach", SCIENTIFIC REPORTS | (2018) 8:14082 | DOI:10.1038/s41598-018-32521-z
NPL5: Richard Dinga, Lianne Schmaal, Brenda W.J.H. Penninx, Marie Josevan Tol, Dick J. Veltman, Laura van Velzen, Maarten Mennes, Nic J.A.van der Wee, Andre F. Marquand, "Evaluating the evidence for biotypes of depression: Methodological replication and extension of Drysdale et al. (2017)", NeuroImage: Clinical 22 (2019) 101796
NPL6: Noble S, et al. Multisite reliability of MR-based functional connectivity. Neuroimage 146, 959-970 (2017)
NPL7: Pearlson G. Multisite collaborations and large databases in psychiatric neuroimaging advantages, problems, and challenges. Schizophr Bull 35, 1-2 (2009)
NPL8: Ayumu Yamashita, Noriaki Yahata, Takashi Itahashi, Giuseppe Lisi, Takashi Yamada, Naho Ichikawa, Masahiro Takamura, Yujiro Yoshihara, Akira Kunimatsu, Naohiro Okada, Hirotaka Yamagata, Koji Matsuo, Ryuichiro Hashimoto, Go Okada, Yuki Sakai, Jun Morimoto, Jin Narumoto, Yasuhiro Shimada, Kiyoto Kasai, Nobumasa Kato, Hidehiko Takahashi, Yasumasa Okamoto, Saori C Tanaka, Mitsuo Kawato, Okito Yamashita, and Hiroshi Imamizu, "Harmonization of resting-state functional MRI data across multiple imaging sites via the separation of site differences into sampling bias and measurement bias", PLOS Biology. DOI: 10. 1371/journal.pbio.3000042, http://journals.plos.org/plosbiology/article?id=0.1371/journal.pbio.3000042

Summary of Invention

Technical Problem

When we consider applying the analysis of brain activities using functional brain imaging such as functional Magnetic Resonance Imaging to treatment of a neurological/mental disorder as described above, the analysis of brain activities using functional brain imaging as the above-described biomarker is promising as a non-invasive functional marker, and applications to development of a diagnostic method and to searching/identification of target molecules aiming toward drug discoveries for realizing basic remedies are also expected.

By way of example, consider a mental disorder. Practical biomarkers using genes are not established yet and, therefore, the development of therapeutic agents remains difficult, since it is difficult to determine the effect of medication.

The present invention was made to solve the above-described problem and its object is to provide a therapy selection support system, a therapy selection support device, a therapy selection support method, and a therapy selection support program that generate a discriminator (identifier) as a diagnostic marker or a classifier as a stratification marker through machine learning on the basis of measurement data on brain activities and that provide information related to selection of a therapy for a subject with depression symptoms on the basis of the results of measurement of brain activities of the subject using the discriminator (identifier) or the classifier as a biomarker.

Another object of the present invention is to provide a screening support system, a screening support device, a screening support method, and a screening support program for supporting screening for a subject on the basis of the results of measurement of the brain activities of the subject in a clinical test for a therapeutic agent candidate substance for depression symptoms.

Solution to Problem

According to an aspect of the invention, an embodiment of the present invention is directed to a therapy selection support system that provides information related to selection of a therapy for a first subject with depression symptoms on the basis of results of measurement of brain activities of the first subject. The therapy selection support system includes a clustering device that executes stratification in which results of measurement of brain functional connectivity correlation values acquired from a plurality of second subjects are divided into a plurality of clusters through a clustering process. The plurality of second subjects include a first cohort having a diagnosis label of a depression and a second cohort not having the diagnosis label of the depression. The clustering device includes a processor and a storage device, the processor being configured to execute the clustering process for the plurality of second subjects. The processor is configured to, in a process of generating a clustering classifier, i) store, in the storage device, features based on a plurality of brain functional connectivity correlation values that represent time correlation of brain activities among a plurality of predetermined brain area pairs for each of the plurality of second subjects, ii) execute machine learning, through supervised learning, to generate an identifier model for discriminating presence or absence of the diagnosis label on the basis of the features stored in the storage device, iii) select features for clustering in accordance with a degree of importance of features that are used to generate an identifier through the machine learning in the machine learning to generate the identifier model, and iv) generate a clustering classifier by clustering the first cohort through a multiple co-clustering method as unsupervised learning on the basis of the selected features for the clustering. The therapy selection support system further includes a database device that stores the clusters obtained as a result of the stratification by the clustering classifier and corresponding predetermined therapy information in association with each other, and a support information providing device that receives an input of the results of the measurement of the brain activities of the first subject and that outputs corresponding therapy information in accordance with results of classification by the clustering classifier for the measurement results.

Preferably, the processor is configured to, in the machine learning to generate the identifier model, generate a plurality of training sub-samples by executing under-sampling and sub-sampling from the first cohort and the second cohort, select features for clustering from a sum-set of features that are used to generate the identifier through the machine learning in accordance with a degree of importance of features that belong to the sum-set for each of the training sub-samples, and generate the clustering classifier by the multiple co-clustering method on the basis of the selected features for the clustering.

Preferably, the support information providing device includes a clustering processor and an interface device; the clustering processor calculates a probability at which the first subject is classified as belonging to each of the clusters by the clustering classifier, and reads at least two pieces of the therapy information selected in accordance with the probability from the database device; and the interface device outputs data for displaying the selected clusters and the corresponding pieces of the therapy information in association with each other.

Preferably, the therapy information is information that indicates a response to a specific therapeutic agent.

Preferably, the therapy information is information that indicates a response to a specific physical therapy.

Preferably, a process of generating the identifier through the machine learning involves ensemble learning in which a plurality of identifier sub-models are generated for the plurality of training sub-samples and the plurality of identifier sub-models are integrated with each other to generate the identifier model.

Preferably, the clustering device receives, from a plurality of brain activity measurement devices provided at a plurality of measurement sites, information that represents time correlation of brain activities among a plurality of predetermined brain area pairs for each of the plurality of second subjects.

Preferably, the processor includes harmonization calculation means for correcting the plurality of brain functional connectivity correlation values for each of the plurality of second subjects so as to remove a measurement bias of the measurement sites and storing corrected adjustment values in the storage device as the features.

Preferably, the predetermined therapy information is information related to a response to a therapy with a selective serotonin reuptake inhibitor.

According to an aspect of the invention, an embodiment of the present invention is directed to a therapy selection support device that provides information related to selection of a therapy for a first subject with depression symptoms on the basis of results of measurement of brain activities of the first subject. The therapy selection support device includes: a database device that stores clusters obtained as a result of stratification of subjects having a diagnosis label of a depression, among a plurality of second subjects, and corresponding predetermined therapy information in association with each other; and a support information providing device that receives an input of the results of measuring the brain activities of the first subject and that outputs corresponding therapy information in accordance with the result of the stratification based on the measurement results. The plurality of second subjects include a first cohort having a diagnosis label of a depression and a second cohort not having the diagnosis label of the depression. The clusters obtained as a result of the stratification are obtained by a clustering classifier obtained through a clustering process for results of measurement of brain functional connectivity correlation values by a clustering device. The clustering device includes a processor that executes the clustering process for the first cohort and a storage device. The processor is configured to, in a process of generating the clustering classifier, i) store, in the storage device, features based on a plurality of brain functional connectivity correlation values that represent time correlation of brain activities among a plurality of predetermined brain area pairs for each of the plurality of second subjects, ii) execute machine learning, through supervised learning, to generate an identifier model for discriminating presence or absence of the diagnosis label on the basis of the features stored in the storage device, iii) select features for clustering in accordance with a degree of importance of features that are used to generate an identifier through the machine learning in the machine learning to generate the identifier model, and iv) generate the clustering classifier by clustering the first cohort through a multiple co-clustering method as unsupervised learning on the basis of the selected features for the clustering.

Preferably, the processor is configured to, in the machine learning to generate the identifier model, generate a plurality of training sub-samples by executing under-sampling and sub-sampling from the first control cohort and the second control cohort, select features for clustering from a sum-set of features that are used to generate the identifier through the machine learning in accordance with a degree of importance of features that belong to the sum-set for each of the training sub-samples, and generate the clustering classifier by the multiple co-clustering method on the basis of the selected features for the clustering.

Preferably, the support information providing device includes a clustering processor and an interface device. The clustering processor calculates a probability at which the first subject is classified as belonging to each of the clusters by the clustering classifier, and reads at least two pieces of the therapy information selected in accordance with the probability from the database device. The interface device outputs data for displaying the selected clusters and the corresponding pieces of the therapy information in association with each other.

Preferably, the therapy information is information that indicates a response to a specific therapeutic agent.

Preferably, the therapy information is information that indicates a response to a specific physical therapy.

Preferably, a process of generating the identifier through the machine learning involves ensemble learning in which a plurality of identifier sub-models are generated for the plurality of training sub-samples and the plurality of identifier sub-models are integrated with each other to generate the identifier model.

Preferably, the clustering device receives, from a plurality of brain activity measurement devices provided at a plurality of measurement sites, information that represents time correlation of brain activities among a plurality of predetermined brain area pairs for each of the plurality of subjects; and the processor includes harmonization calculation means for correcting the plurality of brain functional connectivity correlation values for each of the plurality of subjects so as to remove a measurement bias of the measurement sites and storing corrected adjustment values in the storage device as the features.

Preferably, the predetermined therapy information is information related to a response to a therapy with a selective serotonin reuptake inhibitor.

According to an aspect of the invention, an embodiment of the present invention is directed to a therapy selection support method that provides information related to selection of a therapy for a first subject with depression symptoms on the basis of results of measurement of brain activities of the first subject. The therapy selection support method includes a preparing step of generating a clustering classifier that executes stratification in which results of measurement of brain functional connectivity correlation values acquired from a plurality of second subjects are divided into a plurality of clusters through a clustering process. The plurality of second subjects include a first cohort having a diagnosis label of a depression and a second cohort not having the diagnosis label of the depression. The preparing step includes a processing step of executing the clustering process for the plurality of second subjects. The processing step includes i) a step of acquiring features based on a plurality of brain functional connectivity correlation values that represent time correlation of brain activities among a plurality of predetermined brain area pairs for each of the plurality of second subjects, ii) a step of executing machine learning, through supervised learning, to generate an identifier model for discriminating presence or absence of the diagnosis label on the basis of the acquired features, iii) a step of selecting features for clustering in accordance with a degree of importance of features that are used to generate an identifier through the machine learning in the machine learning to generate the identifier model, and iv) a step of generating the clustering classifier by clustering the first cohort through a multiple co-clustering method as unsupervised learning on the basis of the selected features for the clustering. The therapy selection support method further includes a support information providing step of acquiring, from a database that stores the clusters obtained as a result of the stratification by the clustering classifier and corresponding predetermined therapy information in association with each other, and outputting corresponding therapy information in accordance with results of classification by the clustering classifier for the results of the measurement of the brain activities of the first subject.

Preferably, the predetermined therapy information is information related to a response to a therapy with a selective serotonin reuptake inhibitor.

According to an aspect of the invention, an embodiment of the present invention is directed to a therapy selection support method that provides information related to selection of a therapy for a first subject with depression symptoms on the basis of results of measurement of brain activities of the first subject. The therapy selection support method includes a support information providing step of acquiring, from a database that stores results of stratification of subjects having a diagnosis label of a depression, among a plurality of second subjects, and corresponding predetermined therapy information in association with each other, and outputting corresponding therapy information in accordance with clusters obtained as a result of stratification based on the results of the measurement of the brain activities of the first subject. The plurality of second subjects include a first cohort having a diagnosis label of a depression and a second cohort not having the diagnosis label of the depression. The clusters obtained as a result of the stratification are obtained by a clustering classifier obtained through a clustering process for results of measurement of brain functional connectivity correlation values. The clustering classifier is generated through a processing step of executing the clustering process for the plurality of second subjects. The processing step includes i) a step of acquiring features based on a plurality of brain functional connectivity correlation values that represent time correlation of brain activities among a plurality of predetermined brain area pairs for each of the plurality of second subjects, ii) a step of executing machine learning, through supervised learning, to generate an identifier model for discriminating presence or absence of the diagnosis label on the basis of the acquired features, iii) a step of selecting features for clustering in accordance with a degree of importance of features that are used to generate an identifier through the machine learning in the machine learning to generate the identifier model, and iv) generate the clustering classifier by clustering the first cohort through a multiple co-clustering method as unsupervised learning on the basis of the selected features for the clustering.

Preferably, the predetermined therapy information is information related to a response to a therapy with a selective serotonin reuptake inhibitor.

According to an aspect of the invention, an embodiment of the present invention is directed to a therapy selection support program that provides information related to selection of a therapy for a first subject with depression symptoms on the basis of results of measurement of brain activities of the first subject. The therapy selection support program causes a computer, when executed by the computer, to execute: a step of generating a clustering classifier that executes stratification in which results of measurement of brain functional connectivity correlation values acquired from a plurality of second subjects are divided into a plurality of clusters through a clustering process; and a step of receiving an input of the results of the measurement of the brain activities of the first subject and acquiring, from a database device that stores the clusters obtained as a result of the stratification by the clustering classifier and corresponding predetermined therapy information in association with each other, and outputting corresponding therapy information in accordance with results of classification by the clustering classifier for the measurement results. The plurality of second subjects include a first cohort having a diagnosis label of a depression and a second cohort not having the diagnosis label of the depression. The clustering process includes a processing step of executing the clustering process for the plurality of second subjects. The processing step includes i) a step of storing, in a storage device, features based on a plurality of brain functional connectivity correlation values that represent time correlation of brain activities among a plurality of predetermined brain area pairs for each of the plurality of second subjects, ii) a step of executing machine learning, through supervised learning, to generate an identifier model for discriminating presence or absence of the diagnosis label on the basis of the features stored in the storage device, iii) a step of selecting features for clustering in accordance with a degree of importance of features that are used to generate an identifier through the machine learning in the machine learning to generate the identifier model, and iv) a step of generating the clustering classifier by clustering the first cohort through a multiple co-clustering method as unsupervised learning on the basis of the selected features for the clustering.

Preferably, the predetermined therapy information is information related to a response to a therapy with a selective serotonin reuptake inhibitor.

According to an aspect of the invention, an embodiment of the present invention is directed to a therapy selection support program that provides information related to selection of a therapy for a first subject with depression symptoms on the basis of results of measurement of brain activities of the first subject. The therapy selection support program causes a computer, when executed by the computer, to execute a support information providing step of acquiring, from a database that stores results of stratification of subjects having a diagnosis label of a depression, among a plurality of second subjects, and corresponding predetermined therapy information in association with each other, and outputting corresponding therapy information in accordance with clusters obtained as a result of stratification based on the results of the measurement of the brain activities of the first subject. The clusters obtained as a result of the stratification are obtained by a clustering classifier obtained through a clustering process for results of measurement of brain functional connectivity correlation values. The plurality of second subjects include a first cohort having a diagnosis label of a depression and a second cohort not having the diagnosis label of the depression. The clustering classifier is generated through a processing step of executing the clustering process for the plurality of second subjects. The processing step includes i) a step of acquiring features based on a plurality of brain functional connectivity correlation values that represent time correlation of brain activities among a plurality of predetermined brain area pairs for each of the plurality of second subjects, ii) a step of executing machine learning, through supervised learning, to generate an identifier model for discriminating presence or absence of the diagnosis label on the basis of the acquired features, iii) a step of selecting features for clustering in accordance with a degree of importance of features that are used to generate an identifier through the machine learning in the machine learning to generate the identifier model, and iv) a step of generating the clustering classifier by clustering the first cohort through a multiple co-clustering method as unsupervised learning on the basis of the selected features for the clustering.

Preferably, the predetermined therapy information is information related to a response to a therapy with a selective serotonin reuptake inhibitor.

According to an aspect of the invention, an embodiment of the present invention is directed to a screening support system that supports screening of a first subject on the basis of results of measurement of brain activities of the first subject in a clinical test for a therapeutic means candidate for depression symptoms. The screening support system includes a clustering device that executes stratification in which results of measurement of brain functional connectivity correlation values acquired from a plurality of second subjects are divided into a plurality of clusters through a clustering process. The plurality of second subjects include a first cohort having a diagnosis label of a depression and a second cohort not having the diagnosis label of the depression. The clustering device includes a processor and a storage device, the processor being configured to execute the clustering process for the plurality of second subjects. The processor is configured to i) store, in the storage device, features based on a plurality of brain functional connectivity correlation values that represent time correlation of brain activities among a plurality of predetermined brain area pairs for each of the plurality of second subjects, ii) execute machine learning, through supervised learning, to generate an identifier model for discriminating presence or absence of the diagnosis label on the basis of the features stored in the storage device, iii) select features for clustering in accordance with a degree of importance of features that are used to generate an identifier through the machine learning in the machine learning to generate the identifier model, and iv) generate a clustering classifier by clustering the first cohort through a multiple co-clustering method as unsupervised learning on the basis of the selected features for the clustering. The screening support system further includes a support information providing device that receives an input of the results of the measurement of the brain activities of the first subject, that records results of classification by the clustering classifier for the measurement results in association with the first subject, and that outputs information for supporting the screening of the first subject based on the classification results.

Preferably, the processor is configured to, in the machine learning to generate the identifier model, generate a plurality of training sub-samples by executing under-sampling and sub-sampling from the first cohort and the second cohort, select features for clustering from a sum-set of features that are used to generate the identifier through the machine learning in accordance with a degree of importance of features that belong to the sum-set for each of the training sub-samples, and generate the clustering classifier by the multiple co-clustering method on the basis of the selected features for the clustering.

Preferably, the therapeutic means candidate is a therapy in which a selective serotonin reuptake inhibitor is used.

According to an aspect of the invention, an embodiment of the present invention is directed to a screening support device that supports screening of a first subject on the basis of results of measurement of brain activities of the first subject in a clinical test for a therapeutic means candidate for depression symptoms. The screening support device includes a support information providing device that includes a storage device that stores information for specifying a clustering classifier, that receives an input of the results of the measurement of the brain activities of the first subject, that records results of classification based on the clustering classifier for the measurement results in association with the first subject, and that outputs information for supporting the screening of the first subject based on the classification results. The clusters obtained as a result of the stratification are obtained by the clustering classifier obtained through a clustering process for results of measurement of brain functional connectivity correlation values by a clustering device. The clustering device includes a processor and a storage device, the processor being configured to execute the clustering process for a plurality of second subjects including a first cohort having a diagnosis label of a depression and a second cohort not having the diagnosis label of the depression. The processor is configured to, in a process of generating the clustering classifier, i) store, in the storage device, features based on a plurality of brain functional connectivity correlation values that represent time correlation of brain activities among a plurality of predetermined brain area pairs for each of the plurality of second subjects, ii) execute machine learning, through supervised learning, to generate an identifier model for discriminating presence or absence of the diagnosis label on the basis of the features stored in the storage device, iii) select features for clustering in accordance with a degree of importance of features that are used to generate an identifier through the machine learning in the machine learning to generate the identifier model, and iv) generate the clustering classifier by clustering the first cohort through a multiple co-clustering method as unsupervised learning on the basis of the selected features for the clustering.

Preferably, the therapeutic means candidate is a therapy in which a selective serotonin reuptake inhibitor is used.

According to an aspect of the invention, an embodiment of the present invention is directed to a screening support method that supports screening of a first subject on the basis of results of measurement of brain activities of the first subject in a clinical test for a therapeutic means candidate for depression symptoms. The screening support method includes: a step of a processor executing classification of the first subject in accordance with the results of the measurement of the brain activities on the basis of a clustering classifier specified by information stored in a storage device; and a step of recording results of the classification in association with the first subject and outputting information for supporting the screening of the first subject based on the classification results. A process of generating the clustering classifier includes a processing step of executing the clustering process for a plurality of second subjects including a first cohort having a diagnosis label of a depression and a second cohort not having the diagnosis label of the depression. The processing step includes i) a step of acquiring features based on a plurality of brain functional connectivity correlation values that represent time correlation of brain activities among a plurality of predetermined brain area pairs for each of the plurality of second subjects, ii) a step of executing machine learning, through supervised learning, to generate an identifier model for discriminating presence or absence of the diagnosis label on the basis of the acquired features, iii) a step of selecting features for clustering in accordance with a degree of importance of features that are used to generate an identifier through the machine learning in the machine learning to generate the identifier model, and iv) a step of generating the clustering classifier by clustering the first cohort through a multiple co-clustering method as unsupervised learning on the basis of the selected features for the clustering.

Preferably, the therapeutic means candidate is a therapy in which a selective serotonin reuptake inhibitor is used.

According to an aspect of the invention, an embodiment of the present invention is directed to a screening support program that supports screening of a first subject on the basis of results of measurement of brain activities of the first subject in a clinical test for a therapeutic means candidate for depression symptoms. The screening support program causes a computer, when executed by the computer, to execute: a step of a processor executing classification of the first subject in accordance with the results of the measurement of the brain activities on the basis of a clustering classifier specified by information stored in a storage device; and a step of recording results of the classification in association with the first subject and outputting information for supporting the screening of the first subject based on the classification results. A process of generating the clustering classifier includes a processing step of executing the clustering process for a plurality of second subjects including a first cohort having a diagnosis label of a depression and a second cohort not having the diagnosis label of the depression. The processing step includes i) a step of storing, in a storage device, features based on a plurality of brain functional connectivity correlation values that represent time correlation of brain activities among a plurality of predetermined brain area pairs for each of the plurality of second subjects, ii) a step of executing machine learning, through supervised learning, to generate an identifier model for discriminating presence or absence of the diagnosis label on the basis of the features stored in the storage device, iii) a step of selecting features for clustering in accordance with a degree of importance of features that are used to generate an identifier through the machine learning in the machine learning to generate the identifier model, and iv) a step of generating the clustering classifier by clustering the first cohort through a multiple co-clustering method as unsupervised learning on the basis of the selected features for the clustering.

Preferably, the therapeutic means candidate is a therapy in which a selective serotonin reuptake inhibitor is used.

Advantageous Effects of Invention

It is possible to support selection of a therapy for a subject that suffers from a depression and that requires treatment using a discriminator as a diagnostic marker or a classifier as a stratification marker generated through machine learning.

It is also possible to support screening of a subject that participates in a clinical test for a therapeutic agent candidate substance for depression symptoms using a discriminator as a diagnostic marker or a classifier as a stratification marker.

Brief Description of Drawings

Fig. 1 is a schematic diagram illustrating a harmonization process on data measured by MRI measurement systems installed at a plurality of measurement sites.
Fig. 2 is a schematic illustration showing a procedure for extracting a correlation matrix representing the correlation of functional connectivity in the resting state of Regions of Interest (ROIs) of a subject's brain.
Fig. 3 is a schematic illustration showing examples of "measurement parameters" and "subject attribute data."

Fig. 4 is a schematic diagram showing an overall configuration of an MRI device 100.i ($1 \leq i \leq Ns$) installed at respective measurement sites.

Fig. 5 is a hardware block diagram of a data processing unit 32.

Fig. 6 is an illustration showing a process of generating a discriminator to serve as a diagnostic marker from correlation matrixes and a clustering process.

Fig. 7 is a functional block diagram showing the configuration of a computing system 300.

Fig. 8 is a functional block diagram showing the configuration of a computing system 300.

Fig. 9 is a flowchart representing a procedure of machine learning for generating a disease identifier using ensemble learning.

Fig. 10 shows demographic characteristics of training dataset (Dataset 1).

Fig. 11 shows demographic characteristics of independent validation dataset (Dataset 2).

Fig. 12 shows the prediction performance (output probability distribution) of MDD on the training dataset for all imaging sites.

Fig. 13 shows the prediction performance (identifier output probability distribution) of the MDD on the training dataset for each imaging site.

Fig. 14 shows identifier output probability distributions of the MDD on the independent validation dataset.

Fig. 15 shows identifier output probability distributions of the MDD with respect to the independent validation dataset for each imaging site.

Fig. 16 is a flowchart representing a process of clustering by unsupervised learning by selecting features.

Fig. 17 shows a concept of performing feature selection when a plurality of (for example, Nch) features exist, by "learning with feature selection."

Fig. 18 is an illustration showing finally selected features when one identifier is generated by learning with feature selection.

Fig. 19 is an illustration showing how features are selected when an identifier is generated by performing under-sampling and sub-sampling processes a number of times.

Fig. 20 is an illustration showing a plurality of different manners of clustering depending on features.

Fig. 21 is an illustration showing a concept of clustering when a plurality of objects is characterized by a plurality of features.

Fig. 22 is an illustration showing a concept of multiple clustering and a concept of multiple co-clustering.

Fig. 23 is an illustration showing a concept in which probability models of different types of probability distributions are assumed in one view in "multiple co-clustering." Fig. 24 is a flowchart outlining a method of learning of multiple co-clustering.

Fig. 25 shows a graph expression of Bayesian inference in the method of learning of multiple co-clustering.

Fig. 26 shows Dataset 1 and Dataset 2 of a dataset divided into two.

Fig. 27 is an illustration showing a concept of clustering performed on each dataset.

Fig. 28 is an illustration showing an example of multiple co-clustering of subject data.

Fig. 29 shows results of multiple co-clustering actually performed on Datasets 1 and 2.

Fig. 30 shows, in the form of a table, counts of brain functional connectivity links (FCs) allocated to respective views of Datasets 1 and 2.

Fig. 31 illustrates methods of evaluating similarity of clustering (generalization performance of stratification).

Fig. 32 illustrates a concept of ARI.

Fig. 33 shows results of similarity evaluation between Clustering 1 and Clustering 1' and between Clustering 2 and Clustering 2'.

Fig. 34 shows, in the form of a table, distribution of subjects allocated to respective clusters of View 1 in Clustering 1 and Clustering 1'.

Fig. 35 is an illustration showing a method of evaluating the site-to-site differences of traveling subjects, who travel site-to-site to be subjected to measurements.

Fig. 36 is an illustration showing how to express the b-th functional connectivity of a subject a.

Fig. 37 is a flowchart showing a process of calculating a measurement bias for harmonization.

Fig. 38 is a functional block diagram showing an example in which the data collection, the estimating process, and the measurement of the brain activities of the object are processed in a distributed manner.

Fig. 39 shows the functional configuration of a therapy selection support system 1000.

Fig. 40 shows an example of a therapy information database 5100.

Fig. 41 is a flowchart showing the flow of a process of supporting selection of a therapy for a subject.

Fig. 42 shows a common drug discovery process.

Fig. 43 is a flowchart showing the flow of a process of supporting screening of a subj ect.

Fig. 44 shows the configuration of a screening support device 1000'.

Fig. 45 shows views of the results of clustering through multiple co-clustering for Dataset 1.

Fig. 46 shows views of the results of clustering through multiple co-clustering for Dataset 2.

Fig. 47 shows the stability of clustering between Dataset 1 and Dataset 2.

Fig. 48 shows views of the results of clustering for data on all depression patients (Datasets 1 + 2).

Fig. 49 shows the number of all depression patients and the number of depression patients with clinical data for each subtype for View 3 generated through clustering of Datasets 1 + 2.

Fig. 50 shows brain functional connectivity links (View 3) used in clustering.

Fig. 51 shows the relationship between the degree of severity of depression and the improvement rate in the degree of severity for each subtype in View 3.

Description of Embodiments

I. Training Phase

In the following, for the description of the present invention directed to "the brain functional connectivity correlation value clustering device," "the brain functional connectivity correlation value clustering method," and so on, "clustering" by artificial intelligence technique on brain functional connectivity image data of subjects (including patients of mental disease/psychiatric disorders) measured by a measurement system including a plurality of brain activity measuring devices will be taken as an example.

In the following, a configuration of the measurement system, more specifically an MRI measurement system, in accordance with the embodiments of the present invention will be described with reference to the drawings. In the embodiments below, components or process steps denoted by the same reference characters are the same or corresponding components or steps and, therefore, descriptions thereof will not be repeated unless necessary.

In the following embodiments, the present invention is applied for time-sequentially measuring brain activities between a plurality of brain regions using "brain activity measuring devices" or, more specifically, "MRI devices" installed at a plurality of facilities and for classifying subjects having a specific disorder into a plurality of groups (sub-groups) in a manner allowing generalization at a plurality of facilities, based on time-correlation patterns (referred to as "brain functional connectivity") of these regions.

Though not limiting, "major depressive disorder" will be taken as an example of the "specific disorder." As will be described in the following, however, the disease of subjects is not limited to "major depressive disorder," and it may be any other disorder or disease, as the present invention relates to a technique of classifying "brain functional connectivity correlation values" of subjects in a data-driven manner. Further, any attribute of subjects that can be classified in accordance with the patterns of "brain functional connectivity correlation values" of subjects may be used, other than the disorder.

In such "MRI measurement systems," a plurality of "MRI devices" are installed at a plurality of different facilities and inherently involve the site-to-site differences in measurements at the measurement facilities (measurement sites) including measurement bias derived from differences in measuring devices and differences between populations of subjects (sampling bias), which are independently evaluated as will be described later. Then, for each measurement value of each measurement site, a process is applied for correcting the site-to-site differences by removing the effects of measurement biases. Thus, the process of harmonizing the measurement results among the measurement sites (harmonization) is realized. Description will be made assuming that brain functional connectivity values after harmonization are subjected to "feature selection" by ensemble learning using diagnosis labels of a specific disorder as teacher data, followed by clustering through unsupervised learning, so as to classify subjects' attributes (such as subtypes of psychiatric disorder).

[First Embodiment]

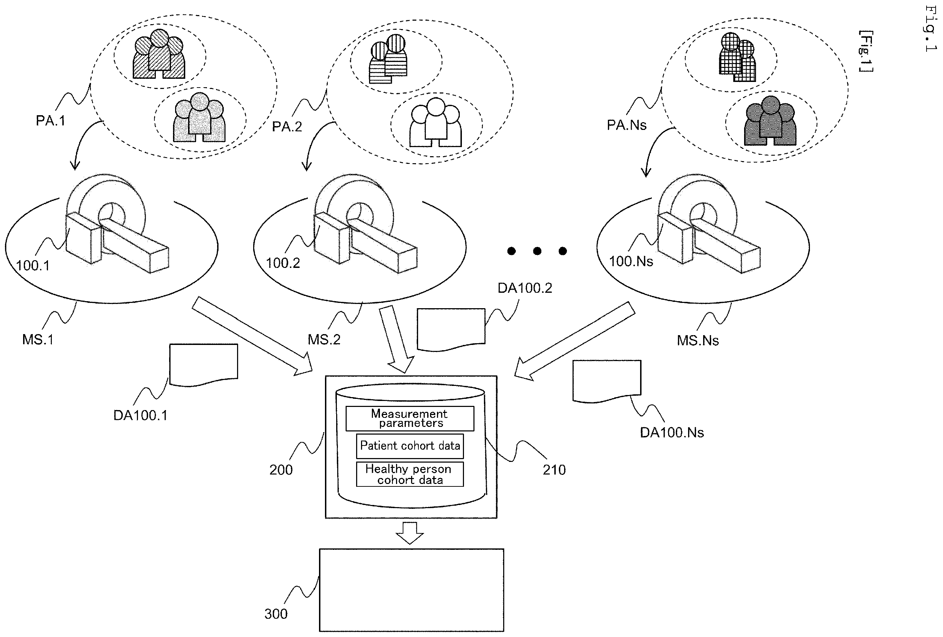

Fig. 1 is a schematic diagram illustrating a clustering (stratification) process of data measured by MRI measurement systems installed at a plurality of measurement sites.

Referring to Fig. 1, it is assumed that MRI devices 100.1 to 100.Ns are installed at measurement sites MS.1 to MS.Ns (Ns: number of sites), respectively.

At measurement sites MS.1 to MS.Ns, measurements of subject cohorts PA.1 to PA.Ns are taken. Herein, the subject cohorts PA.1 to PA.Ns are also referred to as second subject cohorts. People that belong to the second subject cohorts are also referred to second subjects. That is, the second subject cohorts include a plurality of second subjects. Each of the subject cohorts PA.1 to PA.Ns includes at least two cohorts to be classified, for example, a patient cohort and a healthy person cohort. The patient cohort is referred to as a first cohort herein when it is necessary to particularly differentiate the patient cohort from the healthy person cohort. Subjects that belong to the first cohort are subjects that have a diagnosis label of a depression on the basis of a diagnosis according to a diagnosis method such as the DSM-5, for example. The healthy person cohort is referred to as a second cohort herein when it is necessary to particularly differentiate the healthy person cohort from the patient cohort. Subjects that belong to the second cohort are subjects that do not have a diagnosis label of a depression. Though not limiting, a patient cohort includes patients of mental disease and, more specifically, a cohort of "patients of major depressive disorder." The "diagnosis label" determination method is not limited to the "symptom diagnosis method based on the DSM-5" according to the related art discussed above, and may be a determination method in which the results of analysis of measurement data on brain activities are determined as assistive information as described later, for example.

Further, it is assumed that at each measurement site, in principle, measurements of subjects are taken through measurement protocols as standardized as possible considering different specifications of MRI devices.

Though not specifically limited here, the measurement protocols define, by way of example, the following.

1) Direction of scanning subject's head

It is necessary to prescribe whether the head scan is to be done in a direction from the back side (posterior: hereinafter denoted as "P") to the front side (anterior: hereinafter denoted as "A") (in the following, this direction will be referred to as "P -> A direction") or the opposite direction, that is, from the front side to the back side (hereinafter referred to as "A → P direction"), for example. Circumstances may require scanning in both directions.

Default scanning direction may differ from MRI device to device. In some devices, it is impossible to freely set or change the scanning direction.

The scanning direction possibly defines how an image is "distorted" and, hence, condition is set as a protocol, for example.

2) Imaging conditions for brain structural images

Conditions are set for taking either "T1 weighted image" or "T2 weighted image" or both by a so-called spin echo.

3) Imaging conditions for brain functional images

Conditions are set for imaging brain functional images of subjects in the "resting state" by fMRI (functional Magnetic Resonance Imaging).

4) Imaging conditions for diffusion weighted images

Whether DWI (Diffusion (Weighted) Image) is to be obtained or not, and conditions therefor are set.

The diffusion weighted image is one type of MRI sequence, imaging diffusion of water molecules. In the spin echo pulse sequences usually used, signal attenuation caused by diffusion is negligible. When a large gradient magnetic field is continuously applied for a long time, however, phase shift caused by the movement of each magnetizing vector during that time becomes considerable and a region of more vigorous diffusion comes to appear as having lower signals. The diffusion weighted image utilizes this phenomenon.

5) Imaging for correcting EPI distortion by image processing

As a method of correcting EPI distortion by image processing, "field mapping" has been known and thus, conditions for imaging with respect to correction of spatial distortion are set.

In field mapping, EPI images are collected by multiple echo times, and the amount of EPI distortion is calculated based on these EPI images. By applying the field mapping, it is possible to correct EPI distortion included in a new image. On the premise of a set of images of the same anatomical structure with different echo time, EPI distortion can be calculated and image distortion can be corrected.

For example, "field mapping" is described in the reference below.

Reference 1: JP 2015-112474 A

Measurement protocols may include excerpts of necessary sequence portions of the conditions described above as appropriate, or may have other sequences or conditions added as needed.

Referring to Fig. 1 again, picking up subjects to be measured at respective measurement sites MS.1 to MS.Ns will be referred to as "sampling subjects," and the cause of the site-to-site differences of measurement values resulting from the bias in the sampling at various measurement sites will be referred to as a "sampling bias."

In the example above, it is known that patients diagnosed as having "major depressive disorder" on the basis of diagnostic criteria according to the related art actually include several subtypes.

Typical subtypes include "melancholic," "atypical," "seasonal" and "postnatal" depressions. Depression in which "clinically meaningful improvement was not observed following thorough treatment with the use of two or more antidepressants with different action mechanisms and middle or severe symptoms continue" is referred to as "treatment resistant depression," and, according to a report, this type may account for 10% to 20% of depressions. Specifically, a patient cohort generally diagnosed as having "major depression" is known to be far from homogeneous. To date, methods of classifying such subtypes based on objective measurement data have not been practically successful yet.

At the measurement sites, the distribution of subtypes among patients having "major depressive disorder" is not always uniform due to various factors such as bias in nature caused by the locality of patients visiting hospitals of the measurement sites and the tendency of diagnosis at the hospitals. As a result, it is common that the subtype distribution among patients of the various measurement sites is biased, and hence, the "sampling bias" mentioned above arises.

Further, even in a group of subjects referred to as "healthy person cohort," a plurality of subtypes exists in general, and in this point also, the "healthy person cohort" also has a "sampling bias."

Further, as regards MRI devices 100.1 to 100.Ns, it is not always the case that MRI devices having the same measurement characteristics are used at respective measurement sites.

The site-to-site differences among measurement data may be generated depending on the conditions of the MRI devices and the measurement conditions of the MRI devices, such as the manufacturers of the MRI devices, the model numbers of the MRI devices, the static magnetic field strength in the MRI devices, and the number of coils (number of channels) of (transmitting) receiving coils of the MRI devices. Site-to-site differences resulting from such measurement conditions are referred to as "measurement biases."

Even MRI devices of the same model number manufactured by the same manufacturer do not always realize perfectly identical measurement characteristics, due to the inherent uniqueness of each device.

Here, as the (transmitting) receiving coil, generally, a "multi-array coil" is used in order to improve the signal-to-noise ratio of measured signals. The "number of receiving coils" means the number of "element coils" forming the multi-array coil. The receiving sensitivity is improved by improving the sensitivity of each element coil and by bundling the outputs.

In the present embodiment, though not limiting, it becomes possible to independently evaluate the "sampling bias" and the "measurement bias" by the harmonization method as will be described later.

Referring back to Fig. 1, measurement-related data DA100.1 to DA100.Ns obtained from respective measurement sites MS.1 to MS.Ns are accumulated and stored in a storage device 210 in a data center 200.

Here, "measurement-related data" includes "measurement parameters" of respective measurement sites, as well as "patient cohort data" and "healthy person cohort data" measured at respective measurement sites.

Further, "patient cohort data" and "healthy person cohort data" include, for each subject, "patient MRI measurement data" and "healthy person MRI measurement data," respectively.

In the following, the "measurement-related data" as such will be described.

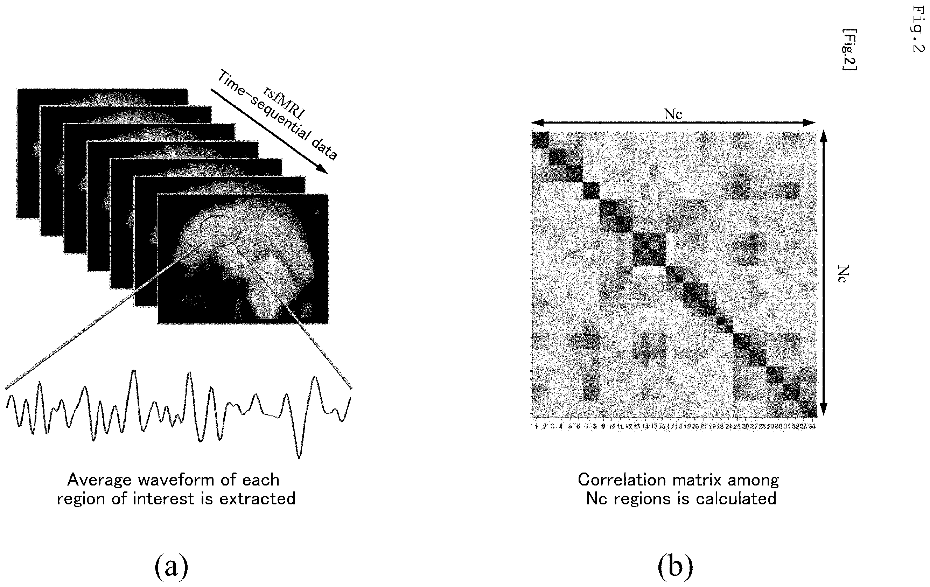

Fig. 2 is a schematic illustration showing a procedure for extracting a correlation matrix representing the correlation of functional connectivity in a resting state of Regions of Interest (ROIs) of a subject's brain.

Here, referring to Fig. 1, the "patient MRI measurement data" and the "healthy person MRI measurement data" in the "patient cohort data" and the "healthy person cohort data" include at least the following data.

    i) Time-sequential "brain function image data" for calculating a correlation matrix data and/or a correlation matrix data itself

Specifically, the data mentioned above is used as base data when computing system 300 shown in Fig. 1 calculates the brain activity biomarker as will be described later based on data stored in storage device 210.

Here, the correlation matrix data may be calculated at each measurement site based on time-sequential "brain function image data," and stored in storage device 210, and the computing system 300 may calculate the brain activity biomarker based on the data of the correlation matrix stored in storage device 210.

Alternatively, time-sequential "brain function image data" may be stored in storage device 210, and computing system 300 may calculate the correlation matrix data and further calculate the brain activity biomarker on the basis of the "brain function image data" stored in storage device 210.

Therefore, each of the "patient MRI measurement data" and the "healthy person MRI measurement data" at least includes either the time-sequential "brain function image data" for calculating the correlation matrix data, or the correlation matrix data itself.

ii) Subject structural image data and diffusion weighted image data

Though not specifically limited, the process for correcting EPI distortion through image processing may be done after the operation is done at each measurement site and the resulting data is stored in storage device 210.

Further, though not specifically limited, from the viewpoint of protecting personal information, an anonymization process may be performed at each measurement site before storing data in the storage device 210. The anonymization process, however, may be performed by computing system 300 if the operator of computing system 300 is legally authorized to handle personal information.

Returning to Fig. 2, as shown in Fig. 2(a), an average "degree of activity" of each region of interest is calculated from fMRI data of n (n: a natural number) time points in the resting state measured on a real-time basis, and as shown in Fig. 2(b), the correlation matrix of functional connectivity ("correlation value of activities") among the brain regions (among the regions of interest) is calculated.

(Parcellation of Brain Regions)

Functional connectivity is calculated as the time-wise correlation of blood oxygen level dependent (BOLD) signal of the resting state functional MRI between two brain regions of each participant.

Here, as the regions of interest, we assume Nr regions as mentioned above and, hence, the number of independent non-diagonal components in the correlation matrix will be $Nr \times (Nr - 1) / 2$, considering the symmetry.

The regions of interest may be set by the following methods.

Method 1) "Regions of interest are defined based on anatomical brain regions."

Here, for the brain activity biomarker, 140 regions are picked up as regions of interest.

As for ROIs, the cerebellums (left and right) and the vermis of the Automated Anatomical Labeling Atlas are used, in addition to 137 ROIs included in the Brain Sulci Atlas (BAL). The functional connectivity (FC) among these 140 ROIs is used as features.

Here, the Brain Sulci Atlas (BAL) and the Automated Anatomical Labeling Atlas are disclosed in the following references.

Reference 2: Perrot et al. Med Image Anal, 15 (4), 2011
Reference 3: Tzourio-Mazoyer et al. Neuroimage, 15 (1), 2002
Such ROIs include, by way of example, the following:

Dorsomedial Prefrontal Cortex (DMPFC);
Ventromedial Prefrontal Cortex (VMPFC);
Anterior Cingulate Cortex (ACC);
Cerebellar Vermis;
Left Thalamus;
Right Inferior Parietal Lobe;
Right Caudate Nucleus;
Right Middle Occipital Lobe; and
Right Middle Cingulate Cortex.

It is noted, however, that the brain regions used may not be limited to those above.

For instance, the regions to be selected may be changed in accordance with the neurological/mental disorder to be studied.

Method 2) "Functional connectivity is defined based on brain regions of a functional brain map covering the entire brain."

Here, the brain regions of such a functional brain map are disclosed in the references listed below and, though not limiting, configuration formed of 268 nodes (brain regions) may be adopted.

Reference 4: Noble S, et al. Multisite reliability of MR-based functional connectivity. Neuroimage 146, 959-970 (2017).

Reference 5: Finn ES, et al. Functional connectome fingerprinting: identifying individuals using patterns of brain connectivity. Nat Neurosci 18, 1664-1671 (2015).

Reference 6: Rosenberg MD, et al. A neuromarker of sustained attention from whole-brain functional connectivity. Nat Neurosci 19, 165-171 (2016).

Reference 7: Shen X, Tokoglu F, Papademetris X, Constable RT. Groupwise whole-brain parcellation from resting-state fMRI data for network node identification. Neuroimage 82, 403-415 (2013).

Method 3) Surface-based method

For parcellation of the brain regions, it is possible to analyze data by "surface-based method" based on a brain map prepared by converting the brain to sheets along cerebral sulcus, by using multimodal imaging of human connectome project (HCP) style (myelin task functional).

For such parcellation, the toolbox disclosed at the site below may be used (ciftify toolbox version 2.0.2). https://edickie.github.io/ciftify/#/

This toolbox enables the analysis of used data (even when T2 enhanced images necessary for the HCP pipeline are lacking) in a surface-based pipeline similar to the HCP.

In the analysis according to Method 3, 379 surface-based areas (360 cortex areas + 19 subcortical areas) disclosed in the reference below are used as regions of interest (ROIs).

Reference 8: Glasser, M.F., Coalson, T.S., Robinson, E.C., Hacker, C.D., Harwell, J., Yacoub, E., et al. (2016). A multi-modal parcellation of human cerebral cortex. Nature 536(7615), 171-178. doi: 10.1038/nature18933.

Therefore, changes of BOLD signals over time are extracted from these 379 regions of interest (ROI).

Further, anatomical names of important ROIs and the names of intrinsic brain networks including the ROIs are specified by the automated anatomical labeling (AAL) as disclosed in the reference below and by the use of Neurosynth (http://neurosynth.org/locations/).

Reference 9: Tzourio-Mazoyer, N., Landeau, B., Papathanassiou, D., Crivello, F., Etard, O., Delcroix, N., et al. (2002). Automated anatomical labeling of activations in SPM using a macroscopic anatomical parcellation of the MNI MRI single-subject brain. Neuroimage 15(1), 273-289. doi: 10.1006/nimg.2001.0978.

Method 4) Data-driven method of determining brain regions

As disclosed in the reference below, this is a method of newly identifying a network from in-phase voxels without previous knowledge (brain map), referred to as "Canonical ICA" or "dictionary learning."

Reference: Kamalaker Dadi, Mehdi Rahim, Alexandre Abraham, Darya Chyzhyk, Michael Milham, Bertrand Thirion, Gael Varoquaux, "Benchmarking functional connectome-based predictive models for resting-state fMRI", Preprint submitted to NeuroImage, October 31, 2018.

In the following, description will be made assuming that the method of defining functional connectivity based on the brain regions of the surface-based brain map basically in accordance with "Method 3" is used.

There are several candidates for measuring functional connectivity such as the tangent method and the partial correlation method as regards the calculation of correlation values.

In the following, however, we use Pearson's correlation coefficient, though it is not limiting.

For time-lapse of each of possible node sets of pre-processed BOLD signals, Pearson's correlation coefficient after Fisher z-transformation is calculated. The results are used for forming a $379 \times 379$ symmetrical connectivity matrix, each element of which represents the strength of connectivity between two nodes.

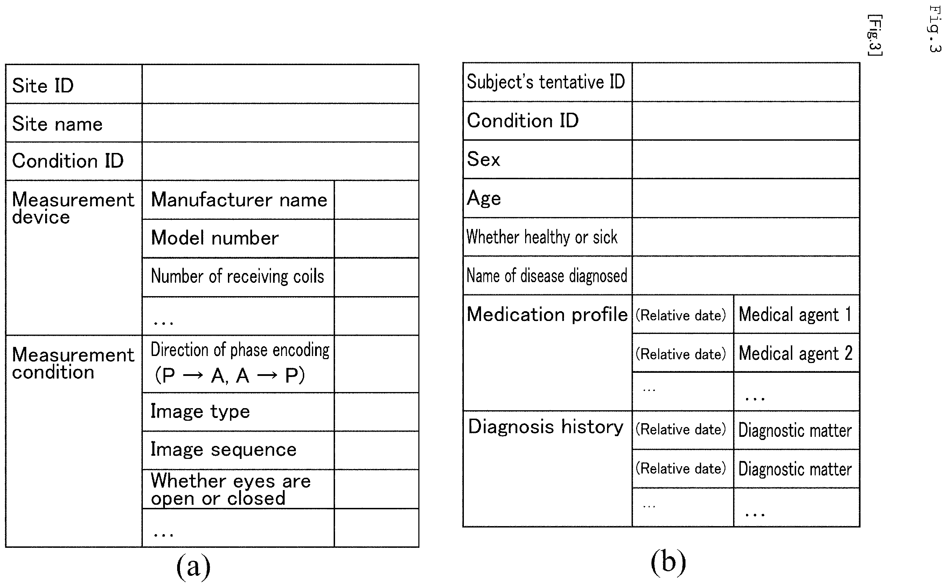

Fig. 3 is a schematic illustration showing examples of "measurement parameters" and "subject attribute data."

It is assumed that "subject attribute data" is stored associated with the "patient MRI measurement data" and the "healthy person MRI measurement data," respectively, in the "patient cohort data" and the "healthy person cohort data" of Fig. 1.

As shown in Fig. 3(a), the examples include a site ID for identifying the measurement site, a site name, a condition ID for identifying the measurement parameters, information related to the measuring device, and information related to the measurement conditions.

The "measurement parameters" include "information related to the measuring device" and "information related to measurement conditions."

The "information related to the measuring device" includes the name of the manufacturer, the model number, and the number of (transmitting) receiving coils of the MRI device for measuring brain activities of subjects at each measurement site.

The "information related to the measuring device" is not limited to these, and it may include static magnetic field strength, uniformity of magnetic field after shimming, and other indexes indicating performance of the measuring device, for example.

The "information related to measurement conditions" includes the direction of phase encoding when an image is re-constructed (P -> A or A -> P), type of image (T1 weighted, T2 weighted, diffusion weighted, etc.), imaging sequence (spin echo or the like), whether the subject's eyes are open/closed during imaging, and so on.

The "information related to measurement conditions" is not limited to these, either.

Referring to Fig. 3(b), the "subject attribute data" includes the subject's tentative ID as a pseudonym to prevent identification of the subject, condition ID indicating the measurement conditions when the subject was measured, and the attribute information of the subject.

The "subject attribute information" includes sex and age of the subject, a label indicating either healthy or sick, the disease name of the subject as diagnosed by a doctor, medication profile and diagnosis history of the subject, and so on.

The "subject attribute information" may be anonymized as needed at, for example, the measurement site.

By way of example, as to the age and sex, the subject's attribute information may be processed to maintain "k-anonymity" that reduces the probability of identifying an individual to 1/k or smaller by transforming data such that at least k data of "quasi-identifier" (same attribute) exist. Here, "quasi-identifier" refers to an attribute such as "age," "sex," and "place of residence" that cannot identify an individual by itself but allows identification of an individual when combined.

Medication profile and diagnosis history may be subjected to anonymization as needed, by randomizing or shifting (relativizing) the dates.

In the following, in the "patient MRI measurement data" and the "healthy person MRI measurement data," the functional connectivity calculated by the above-described method as the correlation of temporal activities between each of the brain regions of each subject will be generally referred to as "functional connectivity" (or "FC" for short) between each brain region. If it is necessary to distinguish one functional connectivity from another for each brain region, a suffix will be added, as will be described later.

(Configuration of MRI Device)

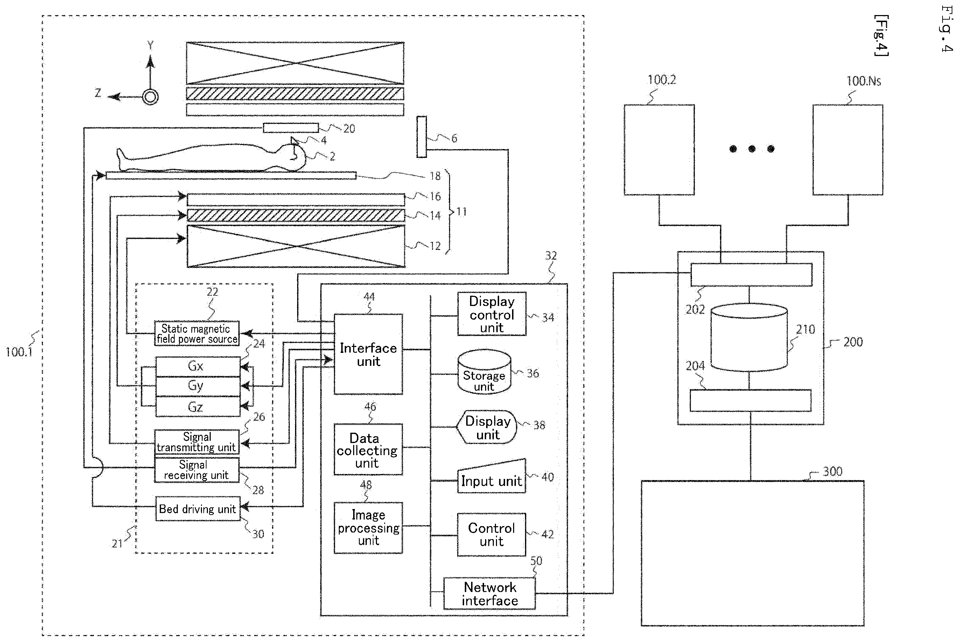

Fig. 4 is a schematic diagram showing an overall configuration of an MRI device 100.i ($1 \leq i \leq Ns$) installed at respective measurement sites.

In Fig. 4, MRI device 100.1 at the first measurement site is shown in detail as an example. Other MRI devices 100.2 to 100.Ns also have similar basic configurations.

As shown in Fig. 4, MRI device 100.1 includes: a magnetic field applying mechanism 11 for applying a controlled magnetic field to, and irradiating with RF wave, a region of interest of a subject 2 (which may be a first subject or a second subject); a receiving coil 20 for receiving a response wave (NMR signal) from subject 2 and outputting an analog signal; a driving unit 21 for controlling the magnetic field applied to subject 2 and controlling the transmission/reception of RF wave; and a data processing unit 32 for configuring a control sequence of driving unit 21 and processing various data signals to generate an image.

Here, the central axis of a cylindrical bore in which subject 2 is placed is regarded as a Z-axis, and a horizontal direction orthogonal to the Z-axis and the vertical direction orthogonal to the Z-axis are defined as X-axis and Y-axis, respectively.

In MRI device 100.1 having such a configuration, because of the static magnetic field applied by magnetic field applying mechanism 11, nuclear spins of atomic nuclei forming subject 2 are oriented in the direction of magnetic field (Z-axis) and precess about the direction of magnetic field, with the Larmor frequency unique to the atomic nuclei.

When irradiated with an RF pulse of the same Larmor frequency, the atoms resonate, absorb energy, and are excited, resulting in nuclear magnetic resonance (NMR). When the irradiation with RF pulse is stopped after the resonance, the atoms discharge energy and return to the original, steady state in a relaxation process. In the relaxation process, the atoms output electromagnetic wave (NMR signal) having the same frequency as the Larmor frequency.

The output NMR signal is received by receiving coil 20 as a response wave from subject 2, and the regions of interest of subject 2 are imaged by data processing unit 32.

Magnetic field applying mechanism 11 includes a static magnetic field generating coil 12, a magnetic field gradient generating coil 14, an RF irradiating unit 16, and a bed 18 for placing subject 2 in the bore.

By way of example, subject 2 lies on his/her back on bed 18. Though not limiting, subject 2 may view a screen displayed on a display 6 mounted perpendicular to the Z-axis using prism glasses 4, for example. Visual stimulus may be applied to subject 2 by an image on display 6 as needed. Alternatively, visual stimulus to subject 2 may be applied by projecting an image in front of subject 2 using a projector.

Such a visual stimulus corresponds to the presentation of feedback information when neurofeedback is given to the subject.

Driving unit 21 includes a static magnetic field power source 22, a magnetic field gradient power source 24, a signal transmitting unit 26, a signal receiving unit 28, and a bed driving unit 30 for moving bed 18 to any position along the Z-axis.

Data processing unit 32 includes: an input unit 40 for receiving various operations and information input from an operator (not shown); a display unit 38 for displaying various images and various pieces of information related to the regions of interest of subject 2; a storage unit 36 for storing programs, control parameters, image data (structural images and the like), and other electronic data to cause execution of various processes; a control unit 42 for controlling operations of various functional units, including generating a control sequence for driving the driving unit 21; an interface unit 44 for performing transmission/reception of various signals to/from driving unit 21; a data collecting unit 46 for collecting data consisting of a group of NMR signals derived from the regions of interest; an image processing unit 48 for forming an image based on the data of NMR signals; and a network interface 50 for performing communication with a network.

Data processing unit 32 may be a dedicated computer, or it may be a general-purpose computer programmed to perform functions causing operations of various functional units, in which designated operations, data processing, and generation of control sequence may be realized by a program or programs installed in storage unit 36. In the following, description will be given assuming that data processing unit 32 is implemented by a general-purpose computer.

Static magnetic field generating coil 12 causes a current supplied from a static magnetic field power source 22 to flow through a helical coil wound around the Z-axis to generate an induction magnetic field, and thereby generates a static magnetic field in the Z-direction in the bore. The regions of interest of subject 2 are placed in the region of highly uniform static magnetic field formed in the bore. More specifically, here, static magnetic field generating coil 12 is comprised of four air core coils, forms a uniform magnetic field inside by the combination of the coils, and attains orientation of the spins of prescribed atomic nuclei in the body of subject 2, or more specifically, the spins of hydrogen atomic nuclei.

Magnetic field gradient generating coil 14 is formed of X-, Y-, and Z-coils (not shown), and provided on an inner peripheral surface of cylindrical static magnetic field generating coil 12.

In order to improve uniformity of magnetic field gradient, a shim coil (not shown) is used and "shimming" is performed.

These X-, Y-, and Z- coils superpose magnetic field gradients on the uniform magnetic field in the bore with the X-axis, Y-axis, and Z-axis directions switched in turn, whereby creating intensity gradient in the static magnetic field. When excited, the Z-coil tilts the magnetic field intensity to the Z-direction and thereby defines a resonance surface; the Y-coil applies a tilt for a short period of time immediately after application of the magnetic field in the Z-direction, and thereby adds phase modulation in proportion to the Y-coordinate, to the detected signal (phase encoding); and thereafter the X-coil applies a tilt when data is collected, and thereby adds frequency modulation in proportion to the X-coordinate, to the detected signal (frequency encoding).

The switching of superposed magnetic field gradients is realized as different pulse signals are output to the X-, Y-, and Z-coils from the transmitting unit 24 in accordance with a control sequence. Thus, the position of subject 2 expressed by the NMR can be specified, and positional information in three-dimensional coordinates necessary to form an image of subject 2 are provided.

Here, images can be taken from various angles using the orthogonally crossing three sets of magnetic field gradients as described above, by allocating slice direction, phase encoding direction, and frequency encoding direction to the magnetic fields respectively and combining these. By way of example, in addition to transverse slice in the same direction as taken by an X-ray CT apparatus, sagittal and coronal slices orthogonal thereto, as well as an oblique slice, of which direction perpendicular to its plane is not parallel to any of the axes of three orthogonally crossing magnetic field gradients, can be imaged.

RF irradiating unit 16 irradiates regions of interest of subject 2 with RF (Radio Frequency) pulses based on a high-frequency signal transmitted from signal transmitting unit 26 in accordance with a control sequence.

Though RF irradiating unit 16 is built in magnetic field applying mechanism 11 in Fig. 1, it may be mounted on bed 18 or integrated with receiving coil 20 as a transmitting/receiving coil 20.

Receiving coil 20 detects a response wave (NMR signal) from subject 2, and in order to detect the NMR signal with high sensitivity, it is arranged close to subject 2.

Here, when an electromagnetic wave of NMR signal crosses a coil strand of receiving coil 20, a weak current is generated by electromagnetic induction. The weak current is amplified by signal receiving unit 28 and converted from an analog signal to a digital signal, and then transmitted to data processing unit 32.

As the (transmitting) receiving coil 20, a multi-array coil is used for improving the SN ratio, as described above.

The mechanism here is as follows. A high-frequency electromagnetic field of resonance frequency is applied through RF irradiating unit 16 to a subject 2 in a state of static magnetic field with Z-axis magnetic field gradient added. Prescribed atomic nuclei, for example, hydrogen atomic nuclei, at a portion where magnetic field intensity satisfies the condition of resonance are selectively excited and start resonating. Prescribed atomic nuclei at a portion satisfying the condition of resonance (for example, a slice of prescribed thickness of subject 2) are excited, and (in a classic image drawing) spin axes concurrently start rotation. When the excitation pulse is stopped, electromagnetic waves irradiated by the spin axes in rotation induce a signal in receiving coil 20 and, for some time, this signal is continuously detected. A tissue containing the prescribed atoms in the body of subject 2 is monitored in accordance with this signal. In order to know the position where the signal comes from, X- and Y-magnetic field gradients are added and the signal is detected.

Based on the data built in storage unit 36, image processing unit 48 measures detected signals while repeatedly applying excitation signals, reduces resonance frequency to X-coordinate by a first Fourier transform, restores Y-coordinate by a second Fourier transform to obtain an image, and thus, displays the corresponding image on display unit 38.

For example, by picking-up the BOLD signal on a real-time basis using the MRI system as described above and performing an analysis, which will be described later, on the time-sequentially picked-up images by control unit 42, it is possible to take images of the resting-state functional connectivity MRI (rs-fcMRI).

In Fig. 4, measurement data, measurement parameters, and subject attribute data from MRI device 100.1 as well as from MRI devices 100.2 to 100.Ns at other measurement sites are accumulated and stored in storage device 210 through a communication interface 202 in data center 200. Further, computing system 300 is configured to access data in storage device 210 through communication interface 204.

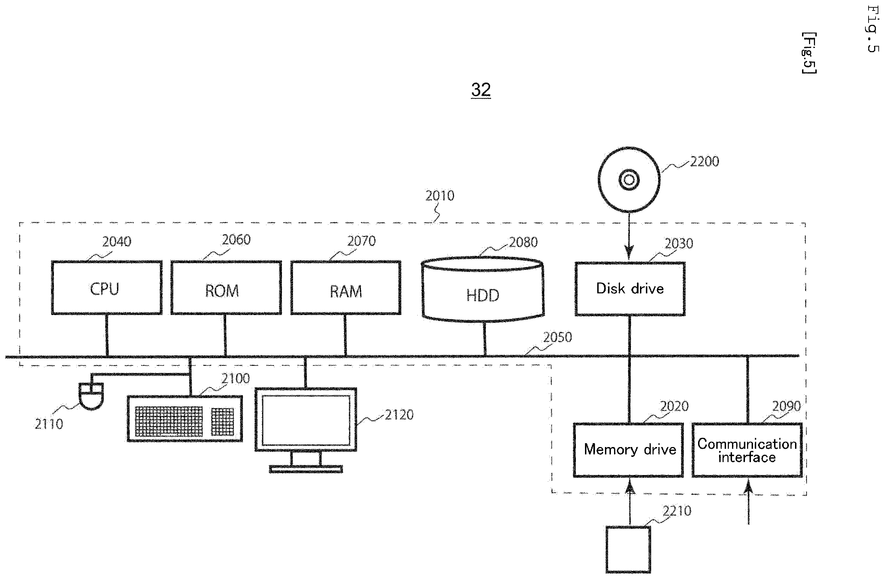

Fig. 5 is a hardware block diagram of data processing unit 32.

Though the hardware of data processing unit 32 is not specifically limited as described above, a general-purpose computer may be used.

Referring to Fig. 5, a computer main body 2010 of data processing unit 32 includes, in addition to a memory drive 2020 and a disk drive 2030, a processor (CPU) 2040, a bus 2050 connected to disk drive 2030 and memory drive 2020, a ROM 2060 for storing programs such as a boot-up program, a RAM 2070 for temporarily storing instructions of an application program and providing a temporary memory space, a non-volatile storage device 2080 for storing application programs, system programs, and data, and a communication interface 2090. Communication interface 2090 corresponds to an interface unit 44 for transmitting/receiving signals to/from driving unit 21 and the like and a network interface 50 for communicating with another computer through a network (not shown). As non-volatile storage device 2080, a hard disk (HDD), a solid-state drive (SSD), or the like may be used. Non-volatile storage device 2080 corresponds to storage unit 36.

Various functions of data processing unit 32 including, for example, functions of control unit 42, data collecting unit 46, and image processing unit 48 are realized by operation processes performed by CPU 2040 in accordance with a program.

A program or programs causing data processing unit 32 to perform the functions of the present embodiment as described above may be stored in a DVD-ROM 2200 or a memory medium 2210 and inserted to disk drive 2030 or memory drive 2020 and may be further transferred to non-volatile storage device 2080. The program or programs will be loaded to RAM 2070 when to be performed.

Data processing unit 32 further includes a keyboard 2100 and a mouse 2110 as input devices, and a display 2120 as an output device. Keyboard 2100 and mouse 2110 correspond to input unit 40, and display 2120 corresponds to display unit 38.

The program or programs realizing the functions as data processing unit 32 as described above may not necessarily include an operating system (OS) for causing computer main body 2010 to perform the functions of information processing apparatus and the like. The program or programs may only include those portions of instructions which can call appropriate functions (modules) in a controlled manner to attain a desired result. The manner how data processing unit 32 operates is well known and, therefore, detailed description will not be given here.

Further, the above-described program or programs may be performed by one computer or by a plurality of computers. In other words, both centralized processing and distributed processing are possible.

Hardware of computing system 300 has basically the same configuration as that shown in Fig. 5, though there may be some difference such as use of parallel processing units or use of GPGPU (General-Purpose computing on graphic processing units).

(Diseased/Healthy Discriminator Generating Process and Clustering Process Based on Brain Functional Connectivity)

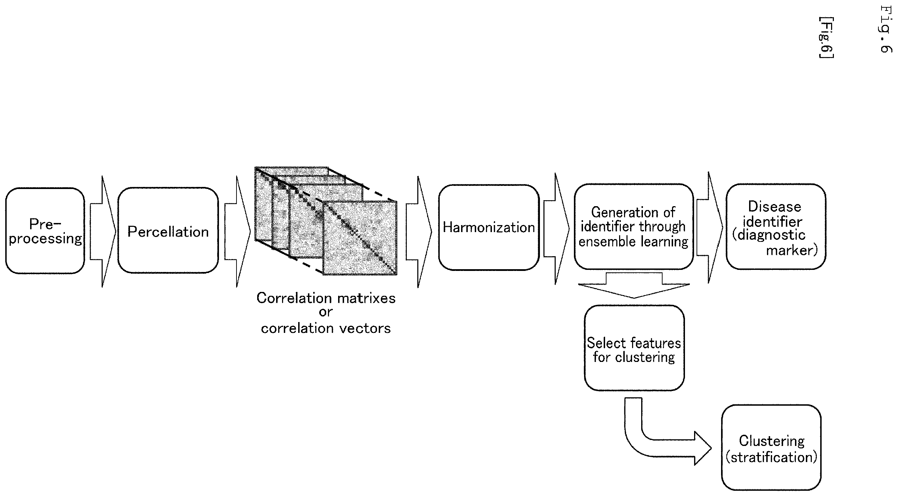

Fig. 6 is an illustration showing a process of generating a discriminator to serve as a diagnostic marker from correlation matrixes as described with reference to Fig. 2 and a clustering process.

Regarding machine learning, generation of a discriminator involves a so-called "supervised learning" process, and clustering involves "unsupervised learning."

The clustering process itself is realized by "unsupervised learning" and it does not use diagnostic information of medical doctors. Therefore, each of the resulting clusters represents a group of patients obtained in data-driven manner. If the patients are divided to subtypes, the results serve as a basis for "stratification of patients" having brain functional connectivity as a feature.

As shown in Fig. 6, first, fMRI image data in the resting state of healthy person cohorts and patient cohorts are taken by a plurality of MRI devices, and computing system 300 performs "pre-processing" as described below on the fMRI image data thus obtained. Thereafter, from the measured data of functional connectivity MRI data in the resting state, computing system 300 performs parcellation of brain regions of each subject, and derives correlation matrix of activities between brain regions (regions of interest).

Then, for non-diagonal components of the correlation matrix, corresponding measurement bias is derived as described later. The computing system 300 subtracts the measurement bias from component values of the correlation matrix and, thus, realizes harmonization.

Further, between the harmonized component values of correlation matrix and the disease label of each subject (label indicating sick or healthy), computing system 300 performs generation of an identifier with feature selection while preventing overtraining as "identifier generating process through ensemble learning" as will be described later. Thus, a disease identifier (diagnosis marker) capable of predicting whether a subject is healthy or has a disease is generated.

On the other hand, during the ensemble learning, computing system 300 performs feature selection for clustering as will be described later from the features (brain functional connectivity) specified in the process of generating the identifier and then, performs multiple co-clustering by "unsupervised learning."

In the following, each of the processes shown in Fig. 6 will be described in greater detail.

[Outline of Preprocessing, Generation of Disease Identification, and Clustering]

(Preprocessing and Calculation of Resting State Functional Connectivity FC Matrix)

The first 10 seconds of measured fMRI data are discarded to take the T1 equilibrium into consideration.

At the preprocessing step, the computing system 300 performs the calibration of slice timing, the re-alignment process for correcting body motion artifacts observed at one's head, co-registration of the brain functional image (EPI image) and the morphological image, the distortion correction, segmentation of the T1 enhanced structural image, the normalization to the MNI (Montreal Neurological Institute) space, and spatial smoothing using the isotropic Gaussian kernel of 6 mm full-width at half maximum, for example.

The above-described pipeline pre-processing is disclosed, for example, at the site below.

http://fmriprep.readthedocs.io/en/latest/workflows.html

(Parcellation of Brain Regions)

For parcellation of the brain regions, the "surface-based method" may be utilized in accordance with "Method 3" described above, though not limiting.

(Physiological Noise Regression)

Physiological noise regression is performed by applying the CompCor disclosed in the reference below.

Reference: Behzadi, Y., Restom, K., Liau, J., and Liu, T.T. (2007). A component based noise correction method (CompCor) for BOLD and perfusion based fMRI. Neuroimage 37(1), 90-101. doi: 10.1016/j.neuroimage.2007.04.042.

In order to remove some sources of spurious (undesirable signal sources), the linear regression with regression parameters such as six kinematic parameters, whole brain, etc. is used.

(Temporal Filtering)

Computing system 300 applies a temporal bandpass filter to the time-sequential data using Butterworth filter having pass bands between 0.01 Hz and 0.08 Hz, for example, so that the analysis is limited to low-frequency fluctuation characteristic of the BOLD behavior.

(Head Movement)

Frame-wise displacement (FD) is calculated in each functional session, and in order to reduce variation in spurious of the functional connectivity FC caused by head movement, any volume having FD > 0.5 mm is removed, for example.

FD represents head movement between two temporally continuous volumes as a scalar quantity (that is, the sum of absolute displacements in translation and rotation).

In a specific example described later, for example, if the ratio of volume removed after scrubbing exceeds (average $\pm$ 3 standard deviation) in the specific dataset as mentioned above, the data of the corresponding participant is excluded from the analysis. As a result, thirty-five participants were excluded for the whole dataset. Hence, 683 participants (545 for HC and 138 for MDD) from the training dataset were used, and the data of 444 participants (263 for HC and 181 patients for MDD) from the independent validation dataset was used for the analysis below.

(Calculation of Functional Connectivity (FC) Matrix)

In a specific example in accordance with the present embodiment, after the regions are divided by the above-described parcellation, the functional connectivity FC is calculated as a temporal correlation of the BOLD signals over 379 regions of interest (ROIs) for each participant.

Though not limiting, the Pearson's correlation coefficient is used for calculating functional connectivity.

The Pearson's correlation coefficient subjected to Fisher z-transformation of pre-processed BOLD signals over time of each possible set of ROIs is calculated, and a symmetrical connectivity matrix of 379 rows $\times$ 379 columns, of which components each represent the strength of connection between two ROIs, is formed.

Further, for reasons of analysis, the values of the functional connectivity FC of the lower triangular matrix of 71,631 (= (379 $\times$ 378)/2) of the connectivity matrix are used.

(Harmonization of Brain Activity Biomarkers)

When big data associated with mental diseases or psychiatric disorders is to be collected, it is nearly impossible, as is stated above, to collect massive brain image data (connectomes related to human disease) at one site. Therefore, it is necessary to acquire image data from a plurality of sites.

It is difficult to regulate types of MRI devices (scanners), protocols, and patient segments. Therefore, brain image data picked up under different conditions are used when analyzing collected data.

Particularly, disease factors tend to be confounded with site factors and, therefore, when disease factors are to be extracted by applying machine learning to data obtained under different conditions, the site-to-site differences pose the biggest barrier.

One site (or a hospital) tends to sample only a few types of mental diseases (for example, mainly schizophrenia at site A, mainly autism at site B, and mainly major depressive disorder at site C), leading to confounding.

In order to regulate data under different conditions appropriately, the harmonization of the site-to-site data is indispensable.

The site-to-site differences essentially include two types of biases.

Specifically, a technical bias (or measurement bias) and a biological bias (or sampling bias).

The measurement bias involves differences in imaging parameters, electric field strength, and the characteristics of MRI scanners such as the MRI manufacturers and the scanner models. The sampling bias relates to differences of subject cohorts from one site to another.

Hence, "harmonization" to compensate for such site-to-site difference becomes necessary. Details of harmonization are described in Non-Patent Literature 8 (Ayumu Yamashita et al.) listed above, of which contents will be discussed later.

(Disease Identifiers Based on Ensemble Learning)

In the present specification, the "ensemble learning" refers to a process of preparing K sets of training data by sampling with replacement from original training data, generating K identifiers independently for respective training data through machine learning, integrating these K identifiers, and thereby generating a discriminator.

It is noted that the object here is to determine whether a subject is healthy or has a specific disease based on brain functional connectivity patterns of the subject. Therefore, each identifier will be an identifier that serves to solve the problem of identifying two classes.

When K sets of training data are to be prepared by sampling with replacement from original training data, "under sampling" and "sub-sampling" are performed, as will be described later.

Here, "identifier realized through learning with feature selection" such as an identifier using LASSO (least absolute shrinkage and selection operator) by L1 regularization, or regularization learning such as Ridge regularization (L2 regularization) may be applicable.

Here, "regularization learning" refers to a method of learning that, while using all the features of original training data, assigns a penalty, in a learning algorithm, to increased model complexity during learning, and determines the training model having the minimum sum of training error and the penalty, thereby improving generalization performance. L1 regularization uses sum of absolute values of training model parameters (corresponding to the features) as the penalty, while L2 regularization uses sum of squares of training model parameters as the penalty. L0 regularization is also possible, in which the number of features themselves used for the model is used as the penalty.

Further, LASSO method (L1 regularization) allows for a so-called sparse estimation, and its derivatives include Elastic Net method, Group Lasso method, Fused Lasso method, Adaptive Lasso method, and Graphical Lasso method.

On the other hand, as the "identifier trained with feature selection," it is possible to use a method such as "random forest method" which not only selects features but also specifies their respective importance in generating an identifier.

Hereafter, description will be made in the following mainly taking LASSO method as an example of "identifier generating process through ensemble learning." It is noted, however, that the method is not limited to those described above. By way of example, the parcellation method may be such an ensemble learning wherein Dictionary Learning method is used in configuring brain regions as the object of analysis in a data-dependent manner, tangent-space covariance is used as values of functional brain connectivity, ComBat method, which will be described later, is used in realizing facility-to-facility correction of brain functional connectivity FC in a dataset, and Ridge regularization is used to generate an identifier. Other combinations may be adopted as the parcellation method, the method of calculating brain functional connectivity, the harmonization method, and the method of generating an identifier.

As will be described later, in the present embodiment, the ensemble training specifies the "importance" of each feature for achieving the function of identification during the identifier training.

(Feature Selection for Clustering and Clustering)

In the process of generating K identifiers by the "ensemble learning" for the K sets of training data, a process is performed for specifying, from a sum set of "first features" used in generating each identifier, the second set of features for performing clustering through "unsupervised learning."

Particularly, though not limiting, the "importance" is determined in the following manner.

i) If the method of learning for generating K identifiers in the ensemble learning is "learning with feature selection," features are ranked in accordance with the frequency of use for generating K identifiers in the sum set of "first features" selected for generating the identifiers.

ii) If the method of learning for generating K identifiers in the ensemble learning is a method in which importance of features can be obtained during the generation of identifiers, such as in the case of "random forest method," the features may be ranked and listed in accordance with the importance thus obtained.

iii) If the method of learning for generating K identifiers in the ensemble learning is "Ridge regularization (L2 regularization)" (that does not necessarily involve feature selection) using weighted sum of features as an argument, the features are ranked and listed using, as the importance, the median of absolute values of weight coefficients of features in each identifier calculated for K identifiers. The importance is not necessarily limited to the "median" and any other statistical representative value, such as "integrated value obtained by integrating K identifiers" may be used, for example.

It is possible to use a prescribed number of upper-ranked features in the ranking list generated in accordance with the methods i) to iii) as the "second feature."

The condition for specifying the "second feature" is not limited to the prescribed number from the top of the ranking list. For example, a feature that has a prescribed frequency or higher in the ranking list (the fact that the feature is selected at a certain rate or higher in generating K identifiers) may be used as a condition.

As described above, clustering (stratification of patients) is performed through "multiple co-clustering" which is an unsupervised learning, as will be described later, based on the selected feature(s).

[Process for Generating Identifier for Classifying two Classes]

In the following, among the processes described with reference to Fig. 6, generation of an identifier through ensemble learning will be described in greater detail.

Specifically, the process for generating a classifier for classifying two classes, or more specifically, a process of forming a biomarker for MDD using training dataset as the training data for a disease identifier (two-class classifier for "healthy" and "sick") will be described as an example.

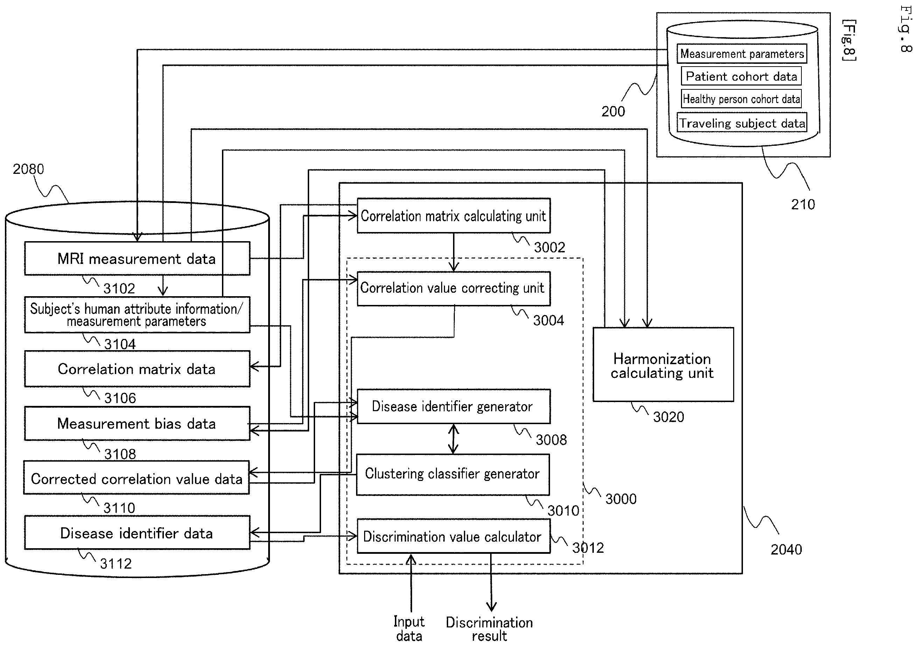

Here, the process of generating a classifier will be described concerning major depressive disorders as an example among mental diseases or psychiatric disorders, i.e., concerning a group of patients diagnosed as having major depressive disorder by doctors in accordance with the conventional method of symptom-based diagnosis approach. Then, an example will be given in the following concerning the process performed by the disease identifier generator 3008 shown in Fig. 8 to generate a classifier that outputs assisting information for discriminating a patient cohort from a healthy person cohort.

A procedure of building an MDD identifier for identifying a healthy person (HC) and an MDD patient based on the functional connectivity FC will be described in the following.

In the following, the method of training an identifier by L1 regularization (LASSO method), as an example of the "identifier realized through learning with feature selection" for forming a disease identifier (MDD identifier), will be described.

As will be described later, in order to specify a functional connectivity FC related to MDD diagnosis, features used for clustering are selected in accordance with the "importance" to the construction of disease identifier of each functional connectivity FC.

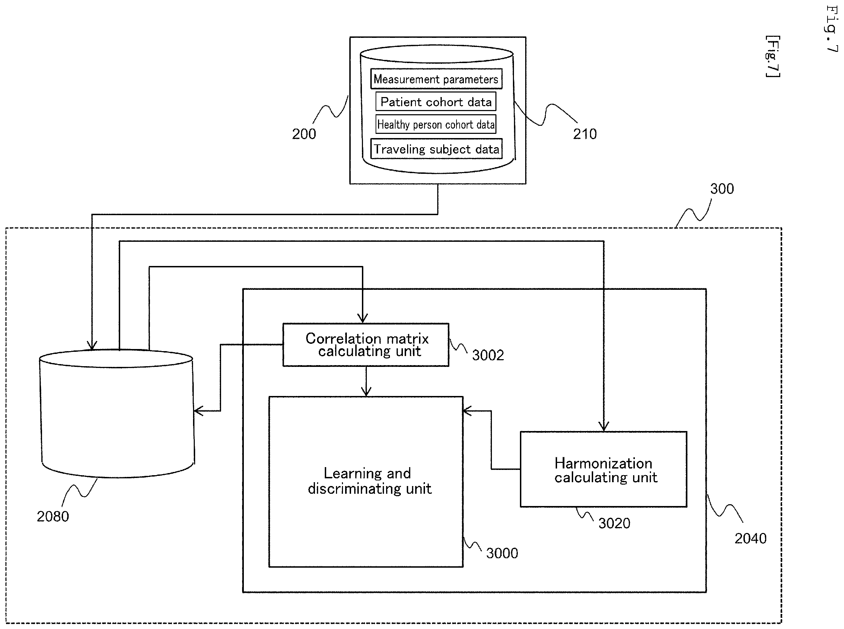

Figs. 7 and 8 are functional block diagrams showing configurations of a computing system 300 performing a harmonization process, a disease identifier generating process, a clustering classifier generating process, and a discriminating process based on the data stored in storage device 210 of data center 200.

Here, it is assumed that the "discriminating process" includes discrimination of disease (whether the subject has a disease or is healthy) and a classifying process of determining to which "cluster" (subtype) the subject of interest belongs.

Referring to Fig. 7, computing system 300 includes: a storage device 2080 for storing data from storage device 210 as well as data generated during the course of the calculation; and a processor 2040 performing operation on data in storage device 2080. Processor 2040 may be a CPU, for example.

Processor 2040 includes: a correlation matrix calculating unit 3002 for calculating elements of the correlation matrix for the MRI measurement data 3102 of patient cohort and healthy person cohort by performing a program and storing the results as correlation matrix data 3106 in storage device 2080; a harmonization calculating unit 3020 for performing the harmonization process; and a learning and discriminating unit 3000 for performing the disease identifier generating process, the clustering classifier generating process, and the discriminating process using the generated disease identifier or clustering classifier, based on the result of harmonization process.

Fig. 8 is a functional block diagram showing the configuration of Fig. 7 in greater detail.

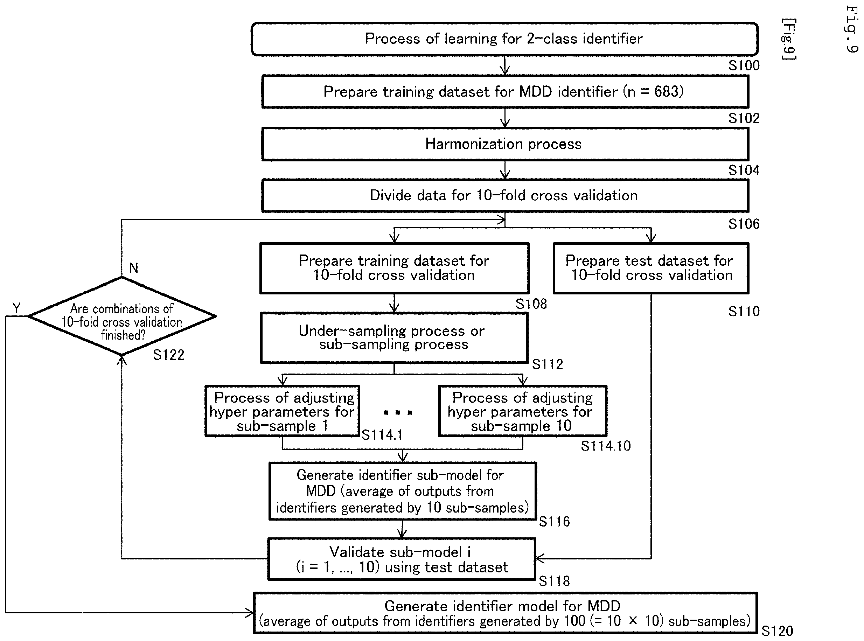

Fig. 9 is a flowchart illustrating the procedure of machine learning for generating the disease identifier through ensemble learning.

First, the harmonization process and the process up to the generation of identifier (disease identifier) through ensemble learning shown in Fig. 6 will be described with reference to Figs. 8 and 9.

It is assumed that fMRI measurement data of subjects (healthy persons and patients), attribute data of subjects, and measurement parameters are collected from respective measurement sites and stored as "training dataset" in storage device 210 of data center 200.

Referring to Figs. 8 and 9, a biomarker for the MDD discrimination is built using the above-described training dataset as the training data for the disease identifier (two-class classifier for "healthy" and "sick"). The marker identifies a healthy person cohort (a group of samples having a diagnosis label of healthy control (HC)) and an MDD patient cohort (a group of samples having a diagnosis label of Major Depressive Disorder) based on 71,631 values of functional connectivity FC.

As will be described in the following, in the learning process for generating an identifier for MDD (hereinafter referred to as an "MDD identifier"), an optimal subset of the functional connectivity FC is selected from 71,631 functional connectivity FC using the logistic regression analysis (one of sparse modeling methods) by the L1 regularization (LASSO: least absolute shrinkage and selection operator).

Generally, when L1 regularization is used, some parameters (in the following description, weight elements) can be reduced to zeros. In other words, feature selection is done, resulting in a sparse model.

It is noted, however, that the method of sparse modeling is not limited to the LASSO method, and other methods such as the sparse logistic regression (SLR) which applies the variational Bayesian method to the logistic regression, as will be described later, may be used.

Referring to Fig. 9, when the process of learning for the MDD identifier starts (S 100), correlation matrix calculating unit 3002 calculates elements of the connectivity matrix using prepared (i.e., stored in storage device 2080) training dataset (S102).

Thereafter, harmonization calculating unit 3020 performs the harmonization process using calculated measurement biases (S104).

As will be described later, the harmonization process using traveling subjects is preferred, though other methods may be used.

For example, it is possible to harmonize datasets by using ComBat method, which will be described later, between discovery dataset and independent validation dataset.

Thereafter, disease identifier generator 3008 generates the MDD identifier by applying a method which is a so-called "ensemble learning method," that is, a modification of "Nested Cross Validation" on the training data.

First, to perform the training process on the training data using the "K-fold cross validation" (K is a natural number) (external cross-validation), disease identifier generator 3008 sets K to 10, for example, and divides the training data into 10 subsets.

Specifically, disease identifier generator 3008 uses one of the subsets of the K-fold (10-divided) data as a "test dataset" for validation, and assigns the remaining (K-1) (nine) data subsets as training dataset (S108, S110).

Then, disease identifier generator 3008 performs an "under-sampling process" and a "sub-sampling process" on the training dataset (S112).

Here, the "under-sampling process" means a process performed to even out the numbers of data in the training dataset corresponding to the specific, different (two or more) attributes that are the targets of the classification, when their numbers are different. Namely, it is a process of removing some data from a larger group of data having an attribute, so that the numbers of the data of different attributes will be equal.

Here, in the training dataset, the number of subjects of the MDD patient cohort is not equal to the number of subjects of healthy person cohort and, therefore, the process to equalize is performed.

Further, the "sub-sampling process" means a process of extracting a prescribed number of random samples from the training dataset.

Specifically, in the K-times iteration of cross validation through steps S108 to S118 and S122, in each cross validation, under-sampling is done for building the classifier since the numbers of the MDD patients and the healthy persons HC are imbalanced in the training dataset. In addition, a prescribed number, for example, 130, of the MDD patients and the same number of healthy persons are sampled at random from the training dataset as the sub-sampling process.

The number "130" is not limiting, and it is determined appropriately to enable the above-described under-sampling, in accordance with the numbers of data in the training dataset (683 in "Dataset 1" which will be described later), the fold number K (here, K = 10, for example), and the degree of imbalance of the numbers of data respectively corresponding to specific attributes as the targets of classification.

Sub-sampling such as described above is performed, since under-sampling is disadvantageous because the removed data will not be used in training the classifier. Random sampling (or sub-sampling) is repeated M times (M = natural number, for example, M = 10) to avoid the disadvantage.

As will be described later, under-sampling and sub-sampling as such are technically meaningful also for "feature selection" at the time of generating a "classifier" for "stratification." This point will be discussed later.

Thereafter, disease identifier generator 3008 performs a process for adjusting hyper parameters for each of the sub-samples 1 to 10 that have been sub-sampled (S1 14.1 to S114.10).

Here, an identifier sub-model is generated by using the following logistic function on each sub-sample. The logistic function is used for defining the probability of a participant in the sub-samples belonging to the MDD class as follows.

[Equation 1]

$$P_{sub}(y_{sub} = 1 \mid c_{sub} ; w) = \frac{1}{1 + exp\left(-w^T c_{sub}\right)}.$$

Here, $y_{sub}$ represents a class label of the participant (MDD, y = 1; HC, y = 0), $c_{sub}$ represents an FC vector for a given participant, and w represents a weight vector.

The weight vector w is determined to minimize the following evaluation function (cost function) (LASSO calculation).

[Equation 2]

$$J(\omega) = -\frac{1}{n_{sub}} \sum_{j=1}^{n_{sub}} [y_j \log t_j + (1 - y_j) \log(1 - t_j)] + \lambda \|\omega\|_1,$$

$$\|\omega\|_1 = \sum_{i=1}^{N} |\omega_i|.$$

Assuming that $t_j = P_j (y_j = 1 \mid c_j ; \omega)$.

In the LASSO calculation, the sum (L1 norm) of absolute values (1st order) of elements of the weight vector exists as the second term in the cost function.

Here, λ represents a hyper parameter and it controls the amount of shrinkage used for the evaluation.

In each sub-sample, though not limiting, disease identifier generator 3008 uses a prescribed number of data as hyper parameter adjusting data and uses the remaining data (for example, data of n = 250 or 248 participants) to determine the weight vector w. Though not specifically limiting, here, assuming that the hyper parameter λ is in the range of 0 < λ ≤ 1.0, disease identifier generator 3008 divides this range into equal P intervals (P: natural number), for example, 25 intervals, and using each of the resulting λ values, determines the weight vector w by the LASSO calculation mentioned above.

Here, as the "Nested Cross Validation" described above, hyper parameter adjustment is done as the "internal cross validation." In the internal cross validation, the "test dataset" of the external cross validation is not used.

Then, disease identifier generator 3008 compares the discrimination performance (for example, accuracy) for the hyper parameter adjusting data using a logistic function corresponding to each generated λ value, and selects the logistic function that corresponds to the parameter λ that attains the highest discrimination performance (hyper parameter adjustment process).

Thereafter, disease identifier generator 3008 configures an "identifier sub-model" to output an average of output values of the logistic function corresponding to the sub-samples generated in the current loop of cross validation (S116). Because the identification performance is determined based on an average of identifier output values calculated for each sub-sample, this can be regarded as one type of "ensemble learning."

Using the test dataset prepared at step S110 as an input, disease identifier generator 3008 performs the validation of the identifier sub-model generated in the current loop of cross validation (S118).

As the method of generating an identifier sub-model by generating sub-samples by under-sampling and sub-sampling and performing feature selection in each sub-sample, sparse modeling methods may be used other than the methods of using the LASSO method together with the hyper parameter adjustment described above.

If it is determined that the K-th (here, 10th) cross validation loop has not been finished yet (N at S122), disease identifier generator 3008 selects a sub-dataset of the K-divided data which is different from those used in past loops as the test dataset, assigns the remaining sub-datasets as the training dataset (S108, S110), and repeats the process.

On the other hand, if it is determined that the K-th (10th) cross validation loop is finished (Y at S122), disease identifier generator 3008 generates an identifier model for the MDD (MDD identifier) (S120) that outputs an average of outputs of K × M (here, 10 × 10 = 100) logistic functions (identifiers) for the input data.

After all, the MDD identifier can be regarded as an "identifier" obtained as a result of "ensemble learning" in that it provides an average of outputs from K × M identifiers as its identifying output.

When the output of the MDD identifier (probability of diagnosis) exceeds 0.5, it can be regarded as an index pointing to an MDD patient.

In the present embodiment also, as indexes for evaluating the performance of the MDD identifier generated in the above-described manner, Matthews correlation coefficients (MCC), AUC (area under the curve) of ROC (Receiver Operating Characteristic curve), accuracy, sensitivity, and specificity are used.

The method of generating an identifier of an object disease (for example, MDD) by using a feature (here, components of the correlation matrix after the harmonization of the measurement biases) selected at each sub-sample is not limited to the process of averaging outputs of a plurality of identifier sub-models as described above. Such a classifier may be generated by majority voting or by using other modeling methods, particularly other sparse modeling methods, on the selected features.

(Examples and Performance of Data Used for MDD Identifier)

As already described above, in order to build reliable classifiers and regression models using the machine learning algorithms, it is necessary to use data of a large sample size collected from many imaging sites.

Therefore, in the following, we discuss such models using a resting state fMRI dataset for learning of about 700 participants, including the MDD patients, collected from four different imaging sites.

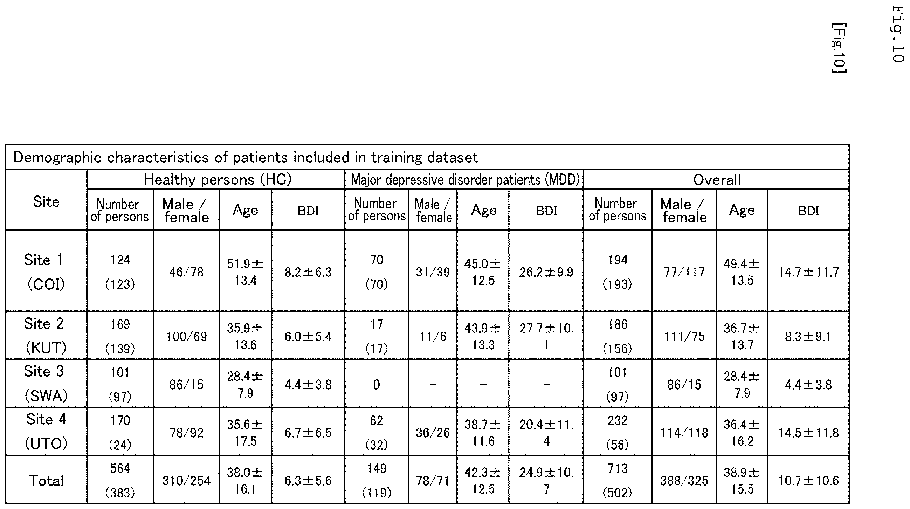

Fig. 10 shows the demographic characteristics of the training dataset (Dataset 1).

Dataset 1 is the data in the above-described SRPBS dataset.

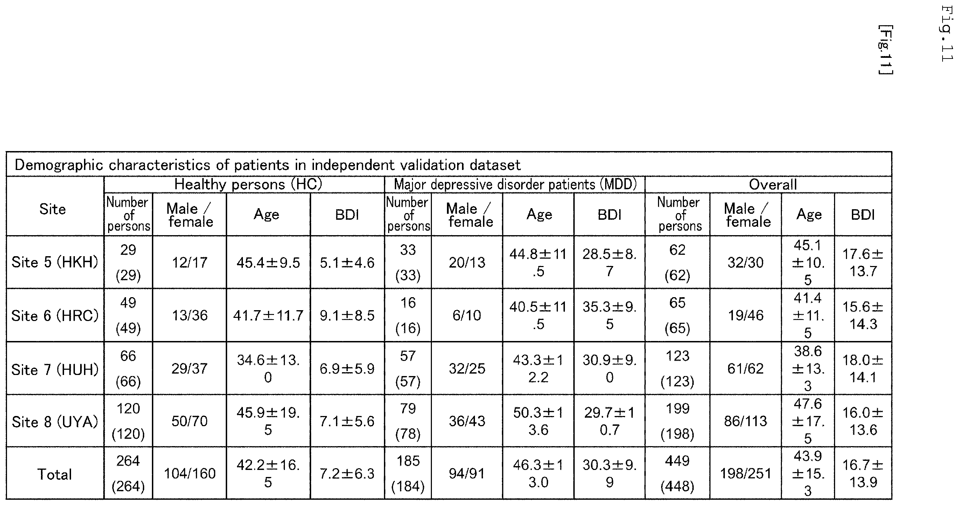

Fig. 11 shows the demographic characteristics of the independent validation dataset (Dataset 2).

Dataset 2 is also, basically, the data in the SRPBS above.

Specifically, the following two resting state functional MRI (rs-fMRI) datasets are used in the following analysis.

(1) As shown in Fig. 10, Dataset 1 includes data of 713 participants (a healthy person cohort HC of 564 persons from four sites, an MDD patient cohort of 149 persons from three sites).
(2) As shown in Fig. 11, Dataset 2 includes data of 449 participants (a healthy person cohort HC of 264 persons from four sites, an MDD patient cohort of 185 persons from four sites).

Further, "depressive symptoms" are evaluated by using the Beck Depression Inventory (BDI) II obtained from most of the participants of each dataset.

Dataset 1 is a "training dataset" and used for building MDD identifiers and clustering classifiers.

Each of the measurements of participants was carried out in a single 10-minute session of resting state functional MRI (rs-fMRI).

Here again, the resting state functional MRI (rs-fMRI) data was obtained under the standardized imaging protocols (http://www.cns.atr.jp/rs-fmri-protocol-2/).

It is actually difficult, however, to ensure that imaging was done using the same parameters at every site, and two-phase modulation directions (P -> A and A -> P), MRI devices of two manufacturers (Siemens and GE), three different coil numbers (12, 24, 32), and scanners of three model numbers were used for measurements.

During the scanning of the resting state functional MRI (rs-fMRI), typically, an instruction such as shown below is given to each participant.

"Please try to relax. Please don't sleep. Keep looking at a cross mark in the center and do not think about any specific thing."

The "demographic characteristics" in the dataset are the characteristics used in the so-called "demographics," and include the age, sex, and attributes such as the diagnosis listed in the table.

In Figs. 10 and 11, the number of persons in the parentheses indicates the number of participants having the BDI score data.

The demographic distributions do not have statistically significant differences between the MDD and the HC sample groups in every training dataset (p > 0.05).

Dataset 2 is the "independent validation dataset," which is used for testing the MDD classifiers and the clustering classifiers.

The sites used for the imaging of Dataset 2 are not included in Dataset 1.

The demographic distribution of age matches between the MDD and the HC sample groups (p > 0.05) in the independent validation dataset, while the demographic distribution of sex does not match between the MDD and the HC sample groups in the independent validation dataset (p < 0.05).

(Site Effect Control)

Further, in the following, description will be made assuming that the traveling subject harmonization method as will be described later is used for the training dataset in order to control site effect on the functional connectivity FC.

It is noted, however, that the harmonization method is not limited to this, and other methods, such as the ComBat method, may be used, for example.

The ComBat method is disclosed, for example, in the reference below.

Reference: Johnson WE, Li C, Rabinovic A. "Adjusting batch effects in microarray expression data using empirical Bayes methods." Biostatistics 8, 118-127 (2007).

The traveling subject harmonization method makes it possible to remove the pure site-to-site differences (measurement biases).

Note that no dataset of the traveling subject existed for the site or sites included in the independent validation dataset and, therefore, harmonization was done using the ComBat method, in order to control site effects in the independent validation dataset.

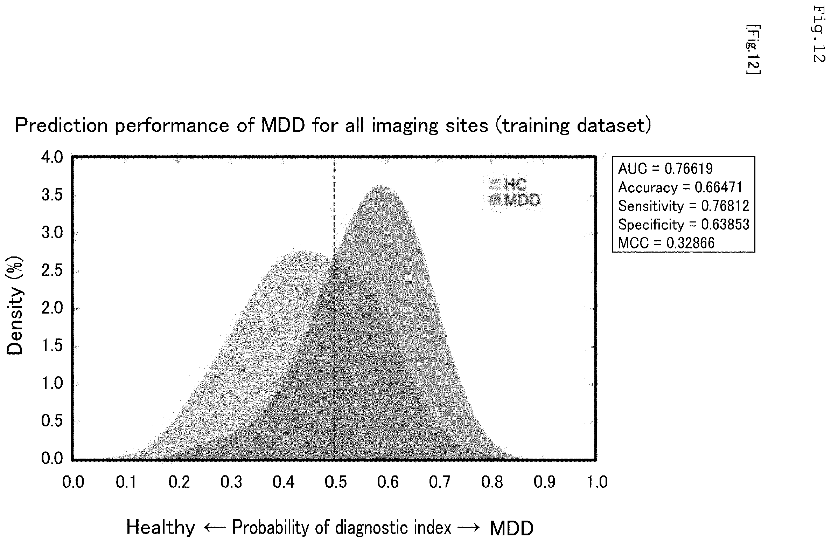

Fig. 12 shows the prediction performance (output probability distribution) of MDD on the training dataset for all imaging sites.

In the outputs from an identifier model for the training dataset, probability distributions of two diagnoses corresponding to the groups of the MDD patients and the healthy persons are clearly separated to right (MDD) and left (HC) at the threshold value of 0.5.

The identifier model separates the MDD patients from the HC cohort with the accuracy of 66%.

The corresponding AUC is 0.77, showing a high identifying ability.

The MCC is about 0.33.

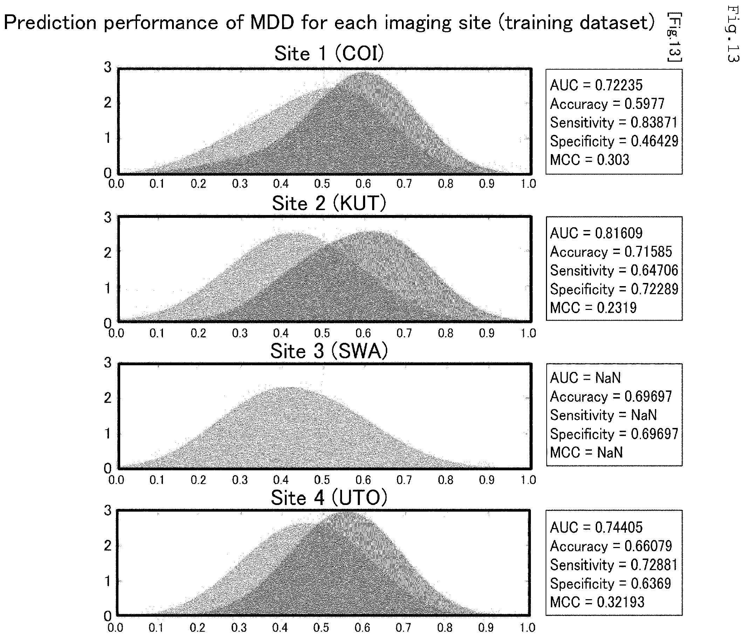

Fig. 13 shows the prediction performance (identifier output probability distribution) of MDD on the training dataset for each imaging site.

It can be seen from Fig. 13 that almost equally high classification precision could be attained not only for the entire dataset but also for respective datasets of the three imaging sites (site 1, site 2, site 4).

It is noted that in the dataset of site 3 (SWA), only a healthy person cohort exists. Its probability distribution, however, corresponds to that of the healthy person cohorts of other sites.

(Identifier Generalization Performance)

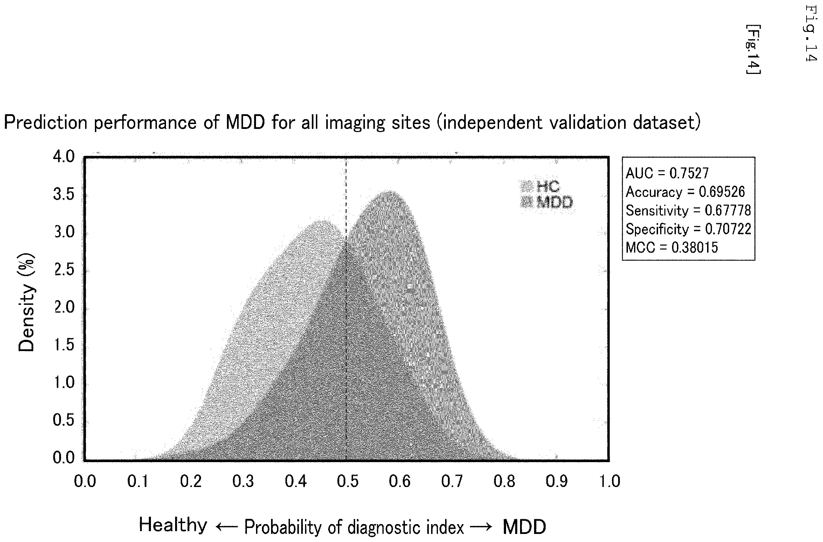

Fig. 14 shows the identifier output probability distribution of the MDD on the independent validation dataset.

Specifically, the generalization performance of the identifier models is tested using an independent validation dataset.

Regarding MDD, in the process shown in Fig. 12, 100 (10-fold $\times$ 10 sub-samplings) logistic function identifiers are generated by machine learning. An independent validation dataset is input to each of the generated 100 identifiers (identifier models as a set of classifiers).

For each participant, an average of outputs from 100 identifiers (probability of diagnosis) is calculated. If the averaged probability of diagnosis is > 0.5, it is determined that the diagnosis label assigned to the corresponding participant is major depressive disorder.

In the independent validation dataset, the generated identifier models separate the MDD sample group from the HC sample group with the precision of about 70%.

The corresponding AUC is 0.75, indicating a performance attaining a high identifying ability (permutation test $p < 0.01$).

For the independent validation dataset, the probability distribution of outputs from the identifier models are clearly separated into two diagnoses corresponding to the groups of the MDD patients and the healthy persons, that is, to the right (MDD) and to the left (HC), by the threshold value of 0.5.

Sensitivity is 68%, and specificity is 71%. This leads to a high MCC value of 0.38 (permutation test $p < 0.01$).

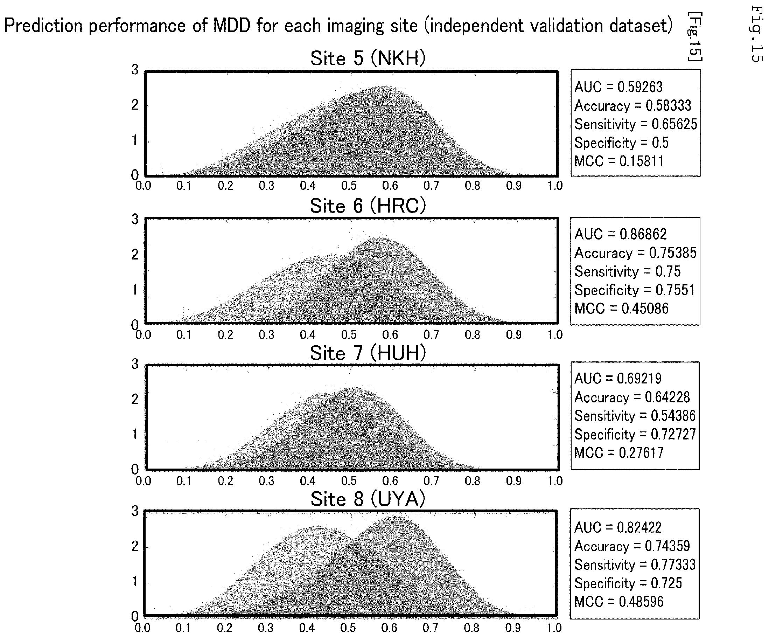

Fig. 15 shows the identifier output probability distribution of the MDD with respect to the independent validation dataset for each imaging site.

It can be seen that a high classification precision can be attained not only for the whole dataset of the four imaging sites but also for the individual dataset.

[Subject Data Clustering Process]

In the following, of the processes described with reference to Fig. 6, feature selection for clustering and clustering based on the selected features will be discussed in greater detail.

Specifically, the process referred to as "feature selection" and "clustering" in Fig. 6 will be described as processes performed by disease identifier generator 3008 and a clustering classifier generator 3010 shown in Fig. 8.

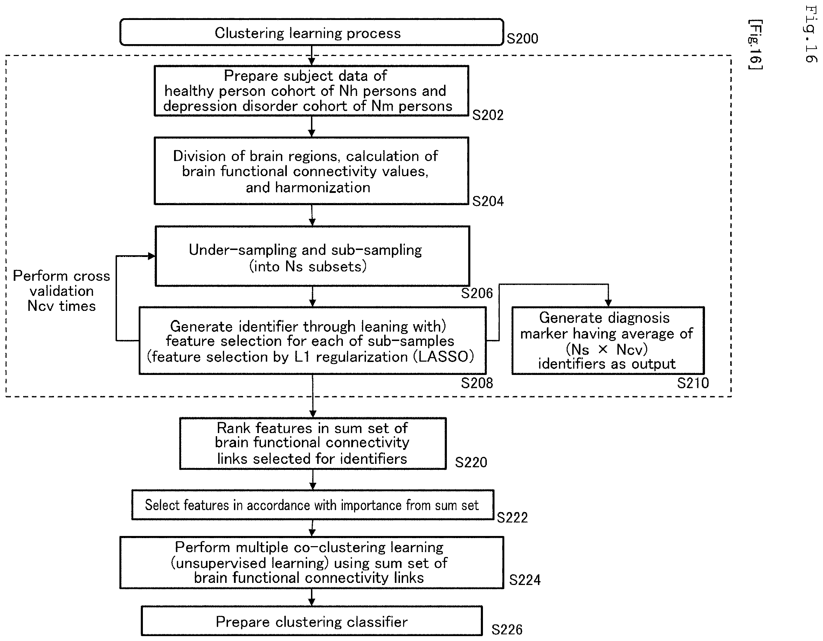

Fig. 16 is a flowchart representing a process of clustering by unsupervised learning by selecting features.

In the following, a process of clustering through unsupervised learning will be described in which features (brain functional connectivity links) used for generating submodels of each identifier in the process of learning "two-class identifier" described with reference to Fig. 9 are ranked and a prescribed number of high-ranking features are used.

As already described above, brain functional connectivity comes to have as high as 70000 dimensions or higher depending on the method of brain parcellation, and hence, it is difficult to perform clustering through unsupervised learning in a common manner. In the present embodiment, to address this clustering problem, in the "generation of identifier through supervised learning," features are ranked in accordance with their importance, and "clustering through unsupervised learning" using the features selected in accordance with the ranking is combined to enable such clustering process.

In the following, for convenience of description, the clustering process is described as a process different from the process for generating a disease identifier. It is noted, however, that steps S200 to S210 of Fig. 16 are the same as steps S100 to S 120 of Fig. 9, and the process for generating a disease identifier and the clustering process can be performed as a series of processes.

Referring to Fig. 16, when the learning process for clustering starts, disease identifier generator 3008 prepares subject data of a healthy person cohort of Nh persons and a depression disorder cohort of Nm persons (S202). With the functional brain activity data of the subjects, disease identifier generator 3008 performs division of brain regions (parcellation), calculates brain functional connectivity values, and performs harmonization (S204).

Thereafter, disease identifier generator 3008 performs data division for Ncv-fold cross validation (Ncv: natural number and Ncv $\geq$ 2) and prepares a training dataset and a test dataset for each divided data. For each training dataset, under-sampling and sub-sampling are performed to generate Ns test data subsets (S206).

Further, disease identifier generator 3008 generates an identifier through learning with feature selection, for each of the sub-samples thus sub-sampled (S208).

Here, as in the case of Fig. 9, it is assumed that feature selection is done by L1 regularization (LASSO).

The process of steps S206 to S208 as described above is repeated on the Ncv-divided training data set while successively changing combinations of training dataset ((Ncv-1) of the divided dataset) and the test dataset (one of the divided data sets), until cross-validation is done Ncv times.

An integrated identifier having the average of (Ns $\times$ Ncv) identifiers generated in this manner as an output is generated as a disease identifier (diagnosis marker) (S210).

The process so far is the same as that of steps S100 to S 120 of Fig. 9, as mentioned above.

On the other hand, at steps S206 to S208 that are repeated Ncv times, regarding the sum set of features (brain functional connectivity links) selected for generating identifiers through learning with feature selection, though not limiting, clustering classifier generator 3010 performs ranking of the features in the sum set in accordance with the number of times each feature is selected (S220).

Here, the "number of times each feature is selected" will be referred to as the importance of the feature in this ranking.

In other words, in the example shown in Fig. 9, for example, one hundred (= 10 $\times$ 10) identifiers are generated by LASSO method, and the number of selections of any brain functional connectivity of having non-zero weight in each identifier is incremented by 1. The connectivity having a larger count is ranked as one having higher importance.

Thereafter, in order to realize clustering of a patient cohort of depression disorder through unsupervised learning, clustering classifier generator 3010 selects a prescribed number of features, for example, in accordance with the importance, from the sum set (S222).

Further, clustering classifier generator 3010 performs clustering, using multiple co-clustering as will be described later as the method of unsupervised learning (S224).

Through the steps as described above, clustering classifier generator 3010 generates a clustering classifier for the patient cohort of depression disorder (S226).

II. Model Use Phase

Specifically, by the process above, clustering classifier generator 3010 specifies, from monitored data of each cluster, a probability distribution model that generates such monitored data, and information of each model is stored in storage device 2080 in disease identifier data. Then, a discrimination value calculator 3012 as the clustering classifier calculates, for any input data other than the training data, posterior probability at which the input data belongs to each cluster based on each probability distribution model and outputs, to the cluster which has the highest posterior probability, the result of classification that the input data belongs to that cluster (MAP: Maximum A posteriori Probability estimation method).

[Additional Description of Learning Method and Trained Model According to Present Method]

In the foregoing, the clustering process has been described as being based on the process of generating identifiers performed by disease identifier generator 3008 on the data of a healthy person cohort of Nh persons and a depression disorder cohort of Nm persons. The clustering method in accordance with the present invention, however, is not limited to such an example. It is applicable to a patient cohort of diseases other than "depression disorder." For example, the method can be used for clustering patient cohort of other mental disorders such as "patient cohort of schizophrenia," "patient cohort of autism," or "patient cohort of obsessive-compulsive disorder."

More generally speaking, any attribute label classified empirically by humans (for example, one's personality, one's specialty, and so on) is considered. If it is found that a certain attribute has a prescribed relation with a pattern of correlation between brain regions of time-change in brain activities, it is possible to use the method for "clustering of subject cohort belonging to the certain attribute" (classification to subtypes) in a data-driven manner, for the subjects classified in accordance with the attribute label.

(Under-Sampling and Sub-Sampling Processes)

In the process described above, "under-sampling and sub-sampling processes" are performed and, therefore, technical meaning of these processes will be briefly described.

First, the effect of "under-sampling process" is that it enables satisfactory setting of identification boundary of the identifiers.

Consider, for example, a task of classifying two classes. In the training data, if the data belonging to each of the two classes vary less in numbers, evaluation precision of identifier performance (such as accuracy) becomes higher in the process flow.

At steps S1 14.1 to S1 14.10 of Fig. 9, the process of "determining the logistic function that corresponds to $\lambda$ attaining the highest discrimination performance" is performed for setting hyper parameters. Therefore, it is essential to correctly evaluate the "discrimination performance."

Assume an extreme example of identifier training using training data including one hundred data items belonging to Class 1 and one data item belonging to Class 2. In that case, even when the identifier should determine that every data belongs to Class 1, accuracy and the like would not be much influenced. In this regard, it is meaningful to perform random sampling to make uniform the number of data belonging to the two classes.

Execution of sub-sampling several times with under-sampling is prerequisite from the following reasons.

First, under-sampling and sub-sampling performed only once leads to data bias, even when performed as random sampling.

Second, as will be described in the following, generation of an "identifier" repeated at steps S108 to S122 of Fig. 9 is realized by "ensemble learning" as described above.

Here, in generating each identifier, importance of features for identification is determined.

The importance is determined in accordance with the degree of contribution of each feature to the identification. For example, what matters in the "learning with feature selection" is the fact that the feature is selected, and what matters in the "learning without feature selection" is the weight of the feature on the calculated identification.

In the following, the meaning of "under-sampling" and "sub-sampling" in determining the importance as such will be described, taking "learning with feature selection" as an example. Even in "learning without feature selection" such as L2 regularization, we can assume that the event that "weight of the feature increases" basically comes from the same technical reason as the event of being "selected as a feature."

Here, the "learning with feature selection" may be a so-called "sparse modeling" such as the LASSO method described above, for example.

In sparse modeling, features are selected sparsely. Specifically, specific features will have non-zero weights while other features have zero weight, and thus, features are selected. One reason why such sparse feature selection can be realized is as follows. When a "group of features contributing in a similar manner" to a "discrimination (identification) process" exists, there is a "penalty term corresponding to the number of features" to allow the learning process to proceed such that one feature of the group is selected and other features of the group come to have the weights of zero. This tendency is particularly dominant in the LASSO method.

Specifically, when feature A and feature B relate to the "discrimination process" in the similar manner, or when features A and B have high correlation, discrimination process with high discrimination performance is possible even when only feature A is selected as the feature.

In clustering, however, there may be a case in which both features A and B have to be considered, for example. If a feature is selected simply in accordance with the degree of contribution to a discrimination process in the course of generating one identifier by the above-described "sparse" selection, the resulting "feature selection" could possibly be insufficient for the clustering.

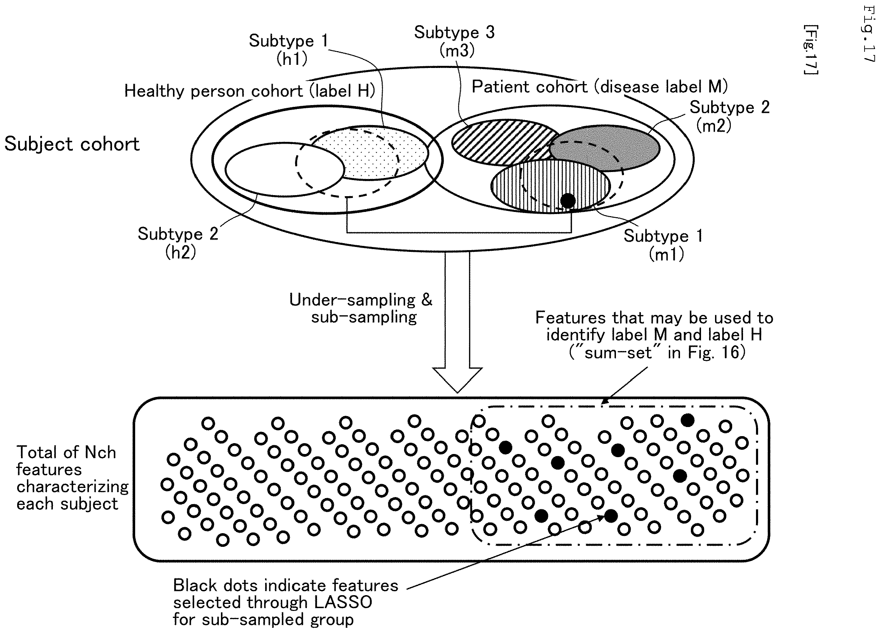

Fig. 17 shows a concept of performing feature selection when a plurality (for example, Nch) of features exist, by "learning with feature selection."

Referring to Fig. 17, it is assumed that the subject cohort as the target for learning includes a healthy person cohort and a patient cohort.

Subjects of the healthy person cohort are labeled H, and the healthy person cohort includes subtypes h1 and h2.

Subjects of the patient cohort are labeled M, and the patient cohort includes subtypes m1, m2, and m3.

Here, it is unknown from observables how many subtypes are included in the healthy person cohort and the patient cohort, and the identification labels of subtypes are latent labels that are not explicitly given to the subjects.

The object of "clustering" is to perform data-driven clustering to the subtypes from the observables.

"Under-sampling" and "sub-sampling" are performed on the subjects from the healthy person cohort and the patient cohort as such at random. Thus, from the "subject cohort" shown Fig. 17, subjects in the circles surrounded by dotted lines, for example, are selected respectively from the healthy person cohort and the patient cohort.

It is assumed that features (brain functional connectivity correlation values) usable for identifying label M and label H are those in the area surrounded by a chain-dotted line in Fig. 17 ("sum-set of brain functional connectivity links" at step S220 of Fig. 16), among the brain functional connectivity links as the features (Nch features in total) characterizing each subject.

It is further assumed that as a result of learning of an identifier for identifying label M and label H through learning with feature selection (here, learning by the LASSO method), features represented by black dots in the area surrounded by the chain-dotted line in Fig. 17 are selected.

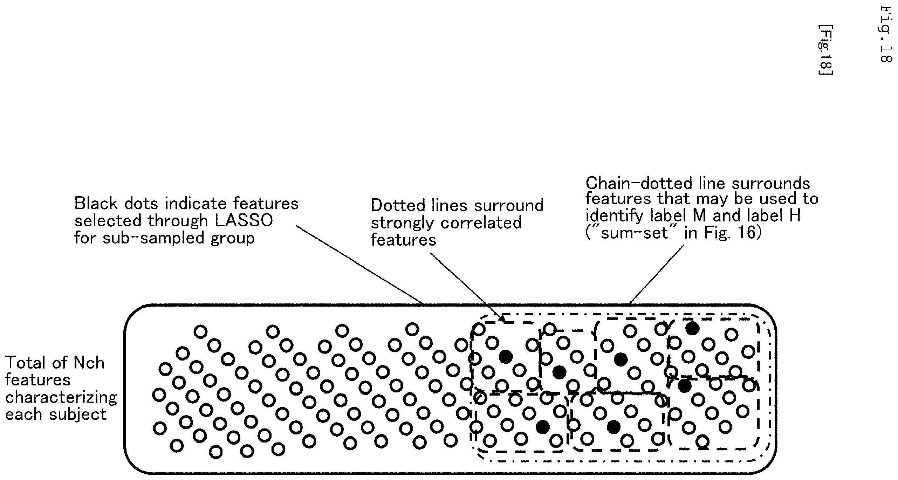

Fig. 18 is an illustration showing the features finally selected after under-sampling and sub-sampling, when one identifier is generated by learning with feature selection.

It is assumed that as shown in Fig. 18, the features usable to identify label M and label H in the area surrounded by the chain-dotted line are further divided to groups of features having high correlations with each other, as represented by frames surrounded by dotted lines.

In the LASSO method, one feature is selected from each group of the dotted frames, and thus, the features are made sparse.

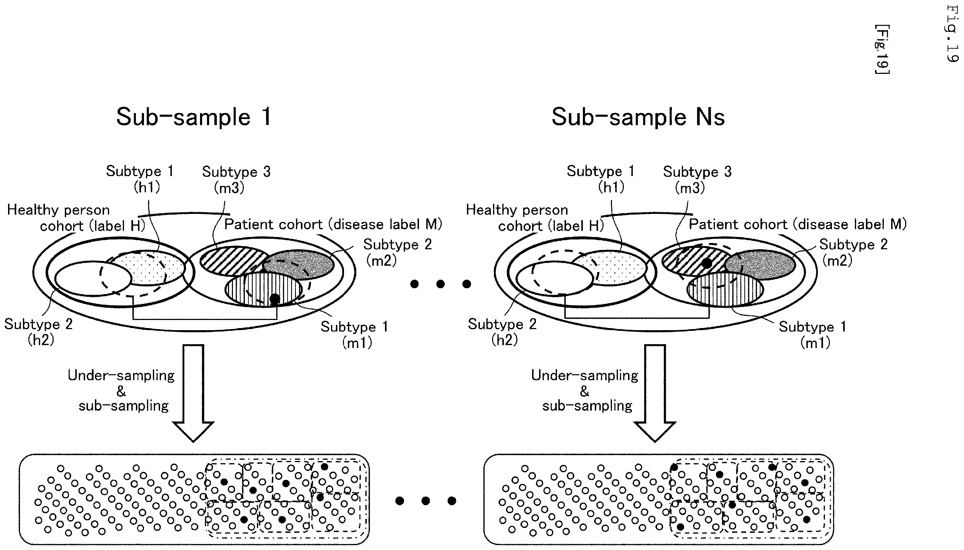

Fig. 19 is an illustration showing how features are selected when an identifier is generated by performing under-sampling and sub-sampling processes a number of times.

Referring to Fig. 19, assuming that sub-sampling is performed Ns times, different subjects would be sub-sampled from the healthy person cohort and the patient cohort at each time.

As a result of learning of an identifier for identifying label M and label H through learning with feature selection on each sub-sample, different features would be selected by different identifiers as represented by black dots, from each group having high correlation, in the area surrounded by the chain-dotted line representing the sum-set mentioned above, for each sub-sample.

As a result, a sum-set of features usable for identifying label M and label H is selected by executing the under-sampling and sub-sampling processes a number of times.

In the present embodiment, the features selected for each sub-sample by the learning of identifiers with feature selection in accordance with the LASSO method are ranked in the order of frequency of selection.

Clustering through unsupervised learning is performed using a prescribed number of features starting from the highest of the rank, for example, the highest one hundred features, by "multiple co-clustering" as will be described later at step S224 of Fig. 16.

In the foregoing, as the "learning of identifiers with feature selection," the LASSO method has been described as an example, and feature selection for clustering is performed in accordance with the rank in accordance with the frequency of selection.

As already described, however, in the clustering process in accordance with the present embodiment, "learning of identifiers with feature selection" is not limited to such a method, and a method such as random forest method may be used, and feature selection for clustering may be done in accordance with prescribed importance.

For example, in the random forest method described above, importance of features is calculated based on Gini impurity for the learning of identifiers and, therefore, features may be ranked in accordance with this importance, and clustering through unsupervised learning may be done using a prescribed number of features from the highest of the rank by "multiple co-clustering" at step S224 of Fig. 16.

Further, as "identifier learning process through ensemble learning," it is possible to use Ridge regularization or the like, and features may be ranked in accordance with the importance that corresponds to the median of the sum of absolute values of weight coefficients as described above. The feature selection for clustering may be performed using a prescribed number of features from the highest one of the ranks prepared in this manner.

[Multiple Co-Clustering]

In the following, the concept of "multiple co-clustering" at step S224 of Fig. 16 will be described to define the term "multiple co-clustering."

(Normal Clustering Method)

As a premise, it is understood that "clustering" refers to a method of data classification through unsupervised learning performed by a computer and, more specifically, to a method of automatically classifying given data without any external criterion. In contrast, "class separation" generally refers to a method of classification through "supervised learning." Further, a "cluster" is defined to be a subset of data having the properties of internal cohesion and external isolation. Here, external isolation refers to the property that objects belonging to different clusters are dissimilar, and the internal cohesion refers to the property that objects in the same cluster are similar to each other. Further, distance between components in the set is defined as the measure of "similarity." Generally, a distance is defined to satisfy the so-called "axioms of distance" and Euclidian distance, Mahalanobis' distance, city block distance, or Minkowski distance is sometimes used as the distance.

Further, generally, known examples of the method of clustering through unsupervised learning include "partitional optimization clustering," which is a method of searching for partitioning that optimizes objective function defining goodness of a cluster, such as the "k-means clustering" which is one of the non-hierarchical methods, as well as "aggregative hierarchical clustering" and "divisive hierarchical clustering," which are hierarchical clustering methods.

These conventional clustering methods, however, are characterized in that all features are used to cluster objects (divide into groups) and the clustering result will be always the same.

Therefore, if there are a plurality of different manners of clustering depending on the features, these methods would not provide satisfactory results. Generally, the larger the number of features is, the higher the possibility of existence of such a plurality of cluster structures is.

The methods of clustering are not limited to the above. There is also an algorithm that, assuming that a plurality of objects to be clustered arise in accordance with a certain probability distribution in each cluster, performs clustering to estimate the "probability distribution." "Clustering with Gaussian mixture distribution," for example, is known as this type of clustering method, and is known to realize more flexible clustering.

(Different Clustering According to Feature Selection)

In the following, first, in order to describe an example in which there are a plurality of different manners of clustering depending on the features, we assume that in an object cohort including a plurality of objects to be clustered, each object is characterized by a plurality of features.

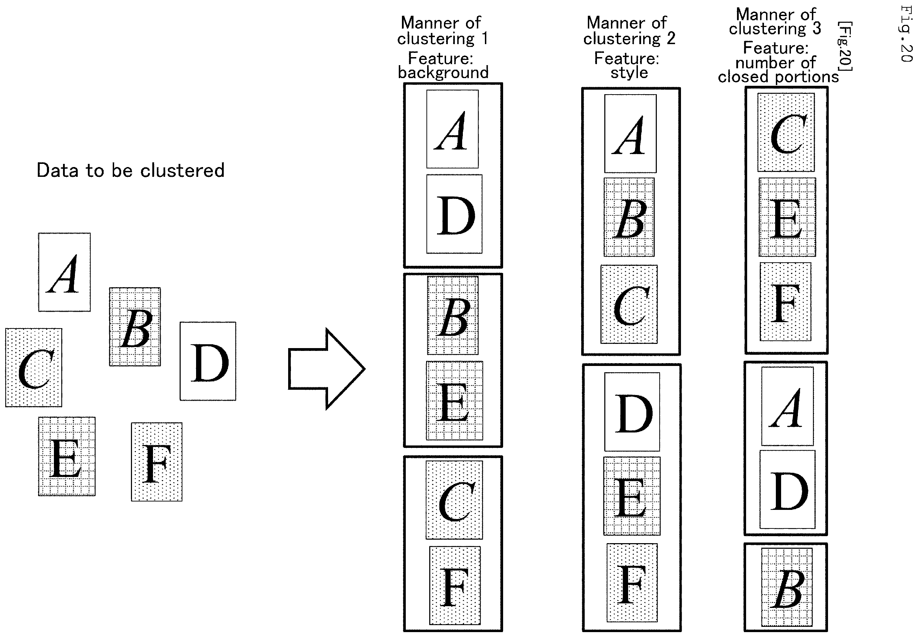

Fig. 20 is an illustration showing a plurality of different manners of clustering depending on features.

Referring to Fig. 20, data items to be clustered (hereinafter simply referred to as "objects") are six letters "A," "B," "C," "D," "E," and "F."

These letters have different background patterns and different fonts (styles).

Possible features characterizing these letters are "background pattern," "style," and "number of closed portions in the letter (number of areas fully surrounded by lines)."

Therefore, one same set of letters can be divided to different clusters depending on which feature is used for clustering.

In the example of Fig. 20, the letters can be clustered to three clusters, that is, {A, D}, {B, E}, and {C, F}, when "background pattern" is used; two clusters of {A, B, C} and {D, E, F} when "style" is used; and three clusters of {C, E, F}, {A, D}, and {B} as having zero, one, and two closed portions when "number of closed portions" is used.

While Fig. 20 shows an example in which one cluster is characterized by one feature, generally, one cluster is characterized by a plurality of features.

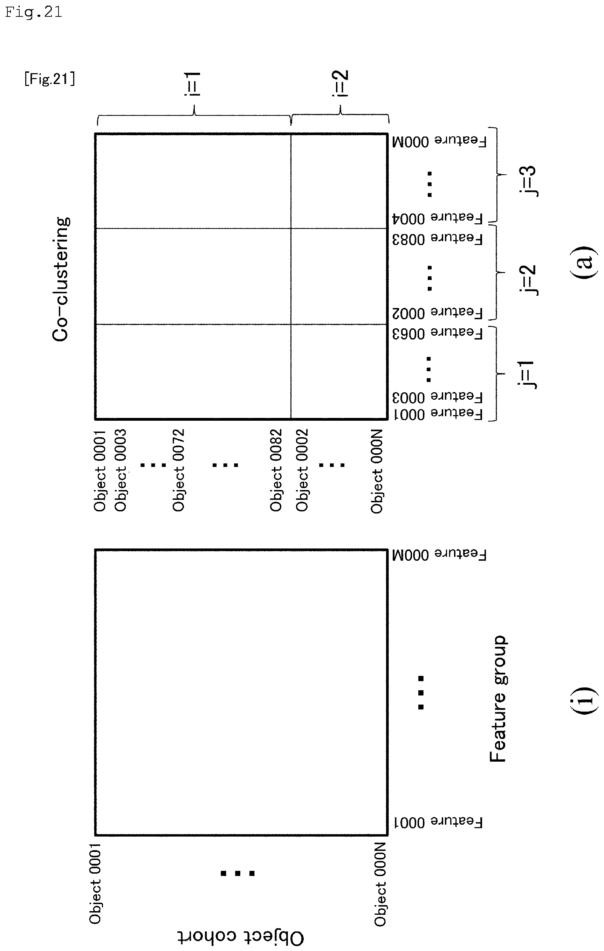

Fig. 21 shows a concept of clustering when a plurality of objects is characterized by a plurality of features.

First, a "data matrix" in which objects to be clustered are arranged along the row direction while features characterizing these objects are arranged along the column direction, as shown in Fig. 21(i), is considered.

A method of clustering objects (partitioning the objects to a plurality of object clusters) and simultaneously clustering features to be related to each cluster as shown in Fig. 21(a) is referred to as "co-clustering," which is disclosed, for example, in the reference below.

Reference: Madeira SC, Oliveira AL. Biclustering algorithms for biological data analysis: a survey. IEEE/ACM Transactions on Computational Biology and Bioinformatics (TCBB). 2004; 1(1):24±45. https://doi.org/10.1109/TCBB.2004.2

In "co-clustering," by interchanging rows or columns of data matrix, that is, by rearranging the objects and the features in accordance with the degree of similarity, the objects are divided into cluster blocks designated by (i, j) (i = 1, 2; j = 1, 2, 3), as shown in Fig. 21(a).

Here, a generative model (probability model) for the objects included in each cluster is assumed and parameters of each probability model are determined such that observed data will have higher likelihood.

Once the probability model is inferred in this manner for each cluster, it becomes possible to discriminate (classify), for specific observed data (test data), to which cluster the data belongs.

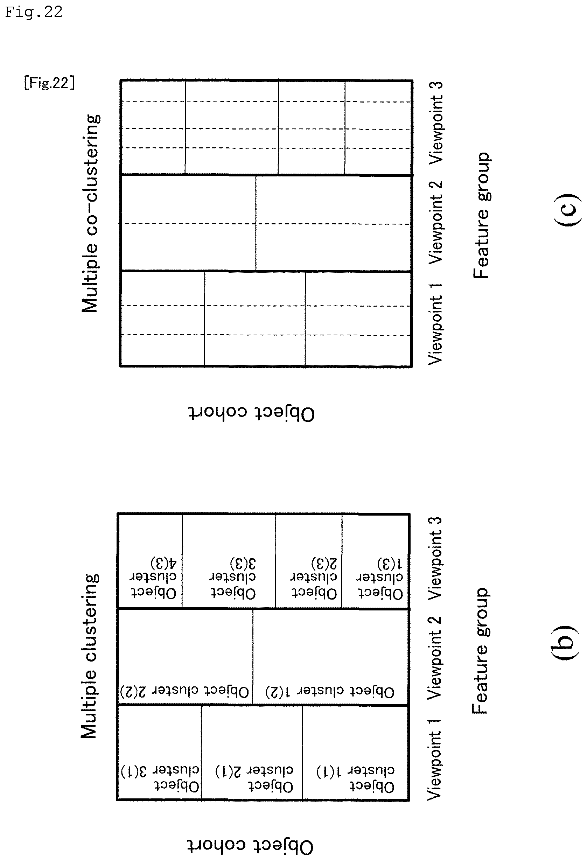

Fig. 22 is an illustration showing a concept of multiple clustering and a concept of multiple co-clustering.

In "co-clustering" shown in Fig. 21(a), the rows and columns of "data matrix" are interchanged to generate clusters having block structures. Therefore, when the features are divided to a plurality of feature clusters, the objects are clustered as commonly aligned along the plurality of feature clusters.

Considering that the features are partitioned to a plurality of feature clusters and the objects are partitioned to object clusters in each feature cluster, it is expected that a probability model of higher likelihood is attained if the arrangement of objects in each object cluster (how the objects included in an object cluster are arranged) is made different in different feature clusters.

In this case, as the manner of partitioning objects (object clustering) differs by feature cluster, each feature cluster is specifically referred to as a "view (viewpoint)."

Clustering different objects for different viewpoints of features in the manner as described above is referred to as "multiple clustering," as shown in Fig. 22(b).

Further, clustering with feature columns and object rows interchanged in each viewpoint may realize inference of a probability model having higher likelihood for the observed data, and this manner of clustering is referred to as "multiple co-clustering" as shown in Fig. 22(c).

Here, the term "multiple co-clustering" is used even when there is only one view or there is only one type of feature cluster in at least one view when a plurality of views exist, and the terms "co-clustering" and "multiple clustering" are used as terms referring to subordinate concepts of "multiple co-clustering."

In the present embodiment, the simple term "clustering" refers to a generation of a set of clusters in one view. When partitioning of features to views is performed simultaneously with clustering of objects as shown in Fig. 22(b), it is referred to as "multiple clustering," and distinguished from "multiple co-clustering" in which partitioning to views is performed simultaneously with co-clustering as shown in Fig. 22(c).

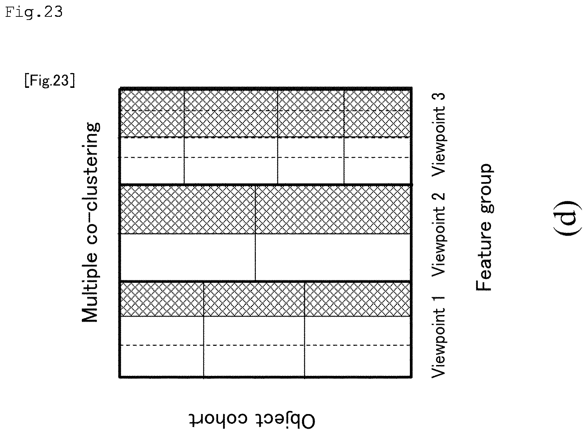

Fig. 23 is an illustration showing a concept in which probability models of different types of probability distributions are assumed in one view in "multiple co-clustering."

In Fig. 23(d), white blocks and hatched blocks have different probability models of different types of probability distributions.

For example, white blocks may have a probability distribution of continuous random variable and the hatched portions may have a probability distribution of discrete random variable.

As will be described in the following, in the method of "learning of multiple co-clustering" in accordance with the present embodiment, it is possible to perform clustering on a distribution family including different distributions.

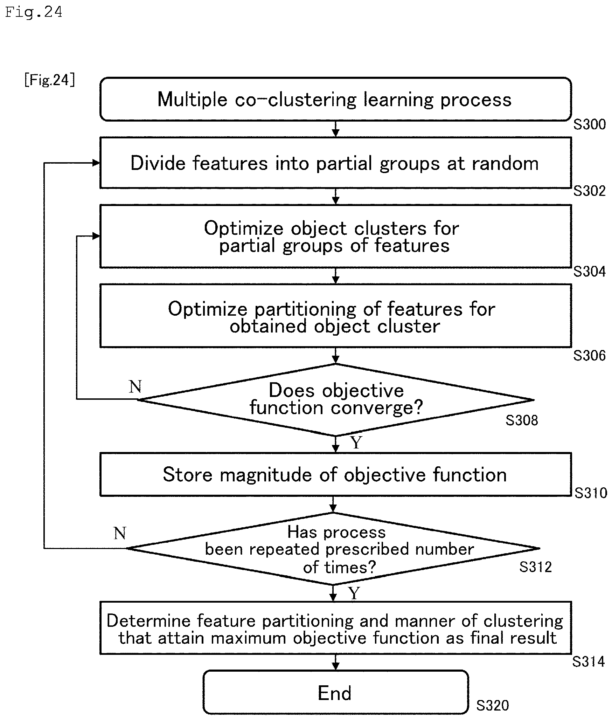

Fig. 24 is a flowchart outlining a method of learning of multiple co-clustering.

When the process of learning of multiple co-clustering starts (S300), clustering classifier generator 3010 divides features of a data matrix to partial groups at random, and thereby generates feature views and feature clusters in the views (S302; corresponding to generation of Y (initialization of Y) described later).

Thereafter, clustering classifier generator 3010 generates partition of object clusters and optimizes the same in accordance with the feature views and the feature clusters generated at step S302 (step S304: corresponding to generation of Z described later).

Further, clustering classifier generator 3010 optimizes partitioning of features for the obtained object cluster (S306: corresponding to the Y generation process as described later, in which Y is optimized using the generated Z).

Thereafter, clustering classifier generator 3010 determines whether or not the objective function satisfies a prescribed condition and converges (S308). This objective function corresponds to a function $L(q(\varphi))$, which will be described later. The function $L(q(\varphi))$ monotonically increases as Y and Z, which will be described later, are updated, and is determined to be converged if it is determined that the rate of this increase has come to be sufficiently small. If it does not converge (NO at step S308), clustering classifier generator 3010 returns the process to step S304 and if it converges (YES at step 308), the process proceeds to the next step.

Then, clustering classifier generator 3010 stores the magnitude of objective function in a storage device 2080 (S310).

Then, clustering classifier generator 3010 determines whether or not the process of steps S302 to S310 has been repeated a prescribed number of times. Clustering classifier generator 3010 returns the process to step S302 if repetition has not reached the prescribed number (NO at step S312), and the process proceeds to the next step if the number is reached (YES at S312).

The manner of feature partitioning and the manner of clustering that attain the maximum objective function are determined as the final result (S314), and clustering classifier generator 3010 ends learning of multiple co-clustering, and thus generates a clustering classifier.

(Details of Multiple Co-Clustering)

In the following, the method of learning of multiple co-clustering described with reference to Fig. 24 will be discussed in greater detail.

Details of multiple co-clustering are disclosed in the reference below and, therefore, outline thereof will be described in the following.

Reference: Tomoki Tokuda, Junichiro Yoshimoto, Yu Shimizu, Go Okada, Masahiro Takamura, Yasumasa Okamoto, Shigeto Yamawaki, Kenji Doya, "Multiple co-clustering based on nonparametric mixture models with heterogeneous marginal distributions", PLOS ONE | https://doi.org/10.1371/journal.pone.0186566 October 19, 2017

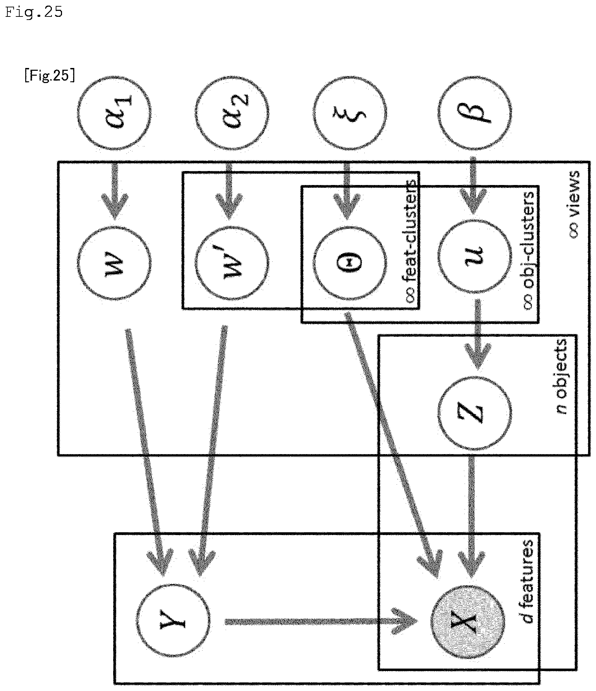

Fig. 25 shows a graph expression of Bayesian inference in the method of learning multiple co-clustering shown in Fig. 24.

The multiple co-clustering model is summarized in the graphical model of Fig. 25, which clarifies causality links between related parameters and data matrix.

(Multiple Co-Clustering Model)

It is assumed that features (brain functional connectivity values) and subjects (here, subjects of the patient cohort) are represented as a data matrix such as shown in Fig. 21(i).

Data matrix X is assumed to be formed of a distribution family consisting of known M distributions.

Probability distributions belonging to the distribution family may include Gaussian distribution, Poisson distribution, and category distribution/multinominal distribution.

Clustering classifier generator 3010 decomposes $X^{(m)}$ as follows with data size n $\times$

$$X = \{X^{(1)}, ..., X^{(m)}, ..., X^{(M)}\}$$

Here, m is an indicator for a distribution family (m = 1, ..., M). Further, the number of views (viewpoints) is denoted by V (common to all distribution families), the number of feature clusters for view v and distribution family m is denoted by $G_v^{(m)}$, and the number of object clusters for view v is represented by $K_v$ (common to all distribution families).

Further, for the simplicity of expression, existence of empty cluster is allowed, and the number of features and the number of clusters are represented as $G^{(m)} = \max_v G_v^{(m)}$ and $K = \max_v K_v$.

With this notation, for independent identical distribution (i.i.d.) $d^{(m)}$-dimensional random vectors $X_1^{(m)}, ..., X_n^{(m)}$ for distribution family m, a $d^{(m)} \times V \times G^{(m)}$ feature partition tensor (third order) $Y^{(m)}$ is considered, and $Y_{j,v,g}^{(m)} = 1$ (0 otherwise) is obtained if feature j of distribution family m belongs to the feature cluster g in view v.

Combining this for different distribution families, $Y = \{Y^{(m)}\}_m$ is obtained.

Similarly, an n $\times$ V $\times$ K object partition (third order) tensor Z is considered, and $Z_{i,v,k} = 1$ is obtained if object i belongs to object cluster k in view v.

Feature j belongs to one of the views ($\Sigma_{v,g}Y_{j,v,g}^{(m)}$ = 1) while object i belongs to each view (i.e., $\Sigma_k Z_{i,v,k}^{(m)}$ =1). Further, Z is common to all distribution families, which implies that the estimated probability model estimates subject cluster solutions using information on all distribution families.

First, referring to Fig. 25, for a prior generative model of Y, a hierarchical structure of views and feature clusters is considered: views are first generated, followed by generation of feature clusters. Thus, features are partitioned in terms of pairs of view and feature cluster memberships, which implies that the allocation of partitioning of feature is jointly determined by its view and feature cluster.

On the other hand, as shown in Fig. 25, objects are partitioned into object clusters in each view and hence, just a single structure of object clusters is considered for Z. It is assumed that these generative models are all based on "SBP" (Stick Breaking Process), as follows.

(Generation Model of Feature Cluster Y)

Assuming that $Y_{j..}^{(m)}$ denotes view/feature cluster membership vector for feature j of distribution family m, which is generated by a hierarchical stick-breaking process, the following expressions are met.

[Equation 3]

$$w_v \quad \sim \quad \text{Beta}(\cdot|1,\alpha_1), \quad v = 1, 2, \ldots$$

$$\pi_v \quad = \quad w_v \prod_{t=1}^{v-1}(1 - w_t),$$

$$w_{g,v}'^{(m)} \quad \sim \quad \text{Beta}(\cdot|1,\alpha_2), \quad g = 1, 2, \ldots, m = 1, \ldots, M$$

$$\pi_{g,v}'^{(m)} \quad = \quad w_{g,v}'^{(m)} \prod_{t=1}^{g-1}(1 - w_{t,v}'^{(m)}),$$

$$\tau_{g,v}^{(m)} \quad = \quad \pi_v \pi_{g,v}'^{(m)}$$

$$Y_{j..}^{(m)} \quad \sim \quad \text{Mul}(\cdot|\tau^{(m)}),$$

where $\tau^{(m)}$ denotes 1 × GV vector $(\tau_{1,1}^{(m)}, \ldots, \tau_{G,V}^{(m)})^T$ (the superscript T indicates a transposed matrix),
Mul($\cdot|\pi$) is a multinominal distribution of 1 sample size with probability parameter $\pi$,
Beta(- |a, b) is a beta distribution with prior sample size (a, b), and
$Y_{j..}^{(m)}$ is a 1 × GV vector $(Y_{j,1,1}^{(m)}, \ldots, Y_{j,V,G}^{(m)})^T$.

Here, the number of views with sufficient large V and the number of feature clusters with G are truncated in accordance with a prescribed condition. See the following reference, for example, for this process.

Reference: Blei DM, Jordan MI, et al. Variational inference for Dirichlet process mixtures. Bayesian analysis. 2006; 1(1) : 121-143,https://doi.org/10.1214/06-BA104

When $Y_{j,v,g}^{(m)}$ = 1, feature j belongs to feature cluster g at view v. By default, concentration parameters $\alpha$1 and $\alpha$2, which are hyper parameters, are set to 1.

(Generation Model of Object Cluster Z)

A subject cluster membership vector of object i in view v, denoted as $Z_{i,v,}$, is generated by the following expressions.

[Equation 4]

$$u_{k,v} \sim \mathrm{Beta}(\cdot|1, \beta), \quad v = 1, 2, \ldots, \quad k = 1, 2, \ldots$$

$$\eta_{k,v} = u_{k,v} \prod_{t=1}^{k-1} (1 - u_{t,v}),$$

$$Z_{i,v\cdot} \sim \mathrm{Mul}(\cdot|\eta_v),$$

In the expressions, $Z_{i,v}$ is a $1 \times K$ (K has a sufficiently large value) vector given by $Z_{i,v} = (Z_{i,v,1}, ..., Z_{i,v,k})^T$. Concentration parameter $\beta$ is set to 1.

(Likelihood and Prior Distribution)

It is assumed that each instance $X_{i,j}^{(m)}$ independently follows a certain distribution, conditional on Y and Z. Parameters of distribution family m in the cluster block of view v, feature cluster g, and object cluster k are denoted as $\theta_{v,g,k}^{(m)}$.

Further, denoting $\Theta = \{\theta_{v,g,k}^{(m)}\}_{v,g,k,m}$, the log-likelihood of X is given by the following equation.

[Equation 5]

$$\log p(X|Y, Z, \Theta) = \sum_{m,v,g,k,j,i} \mathbb{I}(Y_{j,v,g}^{(m)} = 1) \mathbb{I}(Z_{i,v,k} = 1) \log p(X_{i,j}^{(m)}|\theta_{v,g,k}^{(m)}).$$

Here, I(x) is an indicator function, which returns 1 when x is true and returns 0 otherwise.

The likelihood is not directly associated with $w = \{w_v\}_v$, $w' = \{w'_{g,v}{}^{(m)}\}_{g,v}$, and $u = \{u_{k,v}\}_{k,v}$.

Joint prior distribution of unknown variables $\varphi = \{Y, Z, w, w', u, \Theta\}$ (namely, class membership variables and model parameters) is given as follows.

[Equation 6]

$$p(w)p(w')p(Y|w, w')p(u)p(Z|u)p(\Theta).$$

(Variational Inference)

Variational Bayesian EM algorithm is used for MAP (maximum a posteriori) estimation of Y and Z.

Variational Bayesian EM algorithm is disclosed in the reference below.

Reference: Guan Y, Dy JG, Niu D, Ghahramani Z. Variational inference for nonparametric multiple clustering. In: MultiClust Workshop, KDD-2010; 2010.

The logarithm of marginal likelihood p(X) is approximated using Jensen's inequality as shown below.

[Equation 7]

$$\log p(X) \geq \int q(\phi) \log \frac{p(X, \phi)}{q(\phi)} d\phi = \mathcal{L}(q(\phi)). \qquad (1)$$

Jensen's inequality is disclosed in the reference below.

Reference: Jensen V. Sur les fonctions convexes et les inegalites entre les valeurs moyennes. Acta Mathematica. 1906; 30(1): 175-193.https://doi. org/ 10.1007BF02418571

Here, $q(\varphi)$ is an arbitrary distribution of parameter $\varphi$. It can be proved that the difference between the left and right sides is given by the Kullback-Leibler divergence between $q(\varphi)$ and $p(\varphi)$, that is, KL(q($\varphi$), p($\varphi$|X)). Hence, one approach of choosing q($\varphi$) is to minimize KL(q($\varphi$), p($\varphi$|X)); however, its evaluation is generally difficult.

Here, q($\varphi$) is selected so that it is factorized over different parameters (mean-field approximation).

[Equation 8]

$$q(\phi) \quad = \quad q_w(\boldsymbol{w})q_{w'}(\boldsymbol{w'})q_Y(\boldsymbol{Y})q_u(\boldsymbol{u})q_Z(\boldsymbol{Z})q_\Theta(\boldsymbol{\Theta}),$$

In the equation, each q(·) is further factorized over subsets of parameters $w_v$, $w'_{g,v}{}^{(m)}$, $Y_{j..}{}^{(m)}$, $u_{k,v}$, $Z_{i,v}$, and $\theta_{v,g,k}{}^{(m)}$.

In general, the distribution $q_i(\varphi_i)$ that minimizes KL($\Pi_{l=1}{}^L$ $q_l(\varphi_l)$, p ($\varphi|x$)) is given by the following expression.

[Equation 9]

$$q_i(\phi_i) \propto \exp\left\{\mathbb{E}_{-q_i(\phi)} \log p(X, \phi)\right\},$$

where $\mathbb{E}_{-q_i(\phi)}$ denotes averaging with respect to $\Pi_{l\neq i}q_l(\phi_l)$.

This characteristic is disclosed in the reference below.

Reference: Murphy K. Machine Learning: A Probabilistic Perspective. Cambridge, Massachusetts: MIT Press; 2012.

By applying this property to the currently discussed model, the following can be shown.

[Equation 10]

$$q_w(\boldsymbol{w}) \quad = \quad \prod_{v=1}^{V} \mathrm{Beta}(w_v|\gamma_{v,1}, \gamma_{v,2})$$

$$q_{w'}(\boldsymbol{w'}) \quad = \quad \prod_{m=1}^{M}\prod_{v=1}^{V}\prod_{g=1}^{G} \mathrm{Beta}(w_{g,v}^{(m)}|\gamma_{g,v,1}^{(m)}, \gamma_{g,v,2}^{(m)})$$

$$q_Y(\boldsymbol{Y}) \quad = \quad \prod_{m=1}^{M}\prod_{j=1}^{d^{(m)}} \mathrm{Mul}(Y_{j..}^{(m)}|\tau_j^{(m)})$$

$$q_u(\boldsymbol{u}) \quad = \quad \prod_{v=1}^{V}\prod_{k=1}^{K} \mathrm{Beta}(u_{g,v}|\gamma_{k,v,1}, \gamma_{k,v,2})$$

$$q_Z(\boldsymbol{Z}) \quad = \quad \prod_{v=1}^{V}\prod_{i=1}^{n} \mathrm{Mul}(Z_{i,v}|\eta_{i,v})$$

$$\log q_\Theta(\boldsymbol{\Theta}) \quad = \quad \sum_{m,v,g,k,j,i} \tau_{j,v,g}^{(m)}\eta_{i,v,k} \log p(X_{i,j}^{(m)}|\theta_{v,g,k}^{(m)}) + \sum_{m,v,g,k} \log p(\theta_{v,g,k}^{(m)}) + \mathrm{constant}.$$

Here, a function given by the expression below is considered.

[Equation 11]

$$q_\Theta(\boldsymbol{\Theta}).$$

Hyper parameters except for the above are given by the following equations.

[Equation 12]

$$\gamma_{v,1} = 1 + \sum_{m=1}^{M}\sum_{g=1}^{G}\sum_{j=1}^{d^{(m)}}\tau_{j,g,v}^{(m)}$$

$$\gamma_{v,2} = \alpha_1 + \sum_{m=1}^{M}\sum_{t=v+1}^{V}\sum_{g=1}^{G}\sum_{j=1}^{d^{(m)}}\tau_{j,g,t}^{(m)}$$

$$\gamma_{g,v,1}^{(m)} = 1 + \sum_{j=1}^{d^{(m)}}\tau_{j,g,v}^{(m)}$$

$$\gamma_{g,v,2}^{(m)} = \alpha_2 + \sum_{t=g+1}^{G}\sum_{j=1}^{d^{(m)}}\tau_{j,t,v}^{(m)}$$

$$\gamma_{k,v,1} = 1 + \sum_{i=1}^{n}\eta_{i,v,k}$$

$$\gamma_{k,v,2} = \beta + \sum_{t=k+1}^{K}\sum_{i=1}^{n}\eta_{i,v,t}.$$

[Equation 13]

$$\log \tau_{j,g,v}^{(m)} = \sum_{k=1}^{K} \sum_{i=1}^{n} \eta_{i,v,k} \mathbb{E}_{q(\theta)} [\log p(X_{i,j}^{(m)} | \boldsymbol{\theta}_{v,g,k}^{(m)})]$$

$$+ \psi(\gamma_{v,1}) - \psi(\gamma_{v,1} + \gamma_{v,2})$$

$$+ \sum_{t=1}^{v-1} \{ \psi(\gamma_{t,2}) - \psi(\gamma_{t,1} + \gamma_{t,2}) \}$$

$$+ \psi(\gamma_{g,v,1}^{(m)}) - \psi(\gamma_{g,v,1}^{(m)} + \gamma_{g,v,2}^{(m)})$$

$$+ \sum_{t=1}^{G-1} \{ \psi(\gamma_{t,v,2}^{(m)}) - \psi(\gamma_{t,v,1}^{(m)} + \gamma_{t,v,2}^{(m)}) \}$$

$$+ \text{constant}$$

$$\text{(2)}$$

$$\log \eta_{i,v,k} = \sum_{m=1}^{M} \sum_{g=1}^{G} \sum_{j=1}^{d^{(m)}} \tau_{j,g,v}^{(m)} \mathbb{E}_{q(\theta)} [\log p(X_{i,j}^{(m)} | \boldsymbol{\theta}_{v,g,k}^{(m)})]$$

$$+ \psi(\gamma_{k,v,1}) - \psi(\gamma_{k,v,1} + \gamma_{k,v,2})$$

$$+ \sum_{t=1}^{K-1} \{ \psi(\gamma_{t,v,2}) - \psi(\gamma_{t,v,1} + \gamma_{t,v,2}) \}$$

$$+ \text{constant},$$

where $\mathbb{E}_{q(\theta)}$ denotes averaging with respect to the corresponding $q(\theta)$ of $\boldsymbol{\theta}_{v,g,k}^{(m)}$.

$\varphi(\cdot)$ denotes the digamma function defined as the first derivative of logarithm of gamma function.

Note that $\tau_{j,g,v}^{(m)}$ is normalized over pairs (g, v) for each pair (j, m). On the other hand, $\eta_{i,v,k}$ is normalized over k for each pair of (i, v).

Observation models and prior distribution of parameters $\Theta$ are specified in the following.

(Observation Model)

For observation models, Gaussian distribution, Poisson distribution, and categorical distribution /multinominal distributions are considered. For each cluster block, a univariate distribution of these families is fitted with the assumption that features within the cluster block are independent. Conjugate prior distributions are assumed for the parameters of the distribution families.

(Optimization Algorithm)

With the updating equations of the hyper parameters, calculation proceeds using the variational Bayes EM algorithm as follows.

First, $\{\tau^{(m)}\}_m$ and $\{\eta_v\}_v$ are randomly initialized, and then hyper parameters are updated until the lower bound $L(q(\varphi))$ of Equation (1) converges. This yields a locally optimal distribution $q(\varphi)$ in terms of $L(q(\varphi))$. This procedure is repeated a number of times and the best solution with the largest lower bound as the approximated posterior distribution $q^*(\varphi)$ is selected.

MAP estimates of Y and Z are then evaluated as $\text{argmax}_\gamma q^*_\gamma(Y)$ and argmaxz q*z(Z), respectively.

The lower bound $L(q(\varphi))$ is given by the following equation.

$$\mathcal{L}(q(\phi)) = \int q(\phi) \log p(X|\phi)d\phi - \mathbb{KL}(q(\phi), p(\phi)) \qquad (3)$$

[Equation 14]

Both terms on the right side can be derived in closed forms. It can be shown that this monotonically increases as q($\varphi$) is optimized. Specifically, as already described, the function L(q($\varphi$)) has a property of monotonous increase as Y and Z are updated, and it is determined to be converged if the rate of increase is determined to be sufficiently small (for example, though not limiting, when a condition that the increment attains to a prescribed value or smaller is satisfied).

First, a distribution family for each feature is identified, generating a data matrix for the corresponding distribution family. Then, MAP estimates of Y and Z are generated for a set of these data matrices, and object/feature clusters in each view are analyzed using the estimates of Y and Z.

(Model Expression)

The multiple co-clustering model is sufficiently flexible to represent different clustering models because the number of views and the number of feature/object clusters are derived in a data-driven approach. For example, when the number of views is one, the model coincides with a co-clustering model. When the number of feature clusters is one for all views, it matches the multiple-clustering model. Furthermore, when the number of views is one and the number of feature clusters is the same as the number of features, it matches conventional mixture models with independent features. Moreover, the model can detect non-informative features that do not discriminate between object clusters. In such a case, the model yields a view in which the number of object clusters is one. The advantage of this model is to automatically detect such underlying data structures.

The "multiple co-clustering method" as described above enables the following.

1) To identify the manner of partitioning a plurality of clusters behind the data (not only the manner of partitioning objects but also the manner of partitioning features) and the corresponding groups of features in a data-driven manner;
2) to identify a cluster that could not be found by any other method; and
3) to attach meaning to the manner of partitioning clusters by feature, making it easier to interpret each cluster.

[Evaluation of Dataset Clustering Results]

In the following, generalization performance of clustering is verified by first dividing the multi-facility, large scale fMRI data collected from a large number of subjects published as SRPBS mentioned above into two, and then applying the above-described multiple co-clustering to each of the divided data.

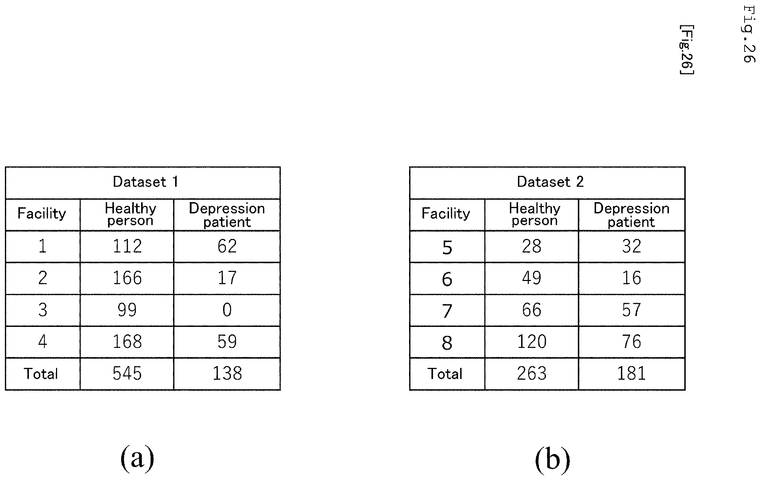

Fig. 26 shows Dataset 1 and Dataset 2 of a dataset divided into two.

Dataset 1 includes data of 545 healthy persons and 138 patients of depression disorder obtained at Facilities 1 to 4, and Dataset 2 includes data of 263 healthy persons and 181 patients of depression disorder obtained at Facilities 5 to 8. Basically, these correspond to the datasets shown in Figs. 10 and 11.

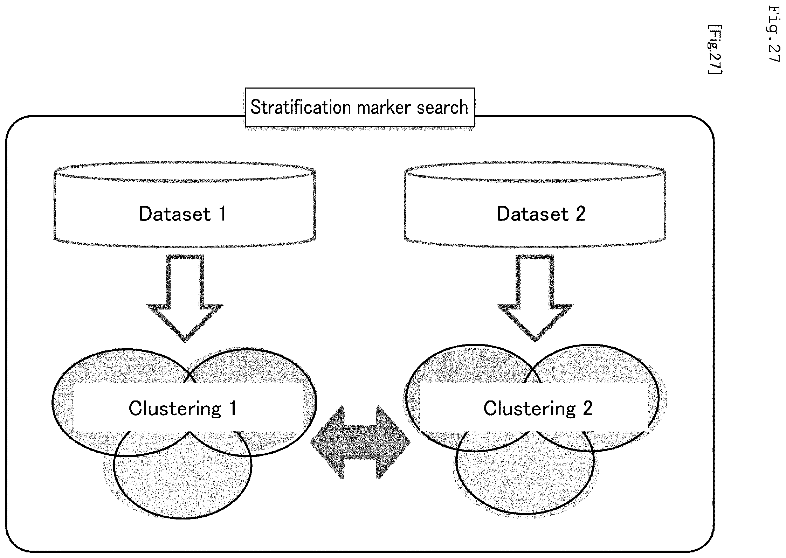

Fig. 27 is an illustration showing a concept of clustering performed on each dataset.

As shown in Fig. 27, Dataset 1 is clustered by multiple co-clustering in accordance with the flow shown in Fig. 24 independently.

Here, what is of interest is the degree of similarity between the clusters obtained by data-driven clustering independently performed on Datasets 1 and 2 (how similar the clusters are to each other).

If clustering of Datasets 1 and 2 results in classification (forming groups) to clusters having the same or similar characteristics (groups of subjects), it means that such a data-driven clustering is performed with high generalization performance, independent of facilities or measuring devices. Here, what matters is how to quantitatively evaluate the attained degree of the "classification to clusters having the same or similar characteristics."

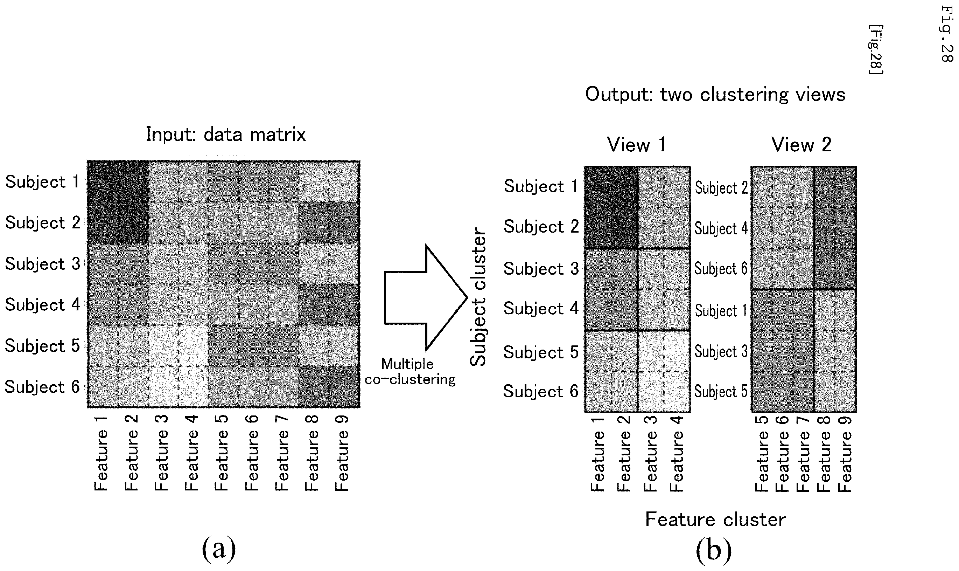

Fig. 28 is an illustration showing an example of multiple co-clustering of subject data.

Referring to Fig. 28(a), it is assumed that an input data matrix has subjects arranged along the row direction and features along the column direction.

By way of example, multiple co-clustering on the input data matrix results in features divided into two views, and the subjects are clustered in each view, as shown in Fig. 28(b).

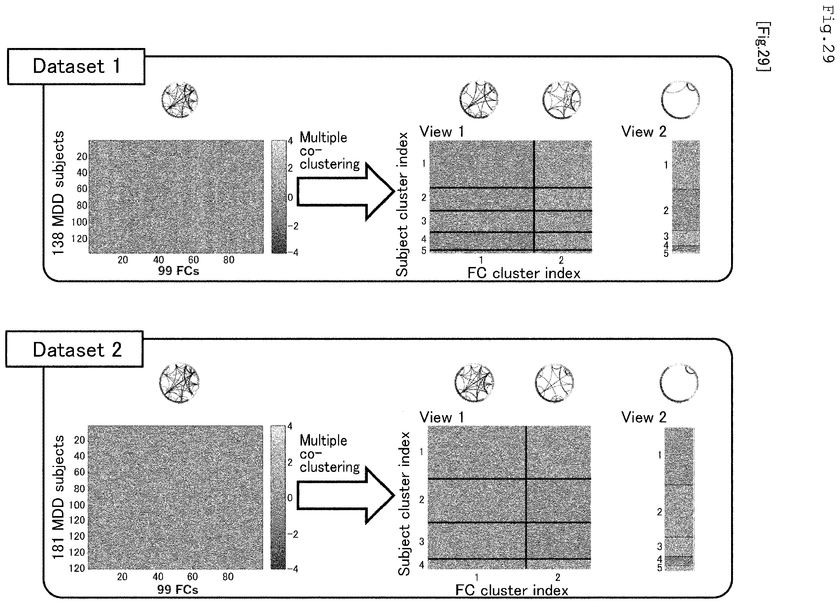

Fig. 29 shows results of multiple co-clustering actually performed on Datasets 1 and 2.

In Fig. 29, 99 features are selected for each of Datasets 1 and 2 as features for clustering.

Then, multiple co-clustering is performed on 138 patients of depression disorder in Dataset 1 and on 181 patients of depression disorder in Dataset 2.

In Dataset 1, the features are partitioned into two views, that is, View 1 and View 2. In View 1, the features are further co-clustered into two feature clusters, and the subjects are partitioned into five subject clusters. In View 2 also, the subjects are partitioned into five clusters.

In Dataset 2 also, the features are partitioned into two views, that is, View 1 and View 2. In View 1, the features are further co-clustered into two feature clusters, and the subjects are partitioned into four subject clusters. In View 2, the subjects are partitioned into five clusters.

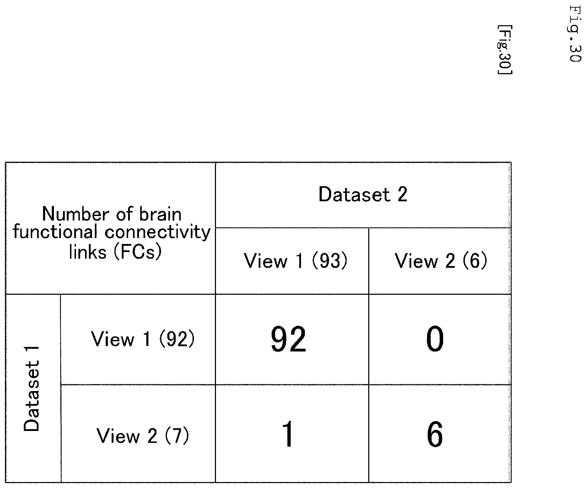

Fig. 30 shows, in the form of a table, counts of brain functional connectivity links (FCs) allocated to respective views of Datasets 1 and 2.

In Dataset 1, 92 FCs are allocated to View 1 and 7 FCs to View 2 as features.

In Dataset 2, 93 FCs are allocated to View 1 and 6 FCs to View 2 as features.

Further, in this table, the numbers of allocated brain functional connectivity links that match in Datasets 1 and 2 are plotted on the diagonal of the table. It can be seen that the brain functional connectivity links allocated to View 1 and View 2 are substantially the same in Datasets 1 and 2.

(Method of Verifying Generalization Performance (Similarity Between Datasets) of Clustering (Stratification))

The following will quantitatively evaluate the similarity between the clusters (the degree of agreement), which are obtained by data-driven clustering individually performed on Datasets 1 and 2.

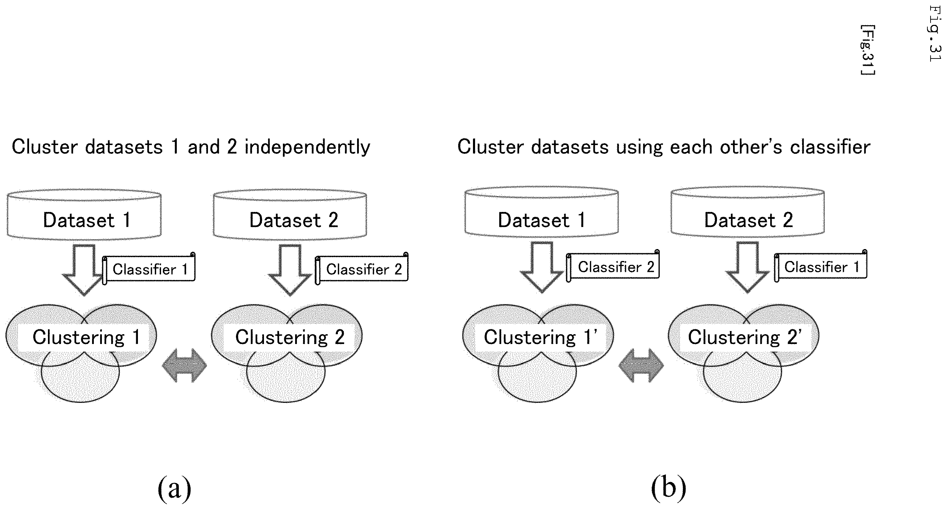

Fig. 31 illustrates methods of evaluating similarity of clustering (generalization performance of stratification).

First, referring to Fig. 31(a), it is assumed that Datasets 1 and 2 are partitioned into clusters independently by multiple co-clustering as described above. In this case, it is difficult to compare similarity of clustering, since the subjects are independent from each other in the clusters of respective datasets.

Here, the result of classification of subjects in Dataset 1 using a classifier 1 formed by Dataset 1 is denoted as Clustering 1. On the other hand, the result of classification of subjects in Dataset 2 using a classifier 2 formed by Dataset 2 is denoted as Clustering 2.

Meanwhile, referring to Fig. 31(b), the result of classification of subjects in Dataset 2 using a classifier 1 formed by Dataset 1 is denoted as Clustering 1'. On the other hand, the result of classification of subjects in Dataset 1 using a classifier 2 formed by Dataset 2 is denoted as Clustering 2'.

Here, subjects of classification are common between Clustering 1 and Clustering 1' and between Clustering 2 and Clustering 2' and, therefore, it is possible to evaluate similarities between them.

(Evaluation Standard of Measuring Degree of Similarity (Recall of Clustering) between Clustering Processes)

Here, clustering process is data-driven and the values of cluster indexes (order of index values) of Fig. 29 are of no significance. Therefore, the problem is how to evaluate the degree of similarity when one same dataset is subjected to different clustering processes.

For example, it is assumed that there are two results of clustering $\pi$ and $\rho$ on the same dataset X. Rand index is known as a metric for evaluating the similarity (external validity index) between the two results of clustering.

When all data pairs $\{x_1, x_2\} \in X$ ($M = N(N-1)/2$) in a dataset are considered, there are the following types of pairs, and the number of pairs of respective types will be defined as follows.

[Equation 15]

an: The number of pairs belonging to the same cluster in both $\pi$ and $\rho$

$a_{01}$: The number of pairs belonging to different clusters in $\pi$ and belonging to the same cluster in $\rho$

$a_{10}$: The number of pairs belonging to different clusters in $\rho$ and belonging to the same cluster in $\pi$

$a_{00}$: The number of pairs belonging to the different clusters in both $\pi$ and $\rho$

Here, as the accuracy rate of determining whether or not the clusters classified by two different clustering processes agree, Rand index is defined as follows.

[Equation 16]

$$\frac{a_{11} + a_{00}}{M}.$$

It is known, however, that "the Rand index may sometimes have high value even when random clustering is done" if, for example, the numbers of components in clusters in a dataset are biased. Therefore, more strictly, the following Adjusted Rand Index (ARI) is used.

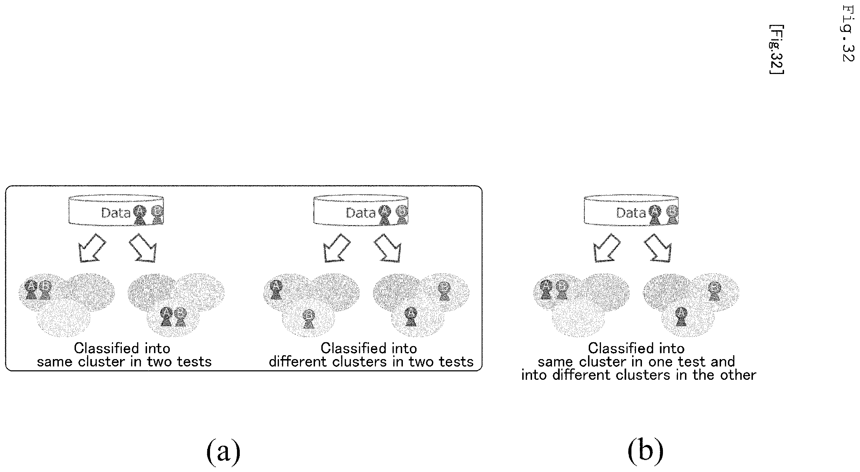

Fig. 32 illustrates concepts of ARI.

ARI is disclosed, for example, in the reference below.

Reference: Jorge M. Santos and Mark Embrechts, "On the Use of the Adjusted Rand Index as a Metric for Evaluating Supervised Classification", ICANN 2009, Part II, LNCS 5769, pp. 175-184, 2009.

As described above, when the results of two clustering tests are to be applied to the same dataset, the dataset may be classified into the same clusters in both tests, or the dataset may be classified into different clusters in both tests as shown in Fig. 32(a). Meanwhile, the dataset may be classified into one same cluster once but to different clusters the next time, as shown in Fig. 32(b).

ARI is calculated by first calculating an expected value that a data pair is classified to the same cluster twice or classified to different clusters twice when subjected to two clustering processes, assuming that the two clustering processes are independent from each other, and then subtracting the expected value from the numerator and the denominator of the Rand Index. Therefore, ARI is adjusted to have the value 0 when the clustering processes are not correlated.

[Equation 17]

$$\mathrm{ARI} = \frac{A - E}{\max(A) - E}.$$

Here, A represents the number of subject pairs that are [(classified to the same cluster twice over) + (classified to different clusters twice over)] through two different clustering processes, max (A) represents the total number of pairs, and E represents the number of subject pairs having the same results of allocation even though the two clustering processes are independent from each other.

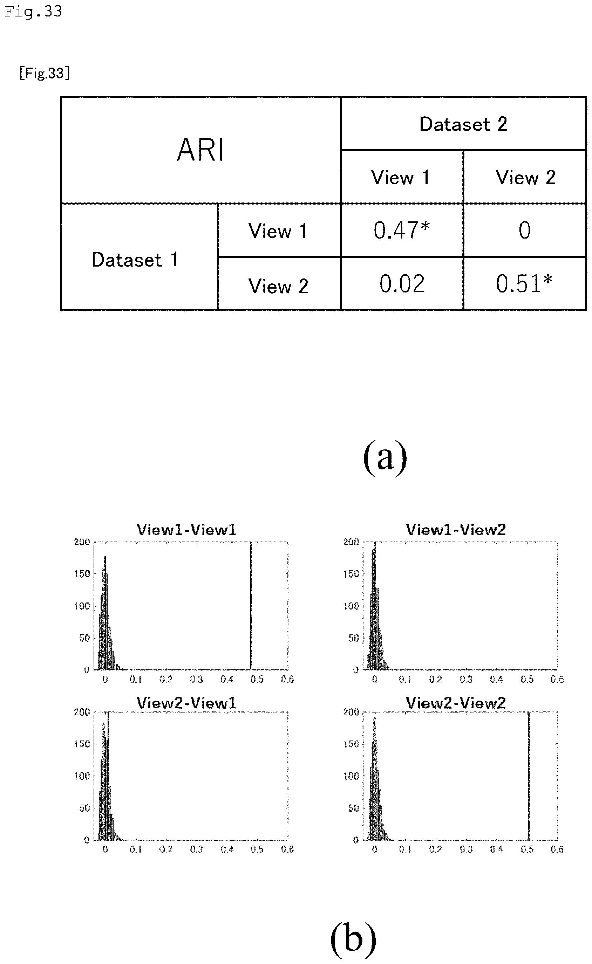

Fig. 33 shows results of similarity evaluation between Clustering 1 and Clustering 1' and between Clustering 2 and Clustering 2', respectively.

Fig. 33(a) shows, in the form of a table, ARIs calculated for respective views of Datasets 1 and 2.

Regarding Datasets 1 and 2, ARI =0.47 for View 1 and ARI = 0.51 for View 2, indicating that there are significant similarities.

Fig. 33(b) shows results of Permutation Test corresponding to Fig. 33(a). As regards Views 1 and 2, as compared with the case in which components are exchanged (represented by the histograms), ARI values (represented by solid lines) are higher with statistical significance in View 1 and View 2.

"Permutation Test" represents the results of ARI value calculations while cluster attribute labels of subjects are exchanged at random, and the drawing shows the distributions thereof as histograms when such exchange was conducted a number of times. If similarity between clustering processes is statistically significant, the ARI value between the clustering processes as the object of comparison will have a value higher than when the components are exchanged at random with significance.

From the foregoing, it is determined that clustering processes (stratifications) between the datasets have similarities with significance, or that generalized clustering is realized.

As already described, multiple co-clustering of Dataset 1 and multiple co-clustering of Dataset 2 are both realized in data-driven manner and, therefore, both can serve as a basis for "stratification of patients" using brain functional connectivity links as features.

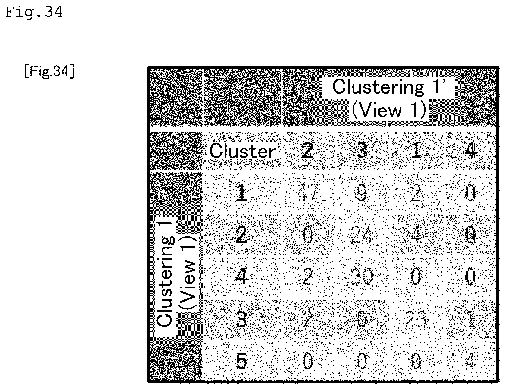

Fig. 34 shows, in the form of a table, distribution of subjects allocated to respective clusters of View 1 in Clustering 1 and Clustering 1'.

By appropriately rearranging subject cluster indexes, it is possible to distribute most of the subjects near the diagonal of the table, and thus, it can visually be recognized that the two clustering processes are similar to each other.

[Harmonization Process]

In the following, the contents of a process referred to as harmonization in Fig. 6 and disclosed in the following references will be described.

[Harmonization in Accordance with Traveling Subject Method]

The following describes a method used for generating a "disease identifier" and a "clustering process" for stratification as described above, for evaluating a measurement bias and harmonizing measurement data independent of a sampling bias.

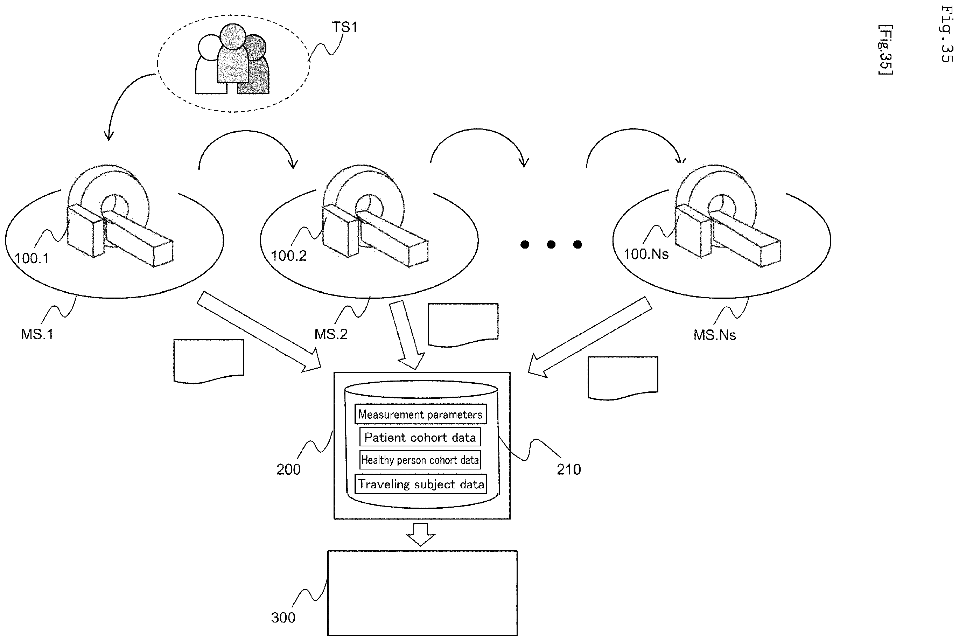

Fig. 35 is an illustration showing a method of evaluating the site-to-site differences of subjects (hereinafter referred to as "traveling subjects"), who travel to be subjected to measurements at sites by rs-fcMRI in accordance with the present embodiment.

As will be described later, in the present embodiment, a harmonization method that can remove only the measurement bias by using the traveling subject dataset will be described.

Referring to Fig. 35, in order to evaluate the measurement bias across measurement sites MS.1 to MS.Ns, a dataset of travelling subjects TS1 (number of subjects: Nts) is obtained.

Assume that the resting state brain activities of Nts healthy participants are imaged at each of the Ns sites, and the Ns sites include all sites where patients' data are imaged.

The obtained dataset of the traveling subjects is stored as the traveling subject data in storage device 210 of data center 200.

Then, as will be described later, the process for the "harmonization of the brain activity biomarkers" is performed by computing system 300.

The traveling subject dataset includes only the healthy person cohort. Further, it is assumed that the participants are the same in every site. Therefore, the site-to-site differences in traveling subjects consist only of the "measurement bias."

In the method of harmonization of the present embodiment described in the following, a process of correcting the measurement data at each measurement site by removing the influence of the "measurement bias" is performed as the method of the "harmonization of brain activity biomarkers."

Specifically, in the following, the "measurement bias" and the "sampling bias" are evaluated using "GLMM (Generalized Linear Mixed Model)," which is one of the methods of "statistical modeling."

Here, typically, GLM (Generalized Linear Model) incorporates the "explanatory variable" explaining the probability distribution of the "response variable." GLM mainly has three elements, that is, the "probability distribution," the "link function," and the "linear predictor." It is possible to represent various different types of data by designating how to combine these three elements.

Further, GLMM (Generalized Linear Mixed Model) is a statistical model that allows the incorporation of "individual differences that cannot or is not measured by humans" that cannot be explained by GLM. For example, when objects consist of a number of subsets (for example, subsets taken from different measurement sites), GLMM allows incorporation of the differences in sites in the model. In other words, it is a (mixture) model having a plurality of probability distributions as elements.

By way of example, the reference below discloses GLMM.

Reference 8: Takuya KUBO, "Data kaiseki no tameno toukei modeling nyu'mon" (Introduction to statistical modeling for data analysis), Iwanami Shoten, 1st edition 2012, 14th edition, 2017.

In the statistical model in accordance with the present embodiment described below, typically, what is referred to as "effect" will be described using the terms "bias" and "factor"; "bias" will be used in the terms "measurement bias" and "sampling bias"; and "factor" will be used in other factors (subject factor or disease factor).

In the following, factors are analyzed without discriminating a "fixed effect" from a "random effect," different from a simple flow of GLMM. The reason for this is that, generally, when GLMM is used, only the variance is estimated for the random effect and the magnitude of effect of each factor will be unknown. Therefore, in order to evaluate the magnitude of each factor's effect, estimation is done with variables transformed as shown below, so that each factor will have a fixed effect average of zero.

    i) The measurement bias of each site is defined as a deviation from the average correlated values for the various functional connectivity across all sites.

    ii) Sampling biases of healthy persons and patients of mental diseases are assumed to be different from each other. Therefore, the sampling bias of each site will be calculated individually for the healthy person cohort and patients' cohorts of various diseases.

    iii) Disease factor is defined as a deviation from the value of healthy person cohort.

Specifically, in the following, Generalized Linear Mixed Model is applied in the following manner to the dataset including patients and to traveling subjects' dataset.

Assume that there are Nts traveling subjects, and of Ns measurement sites, Nsh represents the number of sites where the measurement of healthy persons was done, and Nsd represents the number of sites where the measurement of patients of a certain disease (here, represented by a suffix "dis") was done.

Participant factor (p), measurement bias (m), sampling biases (Shc, Sdis) and mental disease factor (d) are evaluated by fitting a regression model to the correlation values of the functional connectivity of all participants, from the measurement result dataset of patients and the traveling subjects' dataset.

In the following, a vector is denoted by a small letter (for example, m), and it is assumed that every vector is a column vector.

A vector element is denoted by a suffix such as $m_k$.

A regression model of a functional connectivity vector (assumed to be a column vector) consisting of n correlation values among brain regions is represented as follows.

[Equation 18]

$$Connectivity = x_m^T m + x_{Shc}^T s_{hc} + x_{Sdis}^T s_{dis} + x_d^T d + x_p^T p + const + e$$

$$\sum_j^{N_{ts}} p_j = 0, \quad \sum_k^{Ns} m_k = 0, \quad \sum_k^{Nsh} s_{hc\,k} = 0, \quad \sum_k^{Nsd} s_{dis\,k} = 0, \quad d_1(HC) = 0$$

In order to represent characteristics of each participant, a binary code system of 1-of-k is used, and every target vector (for example, $x_m$) for a measurement bias m belonging to a site k has elements all equal to zero except for the element k that is equal to one.

If a participant does not belong to any class (healthy person, patient, traveling subject), the target vector will be a vector of which elements are all equal to zero.

Upper suffix T represents transpose of matrix or vector, and $x^T$ represents a row vector.

Here, m represents the measurement bias (column vector of Ns × 1), shc represents the sampling bias of healthy person cohort (column vector of Nsh × 1), sdis represents the sampling bias of a patient (column vector of Nsd × 1), d represents the disease factor (column vector of 2 × 1, having healthy and disease as elements), p represents the participant factor (column vector of Nts × 1), const represents an average of functional connectivity of all participants (including healthy persons, patients, and traveling subjects) at all measurement sites, and e ~ N (0, $\gamma^{-1}$) represents noise.

Here, for simplicity of description, only one type of disease is assumed. An example involving a plurality of diseases will be described later.

As to the correlation value of each functional connectivity, the design matrix of the regression model does not have sufficient rank and, therefore, respective parameters are evaluated using the least square regression by L2 normalization. Other than the least square regression by L2 normalization, a different method of evaluation such as Bayesian estimation may be used, for example.

After the above-described regression calculation, b-th connectivity of a subject a can be given as follows.

[Equation 19]

$$Connectivity_{a,b} = x_m^{a\ T} m^b + x_{Shc}^{a\ T} s_{hc}^b + x_{Sdis}^{a\ T} s_{dis}^b + x_d^{a\ T} d^b + x_p^{a\ T} p^b + const + e.$$

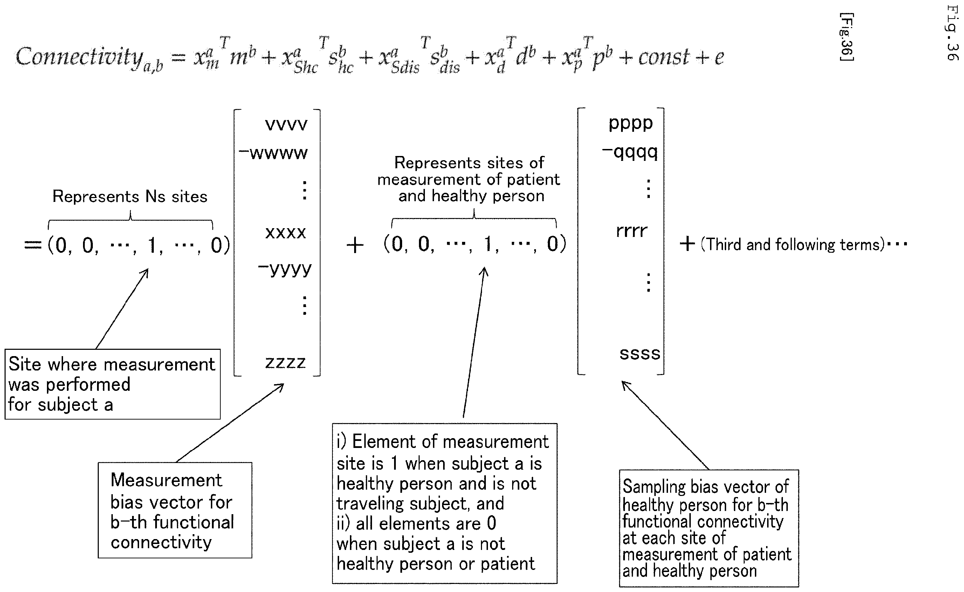

Fig. 36 is an illustration showing how to express b-th functional connectivity of a subject a.

Fig. 36 shows the meanings of target vectors of the first term and the second term as well as the measurement bias vector and the sampling bias vector of a healthy person.

The same applies to the third and the following terms.

(Process Flow of Harmonization)

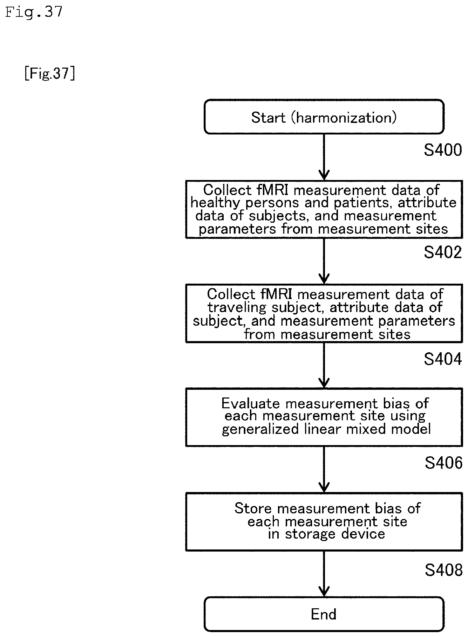

Fig. 37 is a flowchart showing a process of calculating a measurement bias for harmonization.

First, fMRI measurement data of subjects (healthy persons and patients), attribute data of subjects, and measurement parameters are collected from respective measurement sites to storage device 210 of data center 200 (Fig. 37 S402).

Thereafter, brain activities of traveling subject TS1 are measured while the subject travels from measurement site to measurement site, though not limiting, in a prescribed period (for example, in a one-year period) and the fMRI measurement data of the traveling subject, attribute data of the subject, and measurement parameters are collected from respective measurement sites to storage device 210 of data center 200 (Fig. 37 S404).

Using GLMM (Generalized Linear Mixed Model) as described above, the harmonization calculating unit 3020 evaluates the measurement bias of each measurement site with respect to the functional connectivity (Fig. 37 S406).

The harmonization calculating unit 3020 stores the measurement bias of each measurement site calculated in this manner in storage device 2080, as measurement bias data 3108 (Fig. 37 S408).

(Harmonization in Discriminator Generating Process)

Harmonization of brain functional connectivity values in the process of generating a disease identifier for disease or healthy label for the subjects by discriminating unit 3000 will be briefly described.

Such a disease identifier provides assisting information (support information) for diagnosing a subject.

A correlation value correcting unit 3004 reads measurement bias data 3108 of each measurement site stored in storage device 2080, and performs the harmonization process for the off-diagonal components of the correlation matrix of each subject as the object of training for machine learning to generate a disease identifier in accordance with the equation below.

[Equation 20]

$$C_{sub} = Connectivity - x_m{}^T \widehat{m}.$$

Here, functional connectivity "Connectivity" represents the functional connectivity vector before harmonization, and Csub represents the functional connectivity vector after harmonization. Further, m (hat) (a character x with "^" above will be referred to as "x (hat)") represents the measurement bias at a measurement site evaluated by the least square regression by L2 normalization as described above. Thus, the measurement bias corresponding to the measurement site at which functional connectivity "Connectivity" has been measured will be subtracted from the functional connectivity "Connectivity," and hence, subjected to the harmonization process.

Data after the correction is stored as corrected correlation value data 3110 in storage device 2080.

The method of removing a bias between measurement sites is not limited to the method based on the traveling subjects discussed above. For example, other methods such as the ComBat method may also be used.

[Second Embodiment]

In the configuration described in the first embodiment, the brain activity measuring devices (fMRI devices) measure data of brain activities obtained at a plurality of measuring sites, and generation of biomarkers and estimation (prediction) of diagnosis labels by the biomarkers are realized based on the brain activity data by distributed processing.

It is noted, however, that the following process steps may be performed in a distributed manner at different facilities: i) the measurement (data collection) of brain activity data for training a biomarker through machine learning; ii) the process of generating a biomarker through machine learning and the process (estimation process) of estimating (predicting) diagnosis labels by the biomarker for a specific subject (object for estimation; hereinafter referred to as a "first subject"); and iii) the measurement of brain activity data of the specific subject above (measurement of brain activities of the first subject).

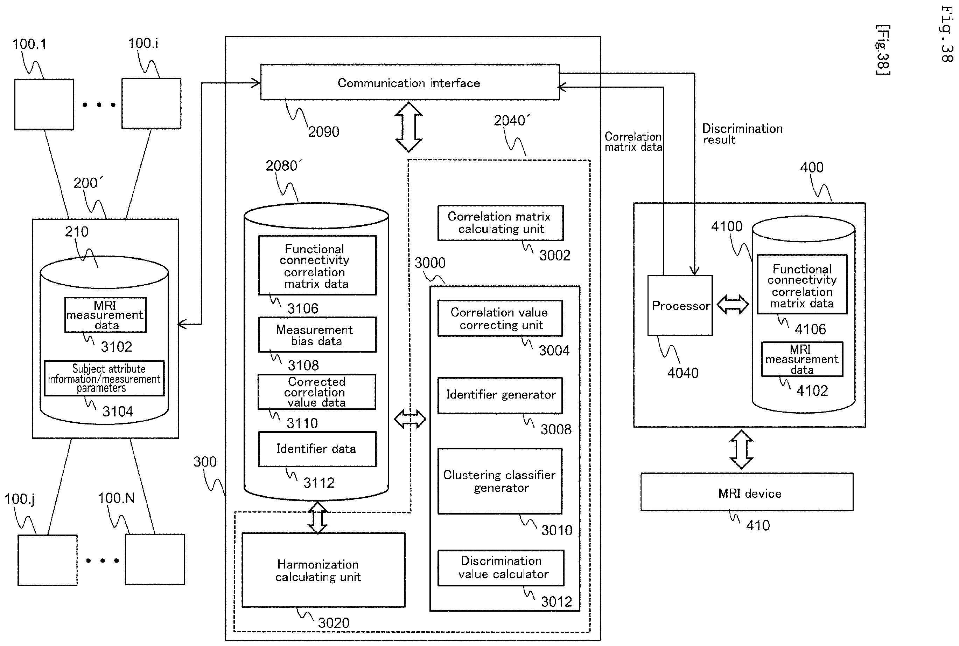

Fig. 38 is a functional block diagram showing an example in which the data collection, the estimating process, and the measurement of brain activities of the object are processed in a distributed manner.

Referring to Fig. 38 sites 100.1 to 100.N represent facilities at which data of patient cohort and healthy person cohort (i.e. second subject cohort) are measured by brain activity measuring devices, and a management server 200 manages measurement data from sites 100.1. to 100.Ns.

Computing system 300 generates an identifier from the data stored in server 200.

Harmonization calculating unit 3020 of computing system 300 is assumed to perform the harmonization process including sites 100.1 to 100.Ns and the site of MRI measuring device 410.

An MRI device 410 is provided at a separate site that utilizes the results from the identifier on computing system 300, and measures data of the brain activities of a specific subj ect.

A computer 400 is provided on a separate site where MRI device 410 is installed, and calculates the correlation data of the brain functional connectivity of the specific subject from the measurement data of MRI device 410, sends the correlation data of functional connectivity to computing system 300, and utilizes the results from the identifier that are sent back.

Server 200 stores the MRI measurement data 3102 of patient cohort and healthy person cohort transmitted from sites 100.1 to 100.Ns as well as the human attribute information 3104 of the subject associated with the MRI measurement data 3102, and transmits these data to computing system 300 in accordance with an access from computing system 300.

Computing system 300 receives MRI measurement data 3102 and human attribute information 3104 of the subject from server 200 through a communication interface 2090.

Hardware configurations of server 200, computing system 300, and computer 400 are basically the same as the configuration of "data processing unit 32" described with reference to Fig. 5 and, therefore, description thereof will not be repeated here.

Returning to Fig. 38, a correlation matrix calculating unit 3002, correlation value correcting unit 3004, disease identifier generator 3008, clustering classifier generator 3010, and discrimination value calculator 3012, as well as correlation matrix data 3106 of functional connectivity, measurement bias data 3108, corrected correlation value data 3110, and identifier data 3112 are the same as those described in the first embodiment and, therefore, description thereof will not be repeated here.

MRI device 410 measures the brain activity data of a subject whose diagnosis label is to be estimated, while processor 4040 of computer 400 stores the measured MRI measurement data 4102 in a non-volatile storage device 4100.

Further, a processor 4040 of computer 400 calculates functional connectivity correlation matrix data 4106 in the similar manner as correlation matrix calculating unit 3002, based on MRI measurement data 4102, and stores the calculated data in non-volatile storage device 4100.

A disease as an object of diagnosis is designated by a user of computer 400, and computer 400 transmits the functional connectivity correlation matrix data 4106 to computing system 300 in accordance with an instruction of transmission by the user. In response, computing system 300 performs the harmonization process corresponding to the site where the MRI device 410 is installed. Discrimination value calculator 3012 calculates the result of discrimination on the designated diagnosis label and evaluation result on subtypes, and computing system 300 transmits the results to computer 400 through communication interface 2090.

Computer 400 informs the user of the result of the discrimination using a display device (not shown) or the like.

By such a configuration, it is possible to provide the result of the estimation of the diagnosis label by the discriminator, based on the data collected from a larger number of subj ects.

Further, server 200 and computing system 300 may be managed by separate administrators. In that case, it becomes possible to improve the security of subject information stored in server 200 by limiting the computers that can access server 200.

Further, from the viewpoint of the operator of computing system 300, the "service of providing result of the discrimination" becomes possible while not providing any information at all of the identifier or information related to the "measurement biases" to the "side (computer 400) receiving the service of discrimination by the identifier."

In the descriptions of the first embodiment and the second embodiment above, it is assumed that real-time fMRI is used as the brain activity detecting apparatus for timesequentially measuring brain activities by functional brain imaging. It is noted, however, that any of the fMRI described above, a magnetoencephalography, a near-infrared spectroscopy (NIRS), and an electroencephalography or any combination of these may be used as the brain activity detecting apparatus. Regarding such a combination, it is noted that fMRI and NIRS detect signals related to change in blood stream in the brain, and have a high spatial resolution. On the other hand, magnetoencephalography and electroencephalography are characterized in that they have a high temporal resolution, for detecting changes in an electromagnetic field associated with the brain activities. Therefore, if fMRI and the magnetoencephalography are combined, brain activities can be measured with both spatially and temporally high resolutions. Alternatively, by combining NIRS and the electroencephalography, a system for measuring the brain activities with both spatially and temporally high resolutions can also be implemented in a small, portable size.

By the configurations above, it is possible to realize a brain activity analyzing apparatus and a brain activity analyzing method functioning as a biomarker using brain function imaging for neurological/mental disorders.

In the foregoing, an example has been described in which a "diagnosis label" is included as an attribute of a subject and, by generating an identifier through machine learning, the identifier is caused to function as a biomarker. The present invention, however, is not necessarily limited to such an example. Provided that a group of subjects whose results of measurements are to be obtained as the object of machine learning is classified into a plurality of classes in advance by an objective method, the correlation of degrees of activities (connection) among brain regions (regions of interest) of the subjects are measured, and an identifier for classification can be generated by machine learning using the measured results, the present invention may be used for other discrimination.

Further, as mentioned above, such a discrimination may indicate probability of the subject having a certain attribute.

Therefore, it is possible to objectively evaluate whether or not taking a certain "training" or following a certain "behavior pattern" is effective to improve the health of a subject, for example. Further, it is also possible to objectively evaluate whether certain ingestions of "food" or "drink" or a certain activity or activities are effective to reach a healthier state before the actual onset of a disease (in the state of being "not diseased yet").

Further, even before the onset of a disease, it is possible to provide the user with an objective numerical value of his/her state of health if an indication such as "probability of being healthy: XX%" is output, for example, as mentioned above. Here, the output may not necessarily be the probability. For example, "a continuous value of degree of healthiness, such as probability of being healthy" converted to a score may be displayed. By providing such a display, the apparatus in accordance with the embodiment of the present invention can be used as an apparatus for health management of users, besides diagnosis assistance.


[Third Embodiment]


[Therapy Selection Support System]

(Configuration of Therapy Selection Support System)


An embodiment of the present invention relates to a therapy selection support system 1000 that provides information related to selection of a therapy for a subject with depression symptoms on the basis of the results of measurement of brain activities of the subject.

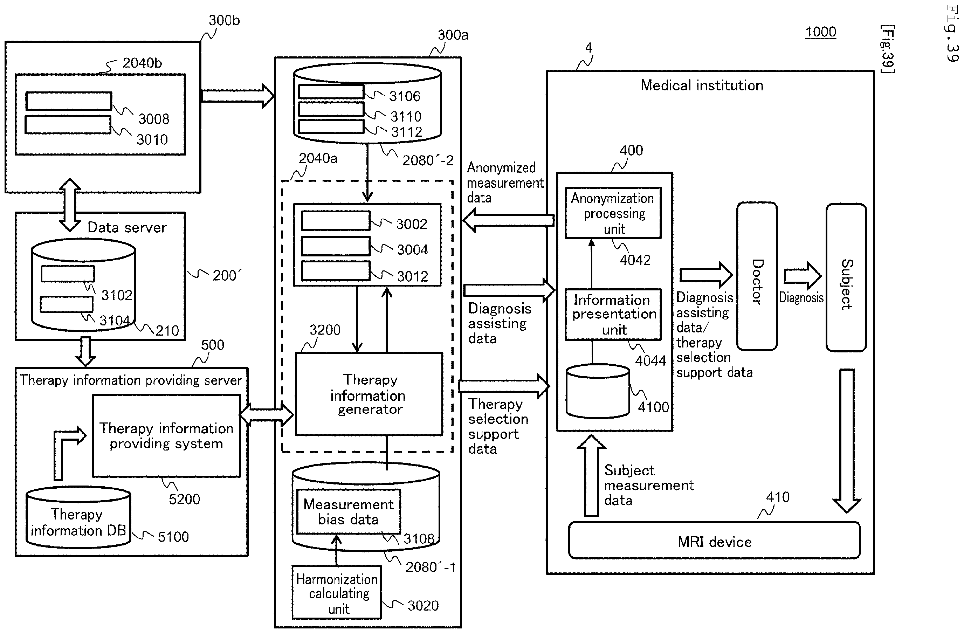

Fig. 39 shows the functional configuration of the therapy selection support system 1000.

The therapy selection support system 1000 includes a computer 400 provided in a medical institution 4 and connected so as to be able to receive data from an MRI device 410 that measures brain activity data of a subject whose diagnosis label is to be estimated, a support information providing device 300a, a classifier generation device 300b that performs a process of generating a clustering classifier, a data server 200', and a therapy information providing server 500.

With reference to Fig. 39, a healthy person/patient database in a storage device 210 in the data server 200' manages MRI measurement data 3102 collected from sites at which MRI devices 100.1 to 100.N (not shown) are set and human attribute information 3104 of the subj ect.

The support information providing device 300a corresponds in configuration to the computing system 300 shown in Fig. 38 except that: i) the support information providing device 300a receives an input of the results of measurement of brain activities of the subject transmitted from the computer 400 in the medical institution 4, and a processor 2040a executes a process of a therapy information generator 3200 to output corresponding therapy information in accordance with the results of classification by a clustering classifier executed as a discrimination value calculator 3012 for the measurement results; and ii) the processes of a disease identifier generator 3008 and a clustering classifier generator 3010 are executed by a processor 2040b in the classifier generation device 300b which is a computing system that is different from the support information providing device 300a.

As in Fig. 38, the support information providing device 300a and the classifier generation device 300b may be implemented as functions on an identical computer system.

The classifier generation device 300b generates a disease identifier and a clustering classifier using data stored in the server 200' as "training data" (or "discovery cohort data"). Data stored in the server 200' (other than the training data) can also be used as validation data (or "validation cohort data") for the disease identifier and the clustering classifier. In the configuration in Fig. 39, the healthy person/patient database in the storage device 210 of the server 200' does not only store the MRI measurement data 3102 and the human attribute information/measurement parameters 3104 of the subject, but also stores functional connectivity correlation matrix data calculated on the basis of the MRI measurement data 3102 and data on correlation values corrected through a harmonization process calculated in advance, although this is not particularly limiting. It is assumed that the disease identifier generator 3008 and the clustering classifier generator 3010 execute a learning process and a validation process on the basis of the corrected correlation values. The classifier generation device 300b itself may execute the processes of calculating functional connectivity correlation matrix data and correcting the correlation values through a harmonization process.

Basically, other portions that are the same as the components in Fig. 38 are given the same reference numerals.

Data acquired by the MRI device 410 may be added to the healthy person/patient database in the storage device 210 to re-train the disease identifier and the clustering classifier, although this is not particularly limiting.

A harmonization calculating unit 3020 of the support information providing device 300a executes a harmonization process for the MRI measurement device 410 for the MRI devices 100.1 to 100.Ns (not shown).

The MRI device 410 is provided in the medical institution 4 which uses the results from the clustering classifier of the support information providing device 300a, and measures brain activity data for a specific subject. Image data (including brain structure image data and brain function image data) measured for the specific subject are subjected to an anonymization process in an anonymization processing unit 4042 of the computer 400, and thereafter transmitted to the support information providing device 300a.

A so-called cloud computer may be used for the support information providing device 300a, although this is not particularly limiting. The computer 400 may be configured to calculate functional connectivity correlation data for the brain of a specific subject from measurement data from the MRI device 410, and to transmit the functional connectivity correlation data to the support information providing device 300a, although this is also not particularly limiting. In this case, conversion of image data into functional connectivity correlation data itself is equivalent to anonymization.

The therapy information generator 3200 receives data in a therapy information DB 5100 in the therapy information providing server 500 from a therapy information providing system 5200, and returns corresponding therapy selection support data to an information presentation unit 4044 in accordance with the results of classification by the clustering classifier. The therapy information generator 3200 may be configured to receive from the therapy information providing system 5200 and store in advance information on the correlation between the classification results and therapy selection support data. Alternatively, the therapy information generator 3200 may be configured to transmit information on the classification results to the therapy information providing system 5200 as inquiry information as occasion requires, and to receive therapy selection support data returned from the therapy information providing system 5200 as an answer to the inquiry.

The therapy information providing server 500 may be managed by a pharmaceutical company that develops therapeutic agents for diseases or a medical device manufacturer that develops therapy devices, for example, although this is not particularly limiting.

The hardware configuration of the server 200', the support information providing device 300a, the classifier generation device 300b, and the computer 400 is basically the same as the configuration of the "data processing unit 32" described with reference to Fig. 5, and thus description thereof will not be repeated here.

Returning to Fig. 39, the correlation matrix calculating unit 3002, the correlation value correcting unit 3004, the disease identifier generator 3008, the clustering classifier generator 3010, and the discrimination value calculator 3012 and the functional connectivity correlation matrix data 3106, the measurement bias data 3108, the corrected correlation value data 3110, and the identifier data 3112 are the same as those described in relation to the first embodiment, and thus description thereof will not be repeated here.

The disease identifier generator 3008, the clustering classifier generator 3010, and the storage device 210 constitute a clustering device. The disease identifier generator 3008 and the clustering classifier generator 3010 are functions executed by the processor 2040b of the clustering device.

The MRI device 410 measures brain activity data of a subject to be subjected to stratification by the clustering classifier. The computer 400 stores the measured MRI measurement data in a non-volatile storage device 4100.

The computer 400 transmits the measurement data on the subject anonymized by the anonymization processing unit 4042 to the support information providing device 300a as the brain activity measurement result in accordance with an instruction for transmission from a user (e.g. a doctor). At this time, the measurement data to be transmitted are provided with a temporary ID for specifying the subject. Preferably, a table of correspondence between the temporary ID and personal information such as a patient name, for example, can be managed so as to be inaccessible from the computer 400. The content of the table of correspondence is configured to be not accessible at all at least in the support information providing device 300a.

Accordingly, a harmonization process corresponding to the site at which the MRI device 410 is installed is preferably executed, as necessary, in the support information providing device 300a. Further, the discrimination value calculator 3012 outputs the classification results, which is the results of stratification by the clustering classifier, for the input measurement results of brain activities of the subject. The therapy information generator 3200 transmits the classification results and corresponding therapy selection support information to the computer 400 via a communication interface in accordance with the classification results.

The information presentation unit 4044 of the computer 400 presents the therapy selection support information and the classification results for the specific subject returned from the therapy information generator 3200 of the support information providing device 300a to a doctor through a display device such as a display (not shown).

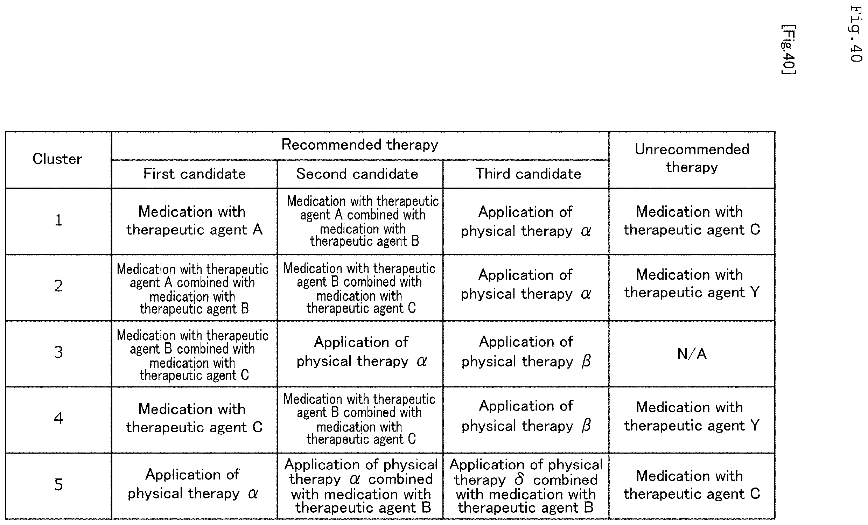

Fig. 40 shows an example of the therapy information database 5100.

The discrimination value calculator 3012 can classify the subject into Clusters 1 to 5, for example, using the clustering classifier. The therapy information database 5100 stores predetermined therapy information associated with therapies in accordance with the clusters.

The therapy information may store information related to a past therapy history of the subjects classified into the clusters (in particular, subjects that belong to a patient cohort whose data are used to generate the clustering classifier) and therapy information (reference therapy information) to be referenced on the basis of effects and/or side effects reported in references and the like. Preferably, the therapy information may store information that indicates the response of the subjects in the clusters to a specific therapeutic agent, information that indicates the response thereof to a specific physical therapy, etc.

In Fig. 40, recommended therapies based on the information related to the therapy history are stored for the clusters, from a first candidate to a third candidate in the descending order of effectiveness. Unrecommended therapies that may cause side effects or the like are also stored for the clusters as the reference therapy information.

Therapeutic agents for depression include, but are not particularly limited to: tricyclic antidepressants such as amitriptyline hydrochloride, amoxapine, and imipramine hydrochloride; tetracyclic antidepressants such as setiptiline maleate, maprotiline hydrochloride, and mianserin hydrochloride; selective serotonin reuptake inhibitors such as escitalopram oxalate, sertraline hydrochloride, paroxetine hydrochloride hydrate, and fluvoxamine maleate; serotonin-noradrenaline reuptake inhibitors such as duloxetine hydrochloride, venlafaxine hydrochloride, and milnacipran hydrochloride; and noradrenergic and specific serotonergic antidepressants such as mirtazapine.

Physical therapies for depression include transcranial magnetic stimulation, neurofeedback, electroconvulsive therapy, cognitive behavioral therapy, etc.

(Therapy Selection Support Process)

Next, a therapy selection support process performed by the therapy information generator 3200 of the support information providing device 300a will be described. The therapy selection support process is performed by a computer executing a therapy selection support program as a process of the therapy information generator 3200.

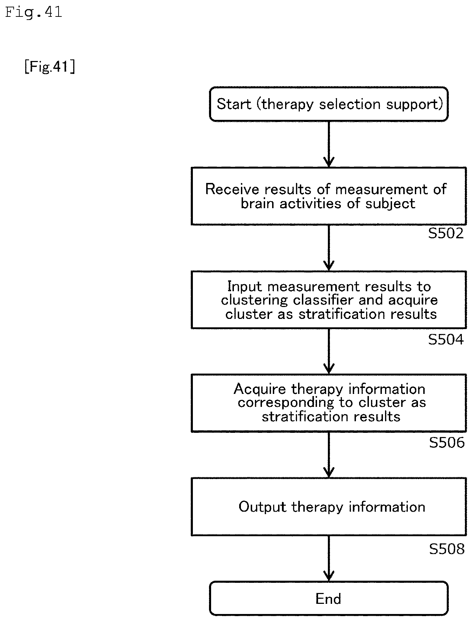

Fig. 41 is a flowchart illustrating the flow of a process of supporting the selection of a therapy for a subject.

In step S502 shown in Fig. 41, the support information providing device 300a receives the results of measurement of brain activities of the subject from the computer 400. The results of measurement of brain activities of the subject include image data (brain structure image data and brain function image data) anonymized by the computer 400.

In step S504 shown in Fig. 41, the support information providing device 300a executes the processes of calculating functional connectivity correlation matrix data and executes a process of correcting correlation values through a harmonization process for the results of measurement of brain activities of the subject, and thereafter inputs the measurement results to the clustering classifier generated by the classifier generation device 300b and acquires a belonging cluster probability which is the results of stratification of the subject. The belonging cluster probability is output as a probability at which the subject belongs to each cluster for all the clusters into which the clustering classifier can perform stratification. The support information providing device 300a can determine a cluster with the highest probability as a cluster to which the subject belongs. Alternatively, the support information providing device 300a may determine two clusters with the highest probabilities as clusters to which the subject can possibly belong. At this time, the support information providing device 300a functions as a clustering processor.

In step S506 shown in Fig. 41, the support information providing device 300a acquires therapy information corresponding to the cluster of the subject from the therapy information database 5100 on the basis of the cluster as a result of stratification of the subject acquired in step S504. At this time, the support information providing device 300a may acquire at least two pieces of therapy information, that is, a first candidate and a second candidate, as the therapy information corresponding to the cluster of the subject. This makes it possible to increase the option of therapies.

In step S508 shown in Fig. 41, the support information providing device 300a outputs the therapy information acquired in step S506 to the computer 400 via a communication interface.

With the configuration described above, the support information providing device 300a can provide a doctor with therapy selection support information for a subject on the basis of measurement data on brain activities of the subject.

[Screening Support System and Screening Support Device]

An aspect in which the support information providing device 300a described with reference to Fig. 39 is used as a support device that executes screening of a subject in drug discovery will be described below.

Herein, the term "therapy" means that a doctor "prescribes a specific medical agent and administers a specific dose of the medical agent to a subject to be taken in accordance with specific usage", or that a doctor "selects a specific therapeutic process and applies the therapy", and the term "therapy candidate" means a "candidate for a therapy" that has not been approved or certified by the competent authorities on the basis of a specific clinical test and the like. If the disease as the object is a mental disease, the term "therapeutic process" means cognitive behavioral therapy, for example.

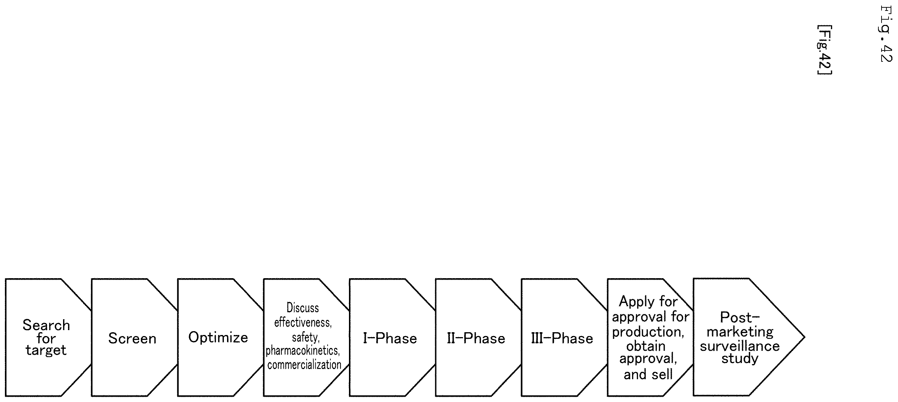

Fig. 42 shows a common drug discovery process.

In general, as shown in Fig. 42, the process includes: searching for a target in accordance with the purpose of drug discovery; screening candidate substances; optimizing such substances; and discussing effectiveness, safety, and pharmacokinetics through animal experiments, in vitro experiments (non-clinical tests) in which drug discovery candidate substances are administered to cells cultured in test tubes to measure reaction, etc. and discussing commercialization.

Candidate substances that have passed the non-clinical tests through the course described above are subjected to a so-called "clinical trial".

The "clinical trial" refers to a clinical test performed in order to obtain approval in accordance with the Pharmaceutical Affairs Law for production and sales of medical products. For medical products, the clinical trial is performed in three stages, namely I-phase to III-phase, in many cases. A I-phase test is directed to healthy adults that volunteer on the basis of their free will. A II-phase test is conducted for a small number of patients with relatively minor symptoms, in consideration of the result of the I-phase test, to discuss effectiveness, safety, pharmacokinetics, etc. A III-phase test is conducted on a greater scale for patients expected to actually use the compound after being placed on the market mainly for the purpose of verifying effectiveness and discussing safety.

The problems with drug discovery for the psychoneurotic system are that it is difficult at present to estimate the effectiveness of therapeutic agent candidate substances on humans using animal models, and that the probability that a therapeutic agent candidate substance will be finally placed on the market after being subjected to the I-phase test is low compared to therapeutic agent candidate substances for other diseases.

Thus, one solution to such problems is to identify an appropriate group of subjects so that candidate compounds that can possibly exhibit effectiveness can efficiently proceed to the III phase after the I-phase test (or the II-phase test), for example.

A case where the support information providing device 300a is used as a device that supports identification of such a group of subjects (screening of subjects) will be described below.

That is, information related to the clusters obtained through classification is returned as screening support data from the support information providing device 300a to the computer 400.

As also shown in FIG. 40, therapies for diseases of the psychoneurotic system are known to include physical therapies such as rTMS, for example, rather than medication. Medical devices and medical device programs that are used for such physical therapies also take the procedure of being subjected to a clinical trial to be approved as medical devices by the competent authorities, as with drug discovery candidate substances. It is assumed that screening of subjects to be discussed later is effective also for this clinical trial.

Thus, the screening support process described in relation to the present embodiment is not only applicable to clinical tests for "therapy candidates" discussed above, but also applicable to clinical tests for "therapeutic means candidates". Herein, the term "therapeutic means" means a "device that is used (by a doctor) to perform a specific therapy" and a "program (or a medium that stores the program or a device in which the program is installed) that is used to perform a specific therapy", and the term "therapeutic means candidate" means a "therapeutic device" or a "program (or a medium that stores the program or a device in which the program is installed)" before being approved or certified by the competent authorities on the basis of a specific clinical test and the like. If the disease as the object is a mental disease, for example, the term "therapeutic means (or "therapeutic device") means a TMS device that is used to perform transcranial magnetic stimulation, which is used in accordance with predetermined usage, a pulse wave therapy device that is used for electroconvulsive therapy, an application program for a smartphone that supports cognitive behavioral therapy, a storage medium that stores the application program, a smartphone in which the application program is installed, etc.

(Operation as Screening Support System)

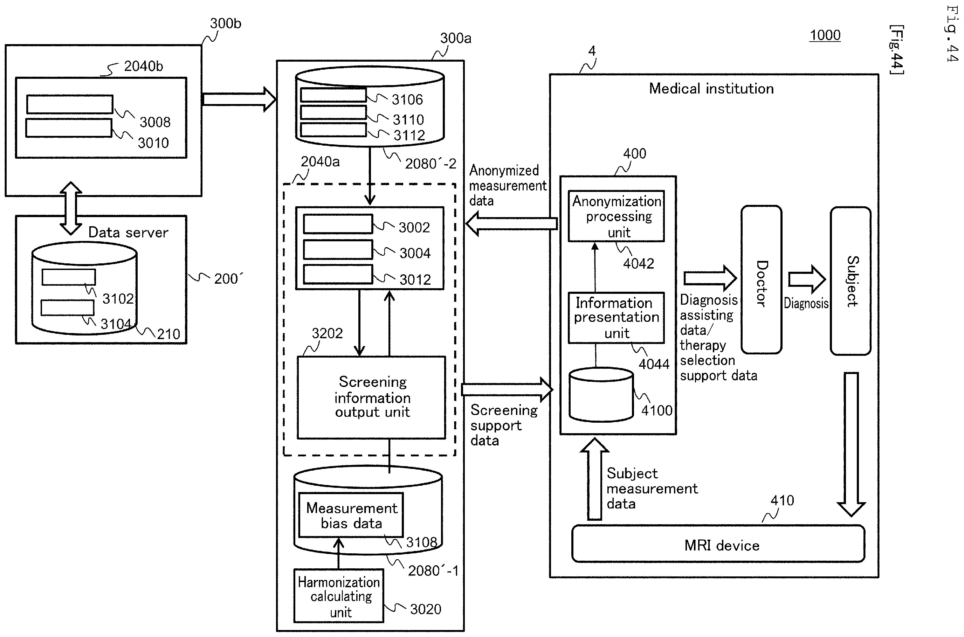

Fig. 44 illustrates the configuration of a screening support device 1000'.

As with the therapy selection support system 1000, the screening support system 1000' includes a computer 400 provided in a medical institution 4 and connected so as to be able to receive data from an MRI device 410 that measures brain activity data of a subject whose diagnosis label is to be estimated, a support information providing device 300a, a classifier generation device 300b that performs a process of generating a clustering classifier, and a data server 200'.

Components of the screening support device 1000' that are the same as the components of the therapy selection support system 1000 shown in Fig. 39 are given the same reference numerals, and description thereof will not be repeated here. Main operation of the screening support device 1000' will be described below.

That is, the MRI device 410 measures brain activity data of a subject to be subjected to stratification by the clustering classifier. The computer 400 stores the measured MRI measurement data in a non-volatile storage device 4100.

The computer 400 transmits the measurement data on the subject anonymized by the anonymization processing unit 4042 to the support information providing device 300a as the brain activity measurement result in accordance with an instruction for transmission from a user (e.g. a doctor). At this time, the measurement data to be transmitted are provided with a temporary ID for specifying the subject. Preferably, a table of correspondence between the temporary ID and personal information such as a patient name, for example, can be managed so as to be inaccessible from the computer 400. The content of the table of correspondence is configured to be not accessible at all at least in the support information providing device 300a.

Accordingly, a harmonization process corresponding to the site at which the MRI device 410 is installed is preferably executed, as necessary, in the support information providing device 300a. Further, the discrimination value calculator 3012 outputs the classification results, which is the results of stratification by the clustering classifier, for the input measurement results of brain activities of the subject. The classification results are stored in a storage device 2080'-1, for example, in association with a temporary ID that specifies the subject. A screening information output unit 3202 generates information for supporting screening based on the classification results in accordance with the classification results, and transmits the generated information to the computer 400 via a communication interface.

The information presentation unit 4044 of the computer 400 presents the information for supporting screening for the specific subject returned from the screening information output unit 3202 of the support information providing device 300a to a doctor through a display device such as a display (not shown).

Here, "information for supporting screening" is returned from the screening information output unit 3202 to the computer 400, because it is assumed that the results of stratification themselves are not returned to the medical institution as a so-called "double blind test" or "randomized controlled trial" is performed in the case where a clinical test or a clinical trial is performed using the results of screening, for example, and the information is displayed on the computer as information for supporting execution of screening in accordance with the characteristics of such tests.

When the test results are evaluated after the test is finished, the test results and the classification results for the subject can be collated with each other on the basis of the temporary ID.

(Screening Support Process by Support Information Providing Device 300a)

Next, a screening support process performed by the processor 2040a of the support information providing device 300a will be described. The screening support process is performed by a computer executing a screening support program.

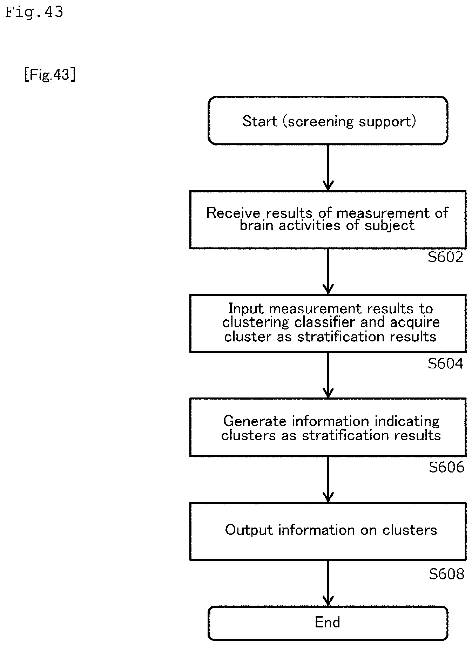

Fig. 43 is a flowchart illustrating the flow of a process of supporting screening of a subject in drug discovery.

In step S602 shown in Fig. 43, the support information providing device 300a receives the results of measurement of brain activities of the subject from the computer 400. The results of measurement of brain activities of the subject include image data (brain structure image data and brain function image data) anonymized by the computer 400.

In step S604 shown in Fig. 43, the support information providing device 300a executes the processes of calculating functional connectivity correlation matrix data and executes a process of correcting correlation values through a harmonization process for the results of measurement of brain activities of the subject, and thereafter inputs the measurement result to the clustering classifier generated by the classifier generation device 300b and acquires a belonging cluster probability which is the results of stratification of the subject. The belonging cluster probability is output as a probability at which the subject belongs to each cluster for all the clusters into which the clustering classifier can perform stratification. The support information providing device 300a can determine a cluster with the highest probability as a cluster to which the subject belongs.

In step S606 shown in Fig. 43, the support information providing device 300a generates information that indicates clusters as the results of stratification of the subject acquired in step S604.

In step S608 illustrated in Fig. 43, the support information providing device 300a outputs the information on the clusters generated in step S606 or information for supporting screening to the computer 400 via a communication interface.

With the configuration described above, the support information providing device 300a can provide a doctor with information on the cluster to which the subject belongs in the process of a clinical trial on the basis of measurement data on brain activities of the subject.

The doctor can perform a clinical trial by screening subjects in the corresponding cluster in accordance with a clinical trial protocol determined in advance.

[Verification Data on Stratification of Depression Patients]

The therapy selection support system 1000 has been described above with reference to Fig. 39, and the screening support device 1000' has been described above with reference to Figs. 42 to 44.

An example of data for verifying the clinical significance of the results of stratification calculated by the clustering classifier for the results of measurement of brain activities of the subject input to the discrimination value calculator 3012 in the embodiments described above will be described below.

(Clustering Classifier Used for Verification Data)

An MDD identifier generated by the disease identifier generator 3008 in generating a clustering classifier that is used for the verification data is configured as follows.

First, brain regions are divided in accordance with a BSA (Brainvisa Sulci Atlas) method as a method of parcellating brain areas.

The BSA method is disclosed in the following reference, for example.

Reference: Matthieu Perro, Denis Riviere, and Jean-Francois Mangin a, Cortical sulci recognition and spatial normalization. Medical Image Analysis Volume 15, Issue 4, August 2011, Pages 529-550

Next, a Pearson's correlation coefficient of an fMRI signal is calculated as an index of connection among brain regions, and an MDD identifier is trained and generated in accordance with discovery cohort data using the random forest method on the basis of brain functional connectivity links for the entire brain. In this case, correction among facilities based on the traveling subject method is not particularly applied.

A process in which the clustering classifier generator 3010 generates a clustering classifier is as follows.

First, in the course of training an MDD identifier, nested cross validation is performed, as a result of which 100 MDD identifiers are generated.

The degree of importance in identification of brain functional connectivity links is determined by the random forest method for each of the identifiers, and the total sum for the 100 identifiers is calculated to obtain the total degree of importance.

The brain functional connectivity links are ranked on the basis of the total degree of importance, and multiple co-clustering is executed using high-ranked connectivity links. The number of high-ranked connectivity links to be used can be determined by performing clustering for a plurality of numbers of connectivity links in predetermined patterns and adopting the number with the highest stability among datasets, although this is not particularly limiting.

The ARI value described with reference to Fig. 33 can be used as an evaluation index for evaluation of the "stability among datasets".

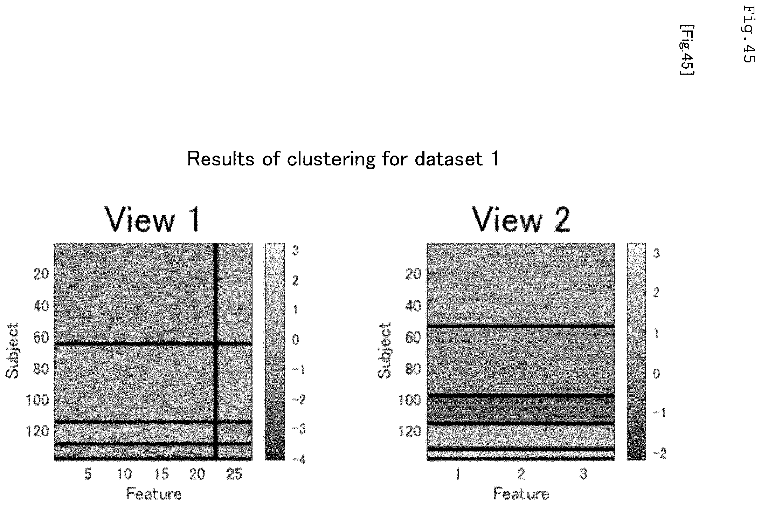

Fig. 45 shows views of the results of clustering through multiple co-clustering in which 30 brain functional connectivity links with the highest degree of importance in the MDD identifiers are used for Dataset 1.

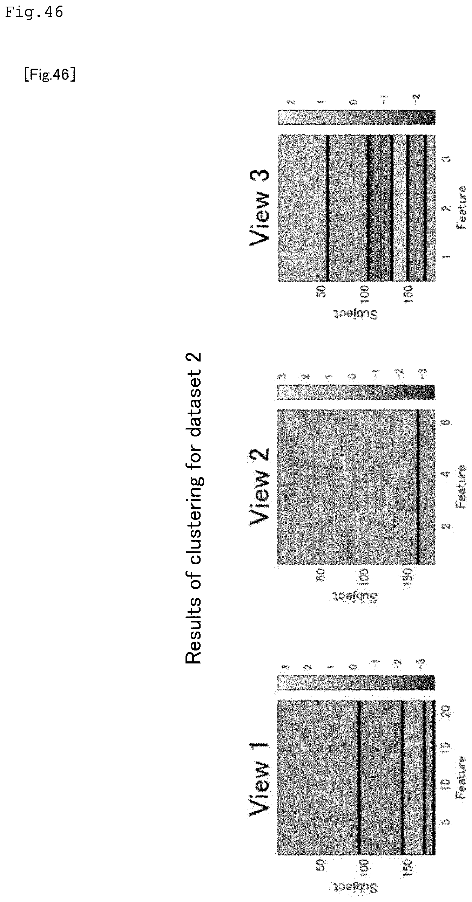

Fig. 46 shows views of the results of clustering through multiple co-clustering for Dataset 2.

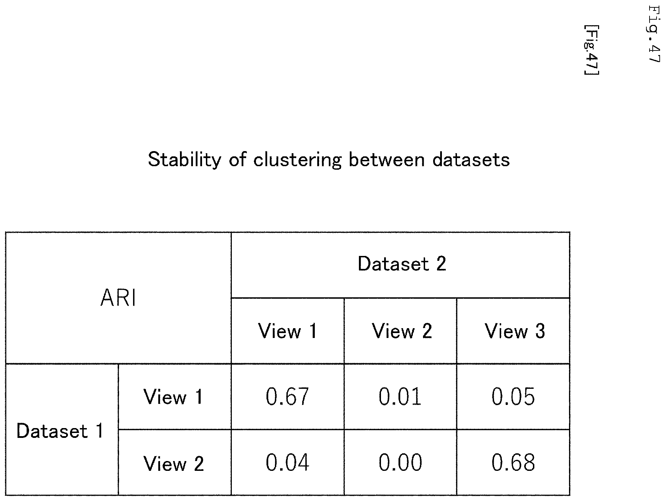

Fig. 47 shows the stability of clustering between Dataset 1 and Dataset 2.

Fig. 47 is a table in which ARI for each view of Dataset 1 and each view of Dataset 2 is calculated, corresponding to the table shown in Fig. 33.

With reference to Fig. 47, View 1 of Dataset 1 and View 1 of Dataset 2 and View 2 of Dataset 1 and View 3 of Dataset 2 have ARI values of 0.67 and 0.68, respectively, and are considered to be similar to each other.

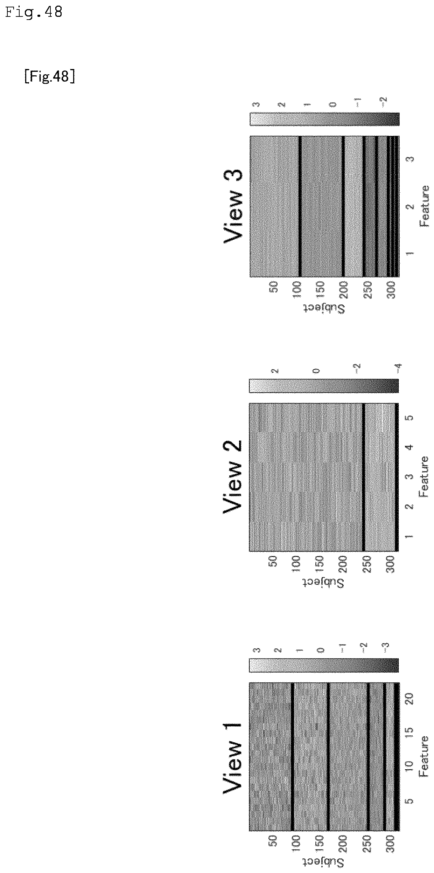

Fig. 48 shows views of the results of clustering for data of all depression patients (Datasets 1 + 2).

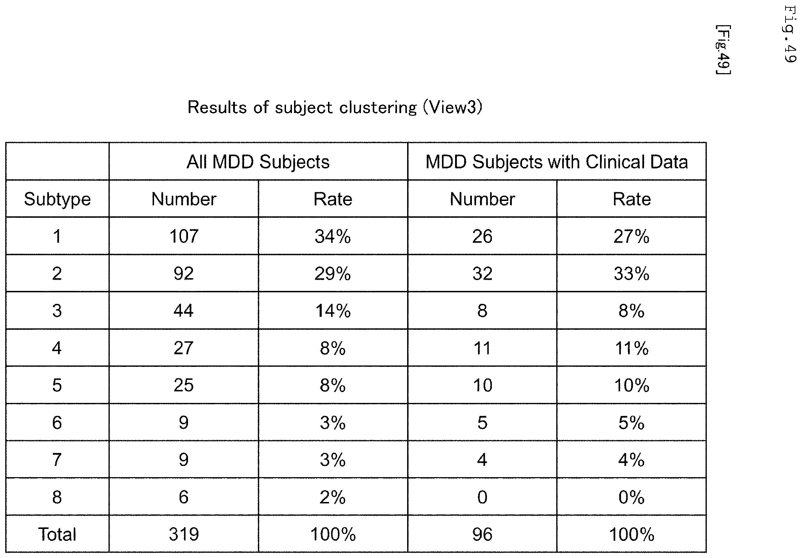

Fig. 49 indicates the number of all depression patients and the number of depression patients with clinical data for each subtype for View 3 generated by clustering of Datasets 1 + 2.

The term "clinical data" refers to "information on medication profile" of each patient, "information on diagnosis of the degree of depression" of each patient, etc.

With reference to Fig. 49, data on patients with clinical data occupy a part of the entire data (a part of both Dataset 1 and Dataset 2), and the distribution of the subtypes for the part of the data is not significantly different from the distribution thereof for the entire data. Thus, it is found that there is no problem if the clinical significance of clustering is discussed for only the "data on patients with clinical data".

View 3 indicated in Figs. 48 and 49 will be further described below as a view found with characteristic clinical significance, specifically with a difference in responsiveness to therapy among the subtypes.

It is also found that the features (average of connectivity links and standard deviation) of the brain functional connectivity links of the subtypes for some of the patients with clinical data are not significantly different from the average and the standard deviation for all the patients.

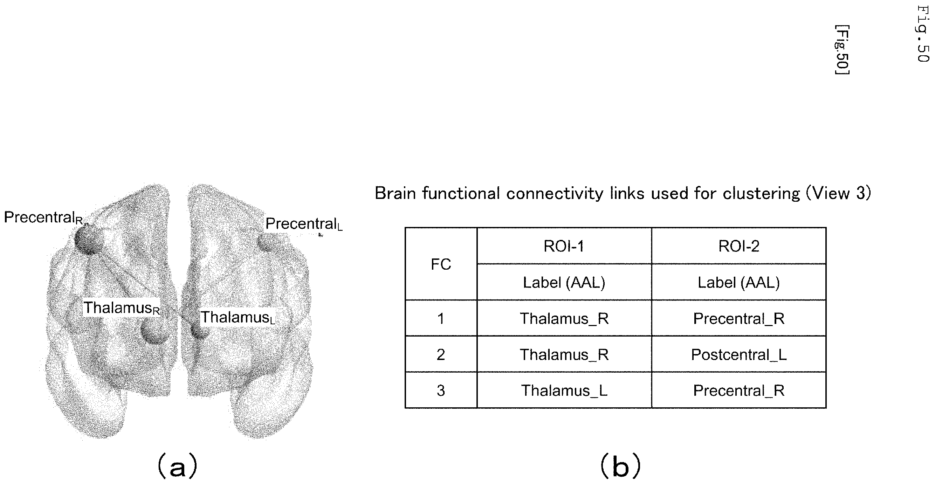

Fig. 50 illustrates brain functional connectivity links (View 3) used in clustering.

Fig. 50(a) indicates the positions in the brain of the brain functional connectivity links used in clustering. Fig. 50(b) indicates the regions of interest of the functional connectivity links.

In View 3, only the three connectivity links indicated in Figs. 50(a) and 50(b) are used in clustering.

Thirty connectivity links with the highest degree of importance in the MDD identifiers that are used to generate a clustering classifier are selected, and such connectivity links are used to perform multiple co-clustering.

That is, clustering is performed without assigning a weight and the like to the 30 selected connectivity links. As a result, three views are generated. In other words, the 30 connectivity links are divided into three groups (View 1: 22 connectivity links, View 2: 5 connectivity links, View 3: 3 connectivity links), and the subjects are clustered on the basis of the groups of the connectivity links.

In multiple co-clustering, an algorithm automatically derives an optimal solution to how the connectivity links are divided and how the patients (subjects) are clustered.

The names of the regions of interest in Fig. 50(b) have the following meanings:

Thalamus_L or R: left or right thalamus
Precentral_R: right precentral gyrus
Postcentral_L: left postcentral gyrus

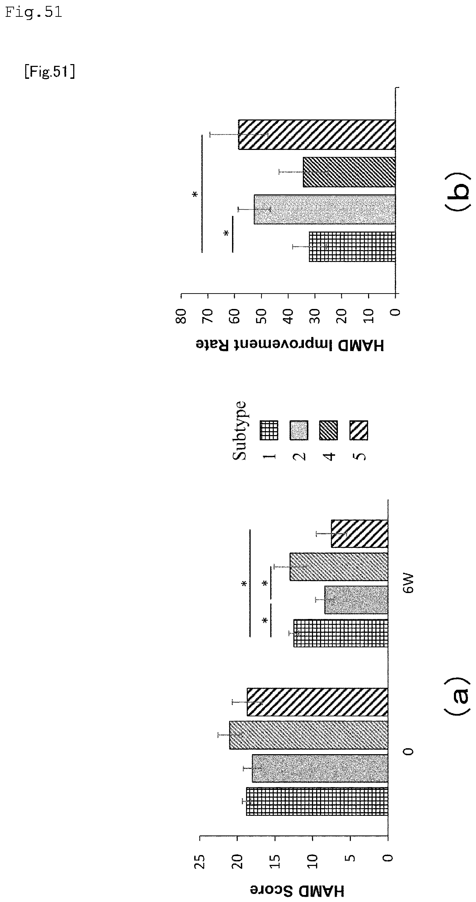

Fig. 51 indicates the relationship between the degree of severity of depression and the improvement rate in the degree of severity for each subtype in View 3 illustrated in Figs. 48 and 49.

Fig. 51(a) indicates the HAMD scores for the 0-th week and the sixth week after the start of therapy.

The HAMD refers to the Hamilton Depression Rating Scale, which is a score for rating the degree of severity of depression. A main 17-item version constituted of 17 items that indicate the degree of severity of depression and a 21-item version with the addition of four additional items are mainly used.

Subtypes 1 to 5 in Fig. 51(a) correspond to the top-five clusters in "Subject Clustering Results (View 3)" in Fig. 49.

The numbers of the subtypes are given in the descending order of the number of persons with clinical data. Subtypes with data on 10 subjects or more were analyzed. Therefore, Subtypes 1, 2, 4, and 5, among Subtypes 1 to 5, are indicated in the graphs.

The 0-th week after the start of therapy means the time of the entry of study and the start of therapy with an SSRI. The sixth week after the start of therapy means the time six weeks after the entry of study and the start of therapy. That is, persons that were treated with an SSRI since before the entry of study are excluded.

The SSRI refers to a "selective serotonin reuptake inhibitor", and includes escitalopram, for example.

The treatment protocol in the study is as follows.

Study is entered for patients of major depressive disorder that have not been treated or that have only been treated with an insufficient amount of medication or for an insufficient period. When study is entered, treatment with an SSRI such as escitalopram is started. The SSRI can be used in an increased amount through a clinical decision. There is no limitation on combined medication. If not improved with the SSRI, therapy with a different medical agent is performed.

Fig. 51(b) indicates the HAMD improvement rate, making a comparison between the 0-th week and the sixth week.

The improvement rate is represented by the following formula:

$$(\mathrm{HAMD}(0) - \mathrm{HAMD}(6W)) / \mathrm{HAMD}(0) \times 100$$

It is seen from Fig. 51(a) that there is no difference in the HAMD, that is, the degree of severity of depression is about the same, among the subtypes in the initial stage.

It is seen from Fig. 51(a) that a difference in the HAMD is observed among the subtypes six weeks after the start of therapy with an SSRI. Fig. 51(b) indicates that, as is seen from the improvement rate from the initial value, the effect of the therapy with the SSRI was low for Subtype 1 compared to Subtypes 2 and 5 and, conversely, the effect of the therapy with the SSRI was high for Subtypes 2 and 5 compared to Subtype 1.

From the above description, it is seen that use of the clustering classifier described in relation to the embodiments makes it possible to estimate the response of patients in the initial stage of therapy to therapy with an agent SSRI for each cluster.

The embodiments as have been described here are mere examples and should not be interpreted as restrictive. The scope of the present invention is determined by each of the claims with appropriate consideration of the written description of the embodiments and embraces modifications within the meaning of, and equivalent to, the languages in the claims.

Reference Signs List

2 subject, 6 display, 10 MRI device, 11 magnetic field applying mechanism, 12 static magnetic field generating coil, 14 magnetic field gradient generating coil, 16 RF irradiating unit, 18 bed, 20 receiving coil, 21 driving unit, 22 static magnetic field power source, 24 magnetic field gradient power source, 26 signal transmitting unit, 28 signal receiving unit, 30 bed driving unit, 32 data processing unit, 36 storage unit, 38 display unit, 40 input unit, 42 control unit, 44 interface unit, 46 data collecting unit, 48 image processing unit, 50 network interface, 300a support information providing device, 300b classifier generation device, 500 therapy information providing server

**Claims**

1. A therapy selection support system that provides information related to selection of a therapy for a first subject with depression symptoms on the basis of results of measurement of brain activities of the first subject, comprising:

a clustering device that executes stratification in which results of measurement of brain functional connectivity correlation values acquired from a plurality of second subjects are divided into a plurality of clusters through a clustering process, wherein:

the plurality of second subjects include a first cohort having a diagnosis label of a depression and a second cohort not having the diagnosis label of the depression;
the clustering device includes a processor and a storage device, the processor being configured to execute the clustering process for the plurality of second subjects;
the processor is configured to, in a process of generating a clustering classifier,

i) store, in the storage device, features based on a plurality of brain functional connectivity correlation values that represent time correlation of brain activities among a plurality of predetermined brain area pairs for each of the plurality of second subjects,
ii) execute machine learning, through supervised learning, to generate an identifier model for discriminating presence or absence of the diagnosis label on the basis of the features stored in the storage device,
iii) select features for clustering in accordance with a degree of importance of features that are used to generate an identifier through the machine learning in the machine learning to generate the identifier model, and
iv) generate a clustering classifier by clustering the first cohort through a multiple co-clustering method as unsupervised learning on the basis of the selected features for the clustering; and

the therapy selection support system further includes

a database device that stores the clusters obtained as a result of the stratification by the clustering classifier and corresponding predetermined therapy information in association with each other, and
a support information providing device that receives an input of the results of the measurement of the brain activities of the first subject and that outputs corresponding therapy information in accordance with results of classification by the clustering classifier for the measurement results.

2. The therapy selection support system according to claim 1, wherein
the processor is configured to, in the machine learning to generate the identifier model,

generate a plurality of training sub-samples by executing under-sampling and subsampling from the first cohort and the second cohort,
select features for clustering from a sum-set of features that are used to generate the identifier through the machine learning in accordance with a degree of importance of features that belong to the sum-set for each of the training sub-samples, and
generate the clustering classifier by the multiple co-clustering method on the basis of the selected features for the clustering.

3. The therapy selection support system according to claim 1 or 2, wherein:

the support information providing device includes a clustering processor and an interface device;
the clustering processor calculates a probability at which the first subject is classified as belonging to each of the clusters by the clustering classifier, and reads at least two pieces of the therapy information selected in accordance with the probability from the database device; and
the interface device outputs data for displaying the selected clusters and the corresponding pieces of the therapy information in association with each other.

4. The therapy selection support system according to any one of claims 1 to 3, wherein
the therapy information is information that indicates a response to a specific therapeutic agent.

5. The therapy selection support system according to any one of claims 1 to 3, wherein
the therapy information is information that indicates a response to a specific physical therapy.

6. The therapy selection support system according to claim 2, wherein
a process of generating the identifier through the machine learning involves ensemble learning in which a plurality of identifier sub-models are generated for the plurality of training sub-samples and the plurality of identifier sub-models are integrated with each other to generate the identifier model.

7. The therapy selection support system according to claim 1 or 2, wherein:

the clustering device receives, from a plurality of brain activity measurement devices provided at a plurality of measurement sites, information that represents time correlation of brain activities among a plurality of predetermined brain area pairs for each of the plurality of second subjects; and
the processor includes harmonization calculation means for correcting the plurality of brain functional connectivity correlation values for each of the plurality of second subjects so as to remove a measurement bias of the measurement sites and storing corrected adjustment values in the storage device as the features.

8. A therapy selection support device that provides information related to selection of a therapy for a first subject with depression symptoms on the basis of results of measurement of brain activities of the first subject, comprising:

a database device that stores clusters obtained as a result of stratification of subjects having a diagnosis label of a depression, among a plurality of second subjects, and corresponding predetermined therapy information in association with each other; and
a support information providing device that receives an input of the results of measuring the brain activities of the first subject and that outputs corresponding therapy information in accordance with the result of the stratification based on the measurement results, wherein:

the plurality of second subjects include a first cohort having a diagnosis label of a depression and a second cohort not having the diagnosis label of the depression;
the clusters obtained as a result of the stratification are obtained by a clustering classifier obtained through a clustering process for results of measurement of brain functional connectivity correlation values by a clustering device;
the clustering device includes a processor that executes the clustering process for the first cohort and a storage device; and
the processor is configured to, in a process of generating the clustering classifier,

i) store, in the storage device, features based on a plurality of brain functional connectivity correlation values that represent time correlation of brain activities among a plurality of predetermined brain area pairs for each of the plurality of second subjects,
ii) execute machine learning, through supervised learning, to generate an identifier model for discriminating presence or absence of the diagnosis label on the basis of the features stored in the storage device,
iii) select features for clustering in accordance with a degree of importance of features that are used to generate an identifier through the machine learning in the machine learning to generate the identifier model, and
iv) generate the clustering classifier by clustering the first cohort through a multiple co-clustering method as unsupervised learning on the basis of the selected features for the clustering.

9. The therapy selection support device according to claim 8, wherein:

the support information providing device includes a clustering processor and an interface device;
the clustering processor calculates a probability at which the first subject is classified as belonging to each of the clusters by the clustering classifier, and reads at least two pieces of the therapy information selected in accordance with the probability from the database device; and
the interface device outputs data for displaying the selected clusters and the corresponding pieces of the therapy information in association with each other.

10. The therapy selection support device according to claim 8 or 9, wherein
the therapy information is information that indicates a response to a specific therapeutic agent.

11. The therapy selection support device according to any one of claims 8 to 10, wherein
the therapy information is information that indicates a response to a specific physical therapy.

12. A therapy selection support method that provides information related to selection of a therapy for a first subject with

**EP 4 183 333 A1**

depression symptoms on the basis of results of measurement of brain activities of the first subject, comprising:
a preparing step of generating a clustering classifier that executes stratification in which results of measurement of brain functional connectivity correlation values acquired from a plurality of second subjects are divided into a plurality of clusters through a clustering process, wherein:

the plurality of second subjects include a first cohort having a diagnosis label of a depression and a second cohort not having the diagnosis label of the depression;
the preparing step includes a processing step of executing the clustering process for the plurality of second subjects;
the processing step includes

i) a step of acquiring features based on a plurality of brain functional connectivity correlation values that represent time correlation of brain activities among a plurality of predetermined brain area pairs for each of the plurality of second subjects,
ii) a step of executing machine learning, through supervised learning, to generate an identifier model for discriminating presence or absence of the diagnosis label on the basis of the acquired features,
iii) a step of selecting features for clustering in accordance with a degree of importance of features that are used to generate an identifier through the machine learning in the machine learning to generate the identifier model, and
iv) a step of generating the clustering classifier by clustering the first cohort through a multiple co-clustering method as unsupervised learning on the basis of the selected features for the clustering; and

the therapy selection support method further includes a support information providing step of acquiring, from a database that stores the clusters obtained as a result of the stratification by the clustering classifier and corresponding predetermined therapy information in association with each other, and outputting corresponding therapy information in accordance with results of classification by the clustering classifier for the results of the measurement of the brain activities of the first subject.

13. A therapy selection support method that provides information related to selection of a therapy for a first subject with depression symptoms on the basis of results of measurement of brain activities of the first subject, comprising:
a support information providing step of acquiring, from a database that stores results of stratification of subjects having a diagnosis label of a depression, among a plurality of second subjects, and corresponding predetermined therapy information in association with each other, and outputting corresponding therapy information in accordance with clusters obtained as a result of stratification based on the results of the measurement of the brain activities of the first subject, wherein:

the plurality of second subjects include a first cohort having a diagnosis label of a depression and a second cohort not having the diagnosis label of the depression;
the clusters obtained as a result of the stratification are obtained by a clustering classifier obtained through a clustering process for results of measurement of brain functional connectivity correlation values;
the clustering classifier is generated through a processing step of executing the clustering process for the plurality of second subjects; and
the processing step includes

i) a step of acquiring features based on a plurality of brain functional connectivity correlation values that represent time correlation of brain activities among a plurality of predetermined brain area pairs for each of the plurality of second subjects,
ii) a step of executing machine learning, through supervised learning, to generate an identifier model for discriminating presence or absence of the diagnosis label on the basis of the acquired features,
iii) a step of selecting features for clustering in accordance with a degree of importance of features that are used to generate an identifier through the machine learning in the machine learning to generate the identifier model, and
iv) a step of generating the clustering classifier by clustering the first cohort through a multiple co-clustering method as unsupervised learning on the basis of the selected features for the clustering.

14. A therapy selection support program that provides information related to selection of a therapy for a first subject with depression symptoms on the basis of results of measurement of brain activities of the first subject,
the therapy selection support program causing a computer, when executed by the computer, to execute:

55

a step of generating a clustering classifier that executes stratification in which results of measurement of brain functional connectivity correlation values acquired from a plurality of second subjects are divided into a plurality of clusters through a clustering process; and

a step of receiving an input of the results of the measurement of the brain activities of the first subject and acquiring, from a database device that stores the clusters obtained as a result of the stratification by the clustering classifier and corresponding predetermined therapy information in association with each other, and outputting corresponding therapy information in accordance with results of classification by the clustering classifier for the measurement results, wherein:

the plurality of second subjects include a first cohort having a diagnosis label of a depression and a second cohort not having the diagnosis label of the depression;

the clustering process includes a processing step of executing the clustering process for the plurality of second subjects; and

the processing step includes

i) a step of storing, in a storage device, features based on a plurality of brain functional connectivity correlation values that represent time correlation of brain activities among a plurality of predetermined brain area pairs for each of the plurality of second subjects,

ii) a step of executing machine learning, through supervised learning, to generate an identifier model for discriminating presence or absence of the diagnosis label on the basis of the features stored in the storage device,

iii) a step of selecting features for clustering in accordance with a degree of importance of features that are used to generate an identifier through the machine learning in the machine learning to generate the identifier model, and

iv) a step of generating the clustering classifier by clustering the first cohort through a multiple co-clustering method as unsupervised learning on the basis of the selected features for the clustering.

15. A therapy selection support program that provides information related to selection of a therapy for a first subject with depression symptoms on the basis of results of measurement of brain activities of the first subject,

the therapy selection support program causing a computer, when executed by the computer, to execute:

a support information providing step of acquiring, from a database that stores results of stratification of subjects having a diagnosis label of a depression, among a plurality of second subjects, and corresponding predetermined therapy information in association with each other, and outputting corresponding therapy information in accordance with clusters obtained as a result of stratification based on the results of the measurement of the brain activities of the first subject, wherein:

the clusters obtained as a result of the stratification are obtained by a clustering classifier obtained through a clustering process for results of measurement of brain functional connectivity correlation values;

the plurality of second subjects include a first cohort having a diagnosis label of a depression and a second cohort not having the diagnosis label of the depression;

the clustering classifier is generated through a processing step of executing the clustering process for the plurality of second subjects; and

the processing step includes

i) a step of acquiring features based on a plurality of brain functional connectivity correlation values that represent time correlation of brain activities among a plurality of predetermined brain area pairs for each of the plurality of second subjects,

ii) a step of executing machine learning, through supervised learning, to generate an identifier model for discriminating presence or absence of the diagnosis label on the basis of the acquired features,

iii) a step of selecting features for clustering in accordance with a degree of importance of features that are used to generate an identifier through the machine learning in the machine learning to generate the identifier model, and

iv) a step of generating the clustering classifier by clustering the first cohort through a multiple co-clustering method as unsupervised learning on the basis of the selected features for the clustering.

16. A screening support system that supports screening of a first subject on the basis of results of measurement of brain activities of the first subject in a clinical test for a therapeutic means candidate for depression symptoms, comprising:

a clustering device that executes stratification in which results of measurement of brain functional connectivity correlation values acquired from a plurality of second subjects are divided into a plurality of clusters through a clustering process, wherein:

the plurality of second subjects include a first cohort having a diagnosis label of a depression and a second cohort not having the diagnosis label of the depression;
the clustering device includes a processor and a storage device, the processor being configured to execute the clustering process for the plurality of second subjects;
the processing device is configured to

i) store, in the storage device, features based on a plurality of brain functional connectivity correlation values that represent time correlation of brain activities among a plurality of predetermined brain area pairs for each of the plurality of second subjects,
ii) execute machine learning, through supervised learning, to generate an identifier model for discriminating presence or absence of the diagnosis label on the basis of the features stored in the storage device,
iii) select features for clustering in accordance with a degree of importance of features that are used to generate an identifier through the machine learning in the machine learning to generate the identifier model, and
iv) generate a clustering classifier by clustering the first cohort through a multiple co-clustering method as unsupervised learning on the basis of the selected features for the clustering; and

the screening support system further includes a support information providing device that receives an input of the results of the measurement of the brain activities of the first subject, that records results of classification by the clustering classifier for the measurement results in association with the first subject, and that outputs information for supporting the screening of the first subject based on the classification results.

17. The screening support system according to claim 16, wherein
the processor is configured to, in the machine learning to generate the identifier model,

generate a plurality of training sub-samples by executing under-sampling and subsampling from the first cohort and the second cohort,
select features for clustering from a sum-set of features that are used to generate the identifier through the machine learning in accordance with a degree of importance of features that belong to the sum-set for each of the training sub-samples, and
generate the clustering classifier by the multiple co-clustering method on the basis of the selected features for the clustering.

18. A screening support device that supports screening of a first subject on the basis of results of measurement of brain activities of the first subject in a clinical test for a therapeutic means candidate for depression symptoms, comprising:
a support information providing device that includes a storage device that stores information for specifying a clustering classifier, that receives an input of the results of the measurement of the brain activities of the first subject, that records results of classification based on the clustering classifier for the measurement results in association with the first subject, and that outputs information for supporting the screening of the first subject based on the classification results, wherein:

the clusters obtained as a result of the stratification are obtained by the clustering classifier obtained through a clustering process for results of measurement of brain functional connectivity correlation values by a clustering device;
the clustering device includes a processor and a storage device, the processor being configured to execute the clustering process for a plurality of second subjects including a first cohort having a diagnosis label of a depression and a second cohort not having the diagnosis label of the depression; and
the processor is configured to, in a process of generating the clustering classifier,

i) store, in the storage device, features based on a plurality of brain functional connectivity correlation values that represent time correlation of brain activities among a plurality of predetermined brain area pairs for each of the plurality of second subjects,
ii) execute machine learning, through supervised learning, to generate an identifier model for discriminating presence or absence of the diagnosis label on the basis of the features stored in the storage device,

iii) select features for clustering in accordance with a degree of importance of features that are used to generate an identifier through the machine learning in the machine learning to generate the identifier model, and

iv) generate the clustering classifier by clustering the first cohort through a multiple co-clustering method as unsupervised learning on the basis of the selected features for the clustering.

19. A screening support method that supports screening of a first subject on the basis of results of measurement of brain activities of the first subject in a clinical test for a therapeutic means candidate for depression symptoms, comprising:

a step of a processor executing classification of the first subject in accordance with the results of the measurement of the brain activities on the basis of a clustering classifier specified by information stored in a storage device; and

a step of recording results of the classification in association with the first subject and outputting information for supporting the screening of the first subject based on the classification results, wherein:

a process of generating the clustering classifier includes a processing step of executing the clustering process for a plurality of second subjects including a first cohort having a diagnosis label of a depression and a second cohort not having the diagnosis label of the depression; and

the processing step includes

i) a step of acquiring features based on a plurality of brain functional connectivity correlation values that represent time correlation of brain activities among a plurality of predetermined brain area pairs for each of the plurality of second subjects,

ii) a step of executing machine learning, through supervised learning, to generate an identifier model for discriminating presence or absence of the diagnosis label on the basis of the acquired features,

iii) a step of selecting features for clustering in accordance with a degree of importance of features that are used to generate an identifier through the machine learning in the machine learning to generate the identifier model, and

iv) a step of generating the clustering classifier by clustering the first cohort through a multiple co-clustering method as unsupervised learning on the basis of the selected features for the clustering.

20. A screening support program that supports screening of a first subject on the basis of results of measurement of brain activities of the first subject in a clinical test for a therapeutic means candidate for depression symptoms, the screening support program causing a computer, when executed by the computer, to execute:

a step of a processor executing classification of the first subject in accordance with the results of the measurement of the brain activities on the basis of a clustering classifier specified by information stored in a storage device; and

a step of recording results of the classification in association with the first subject and outputting information for supporting the screening of the first subject based on the classification results, wherein:

a process of generating the clustering classifier includes a processing step of executing the clustering process for a plurality of second subjects including a first cohort having a diagnosis label of a depression and a second cohort not having the diagnosis label of the depression; and

the processing step includes

i) a step of storing, in a storage device, features based on a plurality of brain functional connectivity correlation values that represent time correlation of brain activities among a plurality of predetermined brain area pairs for each of the plurality of second subjects,

ii) a step of executing machine learning, through supervised learning, to generate an identifier model for discriminating presence or absence of the diagnosis label on the basis of the features stored in the storage device,

iii) a step of selecting features for clustering in accordance with a degree of importance of features that are used to generate an identifier through the machine learning in the machine learning to generate the identifier model, and

iv) a step of generating the clustering classifier by clustering the first cohort through a multiple co-clustering method as unsupervised learning on the basis of the selected features for the clustering.

21. The therapy selection support system according to claim 1, wherein
the predetermined therapy information is information related to a response to a therapy with a selective serotonin reuptake inhibitor.

22. The therapy selection support device according to claim 8, wherein
the predetermined therapy information is information related to a response to a therapy with a selective serotonin reuptake inhibitor.

23. The therapy selection support method according to claim 12 or 13, wherein
the predetermined therapy information is information related to a response to a therapy with a selective serotonin reuptake inhibitor.

24. The therapy selection support program according to claim 14 or 15, wherein
the predetermined therapy information is information related to a response to a therapy with a selective serotonin reuptake inhibitor.

25. The screening support system according to claim 16, wherein
the therapeutic means candidate is a therapy in which a selective serotonin reuptake inhibitor is used.

26. The screening support device according to claim 18, wherein
the therapeutic means candidate is a therapy in which a selective serotonin reuptake inhibitor is used.

27. The screening support method according to claim 19, wherein
the therapeutic means candidate is a therapy in which a selective serotonin reuptake inhibitor is used.

28. The screening support program according to claim 20, wherein
the therapeutic means candidate is a therapy in which a selective serotonin reuptake inhibitor is used.

Fig.1

[Fig.1]

Average waveform of each
region of interest is extracted

(a)

Correlation matrix among
Nc regions is calculated

(b)

[Fig.3]
Fig.3

EP 4 183 333 A1

| Site ID | | |
|---|---|---|
| Site name | | |
| Condition ID | | |
| Measurement device | Manufacturer name | |
| | Model number | |
| | Number of receiving coils | |
| | ... | |
| Measurement condition | Direction of phase encoding (P → A, A → P) | |
| | Image type | |
| | Image sequence | |
| | Whether eyes are open or closed | |
| | ... | |

(a)

| Subject's tentative ID | | |
|---|---|---|
| Condition ID | | |
| Sex | | |
| Age | | |
| Whether healthy or sick | | |
| Name of disease diagnosed | | |
| Medication profile | (Relative date) | Medical agent 1 |
| | (Relative date) | Medical agent 2 |
| | ... | ... |
| Diagnosis history | (Relative date) | Diagnostic matter |
| | (Relative date) | Diagnostic matter |
| | ... | ... |

(b)

[Fig.4]

Fig. 4

Fig.5

[Fig.5]

32

[Fig.6]

Fig. 6

EP 4 183 333 A1

Pre-processing → Percellation → Correlation matrixes or correlation vectors → Harmonization → Generation of identifier through ensemble learning → Disease identifier (diagnostic marker)

Select features for clustering → Clustering (stratification)

Fig.7

[Fig.7]

200 — 210 Measurement parameters / Patient cohort data / Healthy person cohort data / Traveling subject data

300

2040

Harmonization calculating unit — 3020

Correlation matrix calculating unit — 3002

Learning and discriminating unit — 3000

2080

Fig. 8

[Fig.8]

EP 4 183 333 A1

67

Fig. 8

- Measurement parameters
- Patient cohort data
- Healthy person cohort data
- Traveling subject data

200

210

2080

MRI measurement data
3102

Subject's human attribute information/ measurement parameters
3104

Correlation matrix data
3106

Measurement bias data
3108

Corrected correlation value data
3110

Disease identifier data
3112

Correlation matrix calculating unit
3002

Correlation value correcting unit
3004

Disease identifier generator
3008

Clustering classifier generator
3010

Discrimination value calculator
3012

3000

Harmonization calculating unit
3020

2040

Input data

Discrimination result

[Fig.9]
Fig.9

EP 4 183 333 A1

| Process of learning for 2-class identifier |
| --- |

S100

| Prepare training dataset for MDD identifier (n = 683) |
| --- |

S102

| Harmonization process |
| --- |

S104

| Divide data for 10-fold cross validation |
| --- |

S106

| Prepare training dataset for 10-fold cross validation | | Prepare test dataset for 10-fold cross validation |
| --- | --- | --- |

S108      S110

| Under-sampling process or sub-sampling process |
| --- |

S112

N

Are combinations of 10-fold cross validation finished?

S122

Y

| Process of adjusting hyper parameters for sub-sample 1 | ... | Process of adjusting hyper parameters for sub-sample 10 |
| --- | --- | --- |

S114.1      S114.10

| Generate identifier sub-model for MDD (average of outputs from identifiers generated by 10 sub-samples) |
| --- |

S116

| Validate sub-model i (i = 1, ..., 10) using test dataset |
| --- |

S118

| Generate identifier model for MDD (average of outputs from identifiers generated by 100 (= 10 × 10) sub-samples) |
| --- |

S120

Fig.10

[Fig.10]

Demographic characteristics of patients included in training dataset

| Site | Healthy persons (HC) | | | | Major depressive disorder patients (MDD) | | | | Overall | | | |
|---|---|---|---|---|---|---|---|---|---|---|---|---|
| | Number of persons | Male / female | Age | BDI | Number of persons | Male / female | Age | BDI | Number of persons | Male / female | Age | BDI |
| Site 1 (COI) | 124 (123) | 46/78 | 51.9± 13.4 | 8.2±6.3 | 70 (70) | 31/39 | 45.0± 12.5 | 26.2±9.9 | 194 (193) | 77/117 | 49.4± 13.5 | 14.7±11.7 |
| Site 2 (KUT) | 169 (139) | 100/69 | 35.9± 13.6 | 6.0±5.4 | 17 (17) | 11/6 | 43.9± 13.3 | 27.7±10. 1 | 186 (156) | 111/75 | 36.7± 13.7 | 8.3±9.1 |
| Site 3 (SWA) | 101 (97) | 86/15 | 28.4± 7.9 | 4.4±3.8 | 0 | - | - | - | 101 (97) | 86/15 | 28.4± 7.9 | 4.4±3.8 |
| Site 4 (UTO) | 170 (24) | 78/92 | 35.6± 17.5 | 6.7±6.5 | 62 (32) | 36/26 | 38.7± 11.6 | 20.4±11. 4 | 232 (56) | 114/118 | 36.4± 16.2 | 14.5±11.8 |
| Total | 564 (383) | 310/254 | 38.0± 16.1 | 6.3±5.6 | 149 (119) | 78/71 | 42.3± 12.5 | 24.9±10. 7 | 713 (502) | 388/325 | 38.9± 15.5 | 10.7±10.6 |

[Fig.11]

Fig. 11

| Demographic characteristics of patients in independent validation dataset | | | | | | | | | | | |
|---|---|---|---|---|---|---|---|---|---|---|---|
| Site | Healthy persons (HC) | | | | Major depressive disorder patients (MDD) | | | | Overall | | | |
|  | Number of persons | Male / female | Age | BDI | Number of persons | Male / female | Age | BDI | Number of persons | Male / female | Age | BDI |
| Site 5 (HKH) | 29 (29) | 12/17 | 45.4±9.5 | 5.1±4.6 | 33 (33) | 20/13 | 44.8±11.5 | 28.5±8.7 | 62 (62) | 32/30 | 45.1±10.5 | 17.6±13.7 |
| Site 6 (HRC) | 49 (49) | 13/36 | 41.7±11.7 | 9.1±8.5 | 16 (16) | 6/10 | 40.5±11.5 | 35.3±9.5 | 65 (65) | 19/46 | 41.4±11.5 | 15.6±14.3 |
| Site 7 (HUH) | 66 (66) | 29/37 | 34.6±13.0 | 6.9±5.9 | 57 (57) | 32/25 | 43.3±12.2 | 30.9±9.0 | 123 (123) | 61/62 | 38.6±13.3 | 18.0±14.1 |
| Site 8 (UYA) | 120 (120) | 50/70 | 45.9±19.5 | 7.1±5.6 | 79 (78) | 36/43 | 50.3±13.6 | 29.7±10.7 | 199 (198) | 86/113 | 47.6±17.5 | 16.0±13.6 |
| Total | 264 (264) | 104/160 | 42.2±16.5 | 7.2±6.3 | 185 (184) | 94/91 | 46.3±13.0 | 30.3±9.9 | 449 (448) | 198/251 | 43.9±15.3 | 16.7±13.9 |

[Fig.12]

Prediction performance of MDD for all imaging sites (training dataset)

Healthy ⟵ Probability of diagnostic index ⟶ MDD

[Fig.13]

Prediction performance of MDD for each imaging site (training dataset)

### Site 1 (COI)

AUC = 0.72235
Accuracy = 0.5977
Sensitivity = 0.83871
Specificity = 0.46429
MCC = 0.303

### Site 2 (KUT)

AUC = 0.81609
Accuracy = 0.71585
Sensitivity = 0.64706
Specificity = 0.72289
MCC = 0.2319

### Site 3 (SWA)

AUC = NaN
Accuracy = 0.69697
Sensitivity = NaN
Specificity = 0.69697
MCC = NaN

### Site 4 (UTO)

AUC = 0.74405
Accuracy = 0.66079
Sensitivity = 0.72881
Specificity = 0.6369
MCC = 0.32193

Fig.14

[Fig.14]

Fig. 15

Prediction performance of MDD for each imaging site (independent validation dataset) [Fig.15]

## Site 5 (NKH)

AUC = 0.59263
Accuracy = 0.58333
Sensitivity = 0.65625
Specificity = 0.5
MCC = 0.15811

## Site 6 (HRC)

AUC = 0.86862
Accuracy = 0.75385
Sensitivity = 0.75
Specificity = 0.7551
MCC = 0.45086

## Site 7 (HUH)

AUC = 0.69219
Accuracy = 0.64228
Sensitivity = 0.54386
Specificity = 0.72727
MCC = 0.27617

## Site 8 (UYA)

AUC = 0.82422
Accuracy = 0.74359
Sensitivity = 0.77333
Specificity = 0.725
MCC = 0.48596

Fig.16

[Fig.16]

**S200** Clustering learning process

**S202** Prepare subject data of healthy person cohort of Nh persons and depression disorder cohort of Nm persons

**S204** Division of brain regions, calculation of brain functional connectivity values, and harmonization

**S206** Under-sampling and sub-sampling (into Ns subsets)

Perform cross validation Ncv times

**S208** Generate identifier through leaning with feature selection for each of sub-samples (feature selection by L1 regularization (LASSO))

**S210** Generate diagnosis marker having average of (Ns × Ncv) identifiers as output

**S220** Rank features in sum set of brain functional connectivity links selected for identifiers

**S222** Select features in accordance with importance from sum set

**S224** Perform multiple co-clustering learning (unsupervised learning) using sum set of brain functional connectivity links

**S226** Prepare clustering classifier

Fig.17

[Fig.17]

Fig.18

[Fig.18]

Chain-dotted line surrounds
features that may be used to
identify label M and label H
("sum-set" in Fig. 16)

Dotted lines surround
strongly correlated
features

Black dots indicate features
selected through LASSO
for sub-sampled group

Total of Nch
features
characterizing
each subject

Fig.19

[Fig.19]

Data to be clustered

Manner of clustering 1
Feature: background

Manner of clustering 2
Feature: style

Manner of clustering 3
Feature: number of closed portions

Fig. 20

[Fig.20]

EP 4 183 333 A1

Fig.21

[Fig.21]

Co-clustering

i=1  i=2

j=3  j=2  j=1

Feature 000M
Feature 0004
Feature 0083
Feature 0002
Feature 0063
Feature 0003
Feature 0001

Object 0001
Object 0003
Object 0072
Object 0082
Object 0002
Object 000N

(a)

Feature group

Feature 000M

Feature 0001

Object cohort

Object 0001

Object 000N

(i)

Fig.22

[Fig.22]

Multiple co-clustering

Object cohort

Viewpoint 1    Viewpoint 2    Viewpoint 3

Feature group

(c)

Multiple clustering

Object cohort

Object cluster 3(1)    Object cluster 2(1)    Object cluster 1(1)

Object cluster 1(2)    Object cluster 2(2)

Object cluster 1(3)    Object cluster 2(3)    Object cluster 3(3)    Object cluster 4(3)

Viewpoint 1    Viewpoint 2    Viewpoint 3

Feature group

(b)

Fig.23

[Fig.23]

Fig.24

[Fig.24]

Multiple co-clustering learning process
S300

Divide features into partial groups at random
S302

Optimize object clusters for
partial groups of features
S304

Optimize partitioning of features for
obtained object cluster
S306

Does objective
function converge?    N
S308

Y

Store magnitude of objective function
S310

N    Has process
been repeated prescribed number
of times?
S312

Y

Determine feature partitioning and manner of clustering
that attain maximum objective function as final result
S314

End
S320

Fig.25

[Fig.25]

Fig.26

[Fig.26]

| Dataset 1 | | |
|---|---|---|
| Facility | Healthy person | Depression patient |
| 1 | 112 | 62 |
| 2 | 166 | 17 |
| 3 | 99 | 0 |
| 4 | 168 | 59 |
| Total | 545 | 138 |

(a)

| Dataset 2 | | |
|---|---|---|
| Facility | Healthy person | Depression patient |
| 5 | 28 | 32 |
| 6 | 49 | 16 |
| 7 | 66 | 57 |
| 8 | 120 | 76 |
| Total | 263 | 181 |

(b)

[Fig.27]

Fig. 27

Stratification marker search

Dataset 1

Dataset 2

Clustering 1

Clustering 2

Input: data matrix

Output: two clustering views

(a)

(b)

Feature cluster

Fig.29

[Fig.29]

Fig.30

[Fig.30]

| Number of brain functional connectivity links (FCs) | | Dataset 2 | |
|---|---|---|---|
| | | View 1 (93) | View 2 (6) |
| Dataset 1 | View 1 (92) | 92 | 0 |
| | View 2 (7) | 1 | 6 |

[Fig.31]

Cluster datasets 1 and 2 independently

Cluster datasets using each other's classifier

Dataset 1  →  Classifier 1  →  Clustering 1

Dataset 2  →  Classifier 2  →  Clustering 2

Dataset 1  →  Classifier 2  →  Clustering 1'

Dataset 2  →  Classifier 1  →  Clustering 2'

(a)

(b)

Fig.32

[Fig.32]

Classified into
same cluster in one test and
into different clusters in the other

(b)

Classified into
different clusters in two tests

Classified into
same cluster in two tests

(a)

Fig.33

[Fig.33]

| ARI | | Dataset 2 | |
|---|---|---|---|
| | | View 1 | View 2 |
| Dataset 1 | View 1 | 0.47* | 0 |
| | View 2 | 0.02 | 0.51* |

(a)

(b)

Fig.34

[Fig.34]

| | Cluster | Clustering 1' (View 1) | | | |
|---|---|---|---|---|---|
| | | 2 | 3 | 1 | 4 |
| Clustering 1 (View 1) | 1 | 47 | 9 | 2 | 0 |
| | 2 | 0 | 24 | 4 | 0 |
| | 4 | 2 | 20 | 0 | 0 |
| | 3 | 2 | 0 | 23 | 1 |
| | 5 | 0 | 0 | 0 | 4 |

Fig.35

[Fig.35]

[Fig.36]

$$Connectivity_{a,b} = x^a_m{}^T m^b + x^a_{Shc}{}^T s^b_{hc} + x^a_{Sdis}{}^T s^b_{dis} + x^a_d{}^T d^b + x^a_p{}^T p^b + const + e$$

Represents Ns sites

$$= (0,\ 0,\ \cdots,\ 1,\ \cdots,\ 0) \begin{bmatrix} vvvv \\ -wwww \\ \vdots \\ xxxx \\ -yyyy \\ \vdots \\ zzzz \end{bmatrix} + (0,\ 0,\ \cdots,\ 1,\ \cdots,\ 0) \begin{bmatrix} pppp \\ -qqqq \\ \vdots \\ rrrr \\ \vdots \\ ssss \end{bmatrix} + \text{(Third and following terms)} \cdots$$

Represents sites of measurement of patient and healthy person

Site where measurement was performed for subject a

Measurement bias vector for b-th functional connectivity

i) Element of measurement site is 1 when subject a is healthy person and is not traveling subject, and
ii) all elements are 0 when subject a is not healthy person or patient

Sampling bias vector of healthy person for b-th functional connectivity at each site of measurement of patient and healthy person

Fig.36

EP 4 183 333 A1

Fig.37

[Fig.37]

Start (harmonization)

S400

Collect fMRI measurement data of
healthy persons and patients, attribute
data of subjects, and measurement
parameters from measurement sites

S402

Collect fMRI measurement data of
traveling subject, attribute data of
subject, and measurement parameters
from measurement sites

S404

Evaluate measurement bias of
each measurement site using
generalized linear mixed model

S406

Store measurement bias of
each measurement site
in storage device

S408

End

Fig.38

[Fig.38]

Fig.39

[Fig.39]

[Fig.40]

Fig. 40

EP 4 183 333 A1

| Cluster | Recommended therapy | | | Unrecommended therapy |
|---|---|---|---|---|
| | First candidate | Second candidate | Third candidate | |
| 1 | Medication with therapeutic agent A | Medication with therapeutic agent A combined with medication with therapeutic agent B | Application of physical therapy $\alpha$ | Medication with therapeutic agent C |
| 2 | Medication with therapeutic agent A combined with medication with therapeutic agent B | Medication with therapeutic agent B combined with medication with therapeutic agent C | Application of physical therapy $\alpha$ | Medication with therapeutic agent Y |
| 3 | Medication with therapeutic agent B combined with medication with therapeutic agent C | Application of physical therapy $\alpha$ | Application of physical therapy $\beta$ | N/A |
| 4 | Medication with therapeutic agent C | Medication with therapeutic agent B combined with medication with therapeutic agent C | Application of physical therapy $\beta$ | Medication with therapeutic agent Y |
| 5 | Application of physical therapy $\alpha$ | Application of physical therapy $\alpha$ combined with medication with therapeutic agent B | Application of physical therapy $\delta$ combined with medication with therapeutic agent B | Medication with therapeutic agent C |

Fig.41

[Fig.41]

```
┌─────────────────────────────────────┐
│   Start (therapy selection support)  │
└─────────────────────────────────────┘
                    │
                    ▼
┌─────────────────────────────────────┐
│   Receive results of measurement of  │
│      brain activities of subject     │
└─────────────────────────────────────┘
                                  S502
                    │
                    ▼
┌─────────────────────────────────────┐
│     Input measurement results to     │
│   clustering classifier and acquire  │
│    cluster as stratification results │
└─────────────────────────────────────┘
                                  S504
                    │
                    ▼
┌─────────────────────────────────────┐
│      Acquire therapy information     │
│     corresponding to cluster as      │
│        stratification results        │
└─────────────────────────────────────┘
                                  S506
                    │
                    ▼
┌─────────────────────────────────────┐
│      Output therapy information      │
└─────────────────────────────────────┘
                                  S508
                    │
                    ▼
┌─────────────────────────────────────┐
│                 End                  │
└─────────────────────────────────────┘
```

Fig.42

[Fig.42]

Fig.43

[Fig.43]

```
┌─────────────────────────────────┐
│      Start (screening support)   │
└─────────────────────────────────┘
                 │
                 ▼
┌─────────────────────────────────┐
│  Receive results of measurement of │
│    brain activities of subject    │
└─────────────────────────────────┘
                         S602
                 │
                 ▼
┌─────────────────────────────────┐
│    Input measurement results to   │
│  clustering classifier and acquire │
│   cluster as stratification results │
└─────────────────────────────────┘
                         S604
                 │
                 ▼
┌─────────────────────────────────┐
│   Generate information indicating  │
│  clusters as stratification results │
└─────────────────────────────────┘
                         S606
                 │
                 ▼
┌─────────────────────────────────┐
│    Output information on clusters  │
└─────────────────────────────────┘
                         S608
                 │
                 ▼
┌─────────────────────────────────┐
│               End                │
└─────────────────────────────────┘
```

Fig.44

[Fig.44]

Fig.45

[Fig.45]

Results of clustering for dataset 1

Fig.46

[Fig.46]

Results of clustering for dataset 2

[Fig.47]

Stability of clustering between datasets

| ARI | | Dataset 2 | | |
|---|---|---|---|---|
| | | View 1 | View 2 | View 3 |
| Dataset 1 | View 1 | 0.67 | 0.01 | 0.05 |
| | View 2 | 0.04 | 0.00 | 0.68 |

Fig.48

[Fig.48]

Fig.49

[Fig.49]

Results of subject clustering (View3)

| Subtype | All MDD Subjects | | MDD Subjects with Clinical Data | |
|---|---|---|---|---|
| | Number | Rate | Number | Rate |
| 1 | 107 | 34% | 26 | 27% |
| 2 | 92 | 29% | 32 | 33% |
| 3 | 44 | 14% | 8 | 8% |
| 4 | 27 | 8% | 11 | 11% |
| 5 | 25 | 8% | 10 | 10% |
| 6 | 9 | 3% | 5 | 5% |
| 7 | 9 | 3% | 4 | 4% |
| 8 | 6 | 2% | 0 | 0% |
| Total | 319 | 100% | 96 | 100% |

Fig. 50

[Fig.50]

Brain functional connectivity links used for clustering (View 3)

| FC | ROI-1 | ROI-2 |
| --- | --- | --- |
| | Label (AAL) | Label (AAL) |
| 1 | Thalamus_R | Precentral_R |
| 2 | Thalamus_R | Postcentral_L |
| 3 | Thalamus_L | Precentral_R |

(a)

(b)

Fig.51

[Fig.51]

**INTERNATIONAL SEARCH REPORT**

International application No.

**PCT/JP2021/026658**

| A. | CLASSIFICATION OF SUBJECT MATTER |
|---|---|

*A61B 5/055*(2006.01)i; *A61B 5/05*(2021.01)i; *A61B 5/372*(2021.01)i; *G06T 7/00*(2017.01)i
FI: A61B5/055 380; A61B5/05; A61B5/055 311; A61B5/055 390; A61B5/372; G06T7/00 350B; G06T7/00 612

According to International Patent Classification (IPC) or to both national classification and IPC

| B. | FIELDS SEARCHED |
|---|---|

Minimum documentation searched (classification system followed by classification symbols)

A61B5/055; A61B5/05; A61B5/372; G06T7/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2019-063478 A (ADVANCED TELECOMMUNICATIONS RESEARCH INSTITUTE INTERNATIONAL, HIROSHIMA UNIVERSITY) 25 April 2019 (2019-04-25) entire text, all drawings | 1-28 |
| A | US 2019/0090749 A1 (WASHINGTON UNIVERSITY) 28 March 2019 (2019-03-28) entire text, all drawings | 1-28 |
| A | WO 2014/178323 A1 (ADVANCED TELECOMMUNICATIONS RESEARCH INSTITUTE INTERNATIONAL) 06 November 2014 (2014-11-06) entire text, all drawings | 1-28 |
| A | WO 2012/083136 A1 (THE TRUSTEES OF COLUMBIA UNIVERSITY IN THE CITY OF NEW YORK) 21 June 2012 (2012-06-21) entire text, all drawings | 1-28 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 September 2021** | **12 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/026658**

| Patent document cited in search report | | | Publication date (day/month/year) | Patent family member(s) | | | Publication date (day/month/year) |
|---|---|---|---|---|---|---|---|
| JP | 2019-063478 | A | 25 April 2019 | US | 2021/0034912 | A1 | |
| | | | | entire text, all drawings | | | |
| | | | | EP | 3692909 | A1 | |
| | | | | CN | 111447874 | A | |
| | | | | WO | 2019/069955 | A1 | |
| US | 2019/0090749 | A1 | 28 March 2019 | (Family: none) | | | |
| WO | 2014/178323 | A1 | 06 November 2014 | US | 2015/0272461 | A1 | |
| | | | | entire text, all drawings | | | |
| | | | | CN | 105163659 | A | |
| | | | | EP | 2992824 | A1 | |
| | | | | JP | 2015-062817 | A | |
| WO | 2012/083136 | A1 | 21 June 2012 | US | 2014/0155730 | A1 | |
| | | | | entire text, all drawings | | | |

Form PCT/ISA/210 (patent family annex) (January 2015)

EP 4 183 333 A1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018147193 A
- WO 2018147193 A
- JP 2019198376 A
- JP 2020024139 A
- JP 2019516950 A
- WO 2017162773 A
- JP 2005025421 A
- WO 2005025421 A
- JP 2015062817 A
- JP 2017196523 A

**Non-patent literature cited in the description**

- Japan Agency for Medical Research and Development. *AI mounting endoscope diagnosis assisting program approved - Expected to assist physicians' diagnosis,* 10 December 2018, https://www.amed.go.jp/news/release_20181210.html
- **GLASSER MF et al.** The Human Connectome Project's neuroimaging approach. *NatNeurosci,* 2016, vol. 19, 1175-1187
- **ANDREW T DRYSDALE ; LOGAN GROSENICK ; JONATHAN DOWNAR ; KATHARINE DUNLOP ; FARROKH MANSOURI ; YUE MENG1 ; ROBERT N FETCHO ; BENJAMIN ZEBLEY ; DESMOND J OATHES ; AMIT ETKIN.** Resting-state connectivity biomarkers define neurophysiological subtypes of depression. *nature medicine,* January 2017, vol. 23 (1
- **TOMOKI TOKUDA ; JUNICHIRO YOSHIMOTO ; YU SHIMIZU ; GO OKADA ; MASAHIRO TAKAMURA ; YASUMASA OKAMOTO ; SHIGETO YAMAWAKI ; KENJI DOYA.** Identification of depression subtypes and relevant brain regions using a data-driven approach. *SCIENTIFIC REPORTS,* 2018, vol. 8, 14082
- **RICHARD DINGA ; LIANNE SCHMAAL ; BRENDA W.J.H. PENNINX ; MARIE JOSEVAN TOL ; DICK J. VELTMAN ; LAURA VAN VELZEN ; MAARTEN MENNES ; NIC J.A.VAN DER WEE ; ANDRE F. MARQUAND et al.** Evaluating the evidence for biotypes of depression: Methodological replication and extension of Drysdale et al. (2017). *NeuroImage: Clinical,* 2019, vol. 22, 101796
- **NOBLE S et al.** Multisite reliability of MR-based functional connectivity. *Neuroimage,* 2017, vol. 146, 959-970
- **PEARLSON G.** Multisite collaborations and large databases in psychiatric neuroimaging advantages, problems, and challenges. *Schizophr Bull,* 2009, vol. 35, 1-2
- **AYUMU YAMASHITA ; NORIAKI YAHATA ; TAKASHI ITAHASHI ; GIUSEPPE LISI ; TAKASHI YAMADA ; NAHO ICHIKAWA ; MASAHIRO TAKAMURA ; YUJIRO YOSHIHARA ; AKIRA KUNIMATSU ; NAOHIRO OKADA.** Harmonization of resting-state functional MRI data across multiple imaging sites via the separation of site differences into sampling bias and measurement bias. *PLOS Biology,* http://journals.plos.org/plosbiology/article?id=0.1371/journal.pbio.3000042
- **PERROT et al.** *Med Image Anal,* 2011, vol. 15 (4
- **TZOURIO-MAZOYER et al.** *Neuroimage,* 2002, vol. 15 (1
- **FINN ES et al.** Functional connectome fingerprinting: identifying individuals using patterns of brain connectivity. *Nat Neurosci,* 2015, vol. 18, 1664-1671
- **ROSENBERG MD et al.** A neuromarker of sustained attention from whole-brain functional connectivity. *Nat Neurosci,* 2016, vol. 19, 165-171
- **SHEN X ; TOKOGLU F ; PAPADEMETRIS X ; CONSTABLE RT.** Groupwise whole-brain parcellation from resting-state fMRI data for network node identification. *Neuroimage,* 2013, vol. 82, 403-415
- **GLASSER, M.F. ; COALSON, T.S. ; ROBINSON, E.C. ; HACKER, C.D. ; HARWELL, J. ; YACOUB, E. et al.** A multi-modal parcellation of human cerebral cortex. *Nature,* 2016, vol. 536 (7615), 171-178
- **TZOURIO-MAZOYER, N. ; LANDEAU, B. ; PAPATHANASSIOU, D. ; CRIVELLO, F. ; ETARD, O. ; DELCROIX, N. et al.** Automated anatomical labeling of activations in SPM using a macroscopic anatomical parcellation of the MNI MRI single-subject brain. *Neuroimage,* 2002, vol. 15 (1), 273-289

- **KAMALAKER DADI ; MEHDI RAHIM ; ALEXANDRE ABRAHAM ; DARYA CHYZHYK ; MICHAEL MILHAM ; BERTRAND THIRION ; GAEL VAROQUAUX.** Benchmarking functional connectome-based predictive models for resting-state fMRI. *Preprint submitted to NeuroImage,* 31 October 2018
- **BEHZADI, Y. ; RESTOM, K. ; LIAU, J. ; LIU, T.T.** A component based noise correction method (CompCor) for BOLD and perfusion based fMRI. *Neuroimage,* 2007, vol. 37 (1), 90-101
- **JOHNSON WE ; LI C ; RABINOVIC A.** Adjusting batch effects in microarray expression data using empirical Bayes methods. *Biostatistics,* 2007, vol. 8, 118-127
- **MADEIRA SC ; OLIVEIRA AL.** Biclustering algorithms for biological data analysis: a survey. *IEEE/ACM Transactions on Computational Biology and Bioinformatics (TCBB),* 2004, vol. 1 (1), 24-45, https://doi.org/10.1109/TCBB.2004.2
- **TOMOKI TOKUDA ; JUNICHIRO YOSHIMOTO ; YU SHIMIZU ; GO OKADA ; MASAHIRO TAKAMURA ; YASUMASA OKAMOTO ; SHIGETO YAMAWAKI ; KENJI DOYA.** Multiple co-clustering based on nonparametric mixture models with heterogeneous marginal distributions. *PLOS ONE,* 19 October 2017, https://doi.org/10.1371/journal.pone.0186566
- **BLEI DM ; JORDAN MI et al.** Variational inference for Dirichlet process mixtures. *Bayesian analysis,* 2006, vol. 1 (1), 121-143, https://doi.org/10.1214/06-BA104
- **GUAN Y ; DY JG ; NIU D ; GHAHRAMANI Z.** Variational inference for nonparametric multiple clustering. *MultiClust Workshop, KDD-2010,* 2010
- **JENSEN V.** Sur les fonctions convexes et les inegalites entre les valeurs moyennes. *Acta Mathematica,* 1906, vol. 30 (1), 175-193, https://doi. org/10.1007BF02418571
- **MURPHY K.** Machine Learning: A Probabilistic Perspective. MIT Press, 2012
- **JORGE M. SANTOS ; MARK EMBRECHTS.** On the Use of the Adjusted Rand Index as a Metric for Evaluating Supervised Classification. *ICANN 2009, Part II, LNCS,* 2009, vol. 5769, 175-184
- Data kaiseki no tameno toukei modeling nyu'mon. **TAKUYA KUBO.** Introduction to statistical modeling for data analysis. Iwanami Shoten, 2012
- **MATTHIEU PERRO ; DENIS RIVIERE ; JEAN-FRANCOIS MANGIN A.** Cortical sulci recognition and spatial normalization. *Medical Image Analysis,* August 2011, vol. 15 (4), 529-550